(19) Europäisches Patentamt
European Patent Office
Office européen des brevets

(11) **EP 4 356 445 B1**

(12) **EUROPEAN PATENT SPECIFICATION**

(45) Date of publication and mention of the grant of the patent:
**08.10.2025 Bulletin 2025/41**

(21) Application number: **22737391.7**

(22) Date of filing: **15.06.2022**

(51) International Patent Classification (IPC):
***H10K 50/00*** *(2023.01)* ***H10K 85/60*** *(2023.01)*
*H10K 30/00* *(2023.01)*

(52) Cooperative Patent Classification (CPC):
**C07D 307/91; C07C 211/61; C07D 209/88; C07D 307/77; C07D 307/93; C07D 333/76; C07D 405/12; C07F 7/0816; H10K 85/40; H10K 85/633; H10K 85/636; H10K 85/6572; H10K 85/6574; H10K 85/6576;** C07C 2603/18;
(Cont.)

(86) International application number:
**PCT/EP2022/066327**

(87) International publication number:
**WO 2022/263519 (22.12.2022 Gazette 2022/51)**

(54) **ORGANIC ELECTRONIC DEVICE COMPRISING A COMPOUND OF FORMULA (I), DISPLAY DEVICE COMPRISING THE ORGANIC ELECTRONIC DEVICE AS WELL AS COMPOUNDS OF FORMULA (I)**

ORGANISCHE ELEKTRONISCHE VORRICHTUNG MIT EINEM SUBSTRAT, EINER ANODENSCHICHT, EINER KATHODENSCHICHT, MINDESTENS EINER ERSTEN EMISSIONSSCHICHT UND MINDESTENS EINER LOCHINJEKTIONSSCHICHT MIT EINEM METALLKOMPLEX

DISPOSITIF ÉLECTRONIQUE ORGANIQUE COMPRENANT UN SUBSTRAT, UNE COUCHE D’ANODE, UNE COUCHE DE CATHODE, AU MOINS UNE PREMIÈRE COUCHE D’ÉMISSION ET AU MOINS UNE COUCHE D’INJECTION DE TROUS QUI COMPREND UN COMPLEXE MÉTALLIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.06.2021 EP 21180322**
**20.12.2021 EP 21215975**

(43) Date of publication of application:
**24.04.2024 Bulletin 2024/17**

(73) Proprietor: **Novaled GmbH**
**01099 Dresden (DE)**

(72) Inventors:
- **UVAROV, Vladimir**
**01099 Dresden (DE)**
- **HEGGEMANN, Ulrich**
**01099 Dresden (DE)**
- **WILLMANN, Steffen**
**01099 Dresden (DE)**
- **PINTER, Piermaria**
**01099 Dresden (DE)**
- **MOO, Jin Park**
**01099 Dresden (DE)**

(74) Representative: **Michalski Hüttermann & Partner Patentanwälte mbB**
**Kaistraße 16A**
**40221 Düsseldorf (DE)**

(56) References cited:
**EP-A1- 3 133 664**
**CN-A- 107 602 441**
**US-A1- 2018 331 308**
**US-A1- 2020 365 813**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 356 445 B1

(52) Cooperative Patent Classification (CPC): (Cont.)
C07C 2603/40; H10K 30/50; H10K 50/15;
H10K 50/155

## Description

## Technical Field

The present invention relates to an organic electronic device comprising a compound of formula (I) and a display device comprising the organic electronic device. The invention further relates to novel compounds of formula (I) which can be of use in organic electronic devices.

## Background Art

Organic electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

CN 107 602 441 A refers to a fused ring compound of a carbazole type structure and an organic luminescent device thereof, and relates to the technical field of an organic photoelectric material.

US 2020/365813A1 refers to an organic electronic device having at least an anode, a hole transport layer, a light emitting layer, an electron transport layer, and a cathode, in this order in which the hole transport layer contains an indenophenanthrene compound.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the semiconductor layer, and among them, may be affected by characteristics of compounds of formula (I) which are contained in the semiconductor layer.

There remains a need to improve performance of organic semiconductor materials, semiconductor layers, as well as organic electronic devices thereof, in particular to achieve improved operating voltage stability over time through improving the characteristics of the compounds comprised therein.

## DISCLOSURE

An aspect of the present invention provides a benzo diphenyl fluorene compound, represented by formula (I):

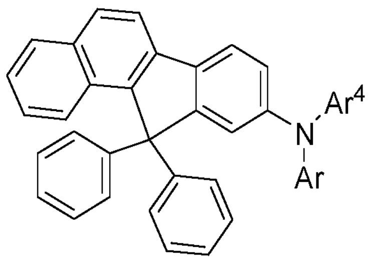

(I);

wherein $Ar^4$ is represented by formula (Ia) or (Ib),

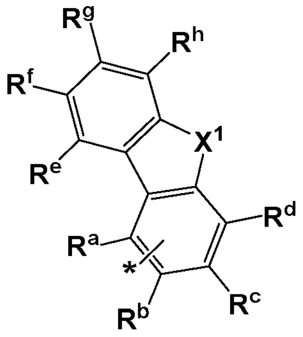 (Ia), 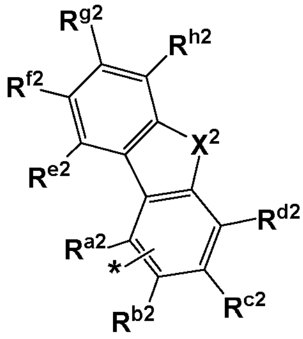 (Ib),

wherein the asterix "*" denotes the binding position of (Ia) and (Ib), and
wherein

| | |
|---|---|
| Ar =$Ar^1$ and $Ar^1$ | is selected from substituted or unsubstituted $C_6$ to $C_{24}$ aryl, or substituted or unsubstituted $C_3$ to $C_{25}$ heteroaryl,<br>at least one substituent on $Ar^1$ is selected from $C_1$ to $C_6$ alkyl, $C_6$ to $C_{12}$ aryl, $C_3$ to $C_{12}$ heteroaryl; |
| $R^a$, $R^b$ , $R^c$ and $R^d$ | are independently selected from H, $C_1$ to $C_6$ alkyl, $C_6$ to $C_{18}$ aryl, and $C_2$ to $C_{18}$ heteroaryl, and one of the $R^a$, $R^b$, $R^c$ or $R^d$ represents a single bond that bonds to N of formula (I), optional two of adjacent substituents selected from the group of $R^a$, $R^b$, $R^c$ and $R^d$ form a substituted or unsubstituted condensed ring system, wherein<br>at least one substituent on the condensed ring system is independently selected from $C_1$ to $C_6$ alkyl, $C_6$ to $C_{12}$ aryl or $C_2$ to $C_{12}$ heteroaryl; |
| $R^e$, $R^f$, $R^g$, and $R^h$ | are independently selected from H, $C_1$ to $C_6$ alkyl, $C_6$ to $C_{18}$ aryl, and $C_2$ to $C_{18}$ heteroaryl; |
| $X^1$ | is selected from O, S, $NAr^{1a}$; |
| $Ar^{1a}$ | is selected from $C_6$ to $C_{12}$ aryl;<br>or |
| Ar =$Ar^2$ and $Ar^2$ | is selected from $C_6$ to $C_{12}$ aryl or $C_2$ to $C_{25}$ heteroaryl; |
| $R^{a2}$, $R^{b2}$, $R^{c2}$ and $R^{d2}$ | are independently selected from H, $C_1$ to $C_6$ alkyl, $C_6$ to $C_{18}$ aryl, and $C_2$ to $C_{18}$ heteroaryl, and one of the $R^{a2}$, $R^{b2}$, $R^{c2}$ or $R^{d2}$ represents a single bond that bonds to N of formula (I), optional two of adjacent substituents selected from the group of $R^{a2}$, $R^{b2}$, $R^{c2}$ and $R^{d2}$ form a substituted or unsubstituted condensed ring system, wherein<br>at least one substituent on the condensed ring system is independently selected from $C_1$ to $C_6$ alkyl, $C_6$ to $C_{12}$ aryl or $C_2$ to $C_{12}$ heteroaryl; |
| $R^{e2}$, $R^{f2}$, $R^{g2}$ and $R^{h2}$ | are independently selected from H, $C_1$ to $C_6$ alkyl, $C_6$ to $C_{18}$ aryl, and $C_2$ to $C_{18}$ heteroaryl; |
| $X^2$ | is selected from O, S, $NAr^{2b}$, $CR^{1b}R^{26}$, $SiR^{1b}R^{2}$ ; |
| $Ar^{2b}$ | is selected from $C_6$ to $C_{12}$ aryl; and<br>$R^{1b}$ and $R^{2b}$ are independently selected from $C_1$ to $C_6$ alkyl, $C_6$ to $C_{12}$ aryl or $C_2$ to $C_{12}$ heteroaryl; |

or represented by compound A36:

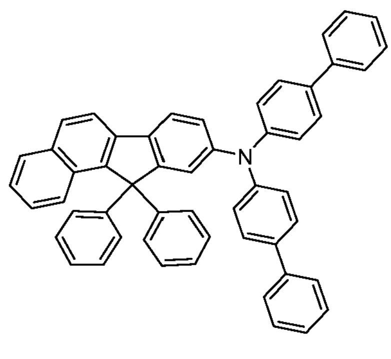 (A36).

It should be noted that throughout the application and the claims any Ar, $Ar^1$, $Ar^2$, $Ar^3$, $Ar^4$, $R^a$, $R^b$, $R^c$, $R^d$, $R^e$, $R^f$,

$R^g$, $R^h$, $X^1$, $X^2$, $X^{1b}$, $Ar^{1a}$, $Ar^{2b}$, $R^{1b}$, $R^{2b}$, $R^{a2}$, $R^{b2}$, $R^{c2}$, $R^{d2}$, $R^{e2}$, $R^{f2}$, $R^{g2}$, $R^{h2}$, and so on always refer to the same moieties, unless otherwise noted.

In the present specification, when a definition is not otherwise provided, "substituted" refers to one substituted with a H, or deuterium, or $C_1$ to $C_6$ alkyl, $C_6$ to $C_{12}$ aryl, $C_5$ to $C_{12}$ heteroaryl, or $C_1$ to $C_6$ alkyl, $C_6$ to $C_{12}$ aryl or $C_2$ to $C_{12}$ heteroaryl, or H, $C_1$ to $C_6$ alkyl, $C_6$ to $C_{18}$ aryl, and $C_2$ to $C_{18}$ heteroaryl.

However, in the present specification "aryl substituted" refers to a substitution with one or more aryl groups, which themselves may be substituted with one or more aryl and/or heteroaryl groups.

Correspondingly, in the present specification "heteroaryl substituted" refers to a substitution with one or more heteroaryl groups, which themselves may be substituted with one or more aryl and/or heteroaryl groups.

In the present specification, when a definition is not otherwise provided, an "alkyl group" refers to a saturated aliphatic hydrocarbyl group. The alkyl group may be a $C_1$ to $C_{12}$ alkyl group. More specifically, the alkyl group may be a $C_1$ to $C_{10}$ alkyl group or a $C_1$ to $C_6$ alkyl group. For example, a $C_1$ to $C_4$ alkyl group includes 1 to 4 carbons in alkyl chain, and may be selected from methyl, ethyl, propyl, iso-propyl, n-butyl, iso-butyl, sec-butyl, and tert-butyl.

Specific examples of the alkyl group may be a methyl group, an ethyl group, a propyl group, an isopropyl group, a butyl group, an iso-butyl group, a sec-butyl group, a tert-butyl group, a pentyl group, a hexyl group.

The term "cycloalkyl" refers to saturated hydrocarbyl groups derived from a cycloalkane by formal abstraction of one hydrogen atom from a ring atom comprised in the corresponding cycloalkane. Examples of the cycloalkyl group may be a cyclopropyl group, a cyclobutyl group, a cyclopentyl group, a cyclohexyl group, a methyl cyclohexyl group, an adamantly group and the like.

The term "hetero" is understood the way that at least one carbon atom, in a structure which may be formed by covalently bound carbon atoms, is replaced by another polyvalent atom. Preferably, the heteroatoms are selected from B, Si, N, P, O, S; more preferably from N, P, O, S.

In the present specification, "aryl group" refers to a hydrocarbyl group which can be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenyl or tolyl, polycyclic groups which comprise more aromatic rings linked by single bonds, like biphenyl, and polycyclic groups comprising fused rings, like naphthyl or fluoren-2-yl.

Analogously, under heteroaryl, it is especially where suitable understood a group derived by formal abstraction of one ring hydrogen from a heterocyclic aromatic ring in a compound comprising at least one such ring.

Under heterocycloalkyl, it is especially where suitable understood a group derived by formal abstraction of one ring hydrogen from a saturated cycloalkyl ring in a compound comprising at least one such ring.

The term "fused aryl rings" or "condensed aryl rings" is understood the way that two aryl rings are considered fused or condensed when they share at least two common $sp^2$-hybridized carbon atoms.

The term "cyano moiety" refers to a CN substituent.

In the present specification, the single bond refers to a direct bond.

The term "n-type charge generation layer" is sometimes in the art also named n-CGL or electron generation layer and is intended to include the both.

The term "p-type charge generation layer" is sometimes in the art also named p-CGL or hole generation layer and is intended to include the both.

According to one embodiment of the present invention the p-type and/or n-type charge generation layer and/or the compound of formula (I) are non-emissive.

The term "free of", "does not contain", "does not comprise" does not exclude impurities which may be present in the compounds prior to deposition. Impurities have no technical effect with respect to the object achieved by the present invention.

The term "contacting sandwiched" refers to an arrangement of three layers whereby the layer in the middle is in direct contact with the two adjacent layers.

The terms "light-absorbing layer" and "light absorption layer" are used synonymously.

The terms "light-emitting layer", "light emission layer" and "emission layer" are used synonymously.

The terms "OLED", "organic light-emitting diode" and "organic light-emitting device" are used synonymously.

The terms "anode", "anode layer" and "anode electrode" are used synonymously.

The terms "cathode", "cathode layer" and "cathode electrode" are used synonymously.

The term "top emission device" is understood to mean an organic electronic device wherein the light is emitted through the cathode layer.

The term "bottom emission device" is understood to mean an organic electronic device wherein the light is emitted through the substrate.

The operating voltage U is measured in Volt.

In the context of the present specification the term "essentially non-emissive" or "non-emissive" means that the contribution of the compounds of formula I, Ia, Ib, Ic, Id, Ie, If, Ig, Ih, Ik, the organic electron transport compound, the organic hole transport compound, the matrix compounds of formula (VI) or formula (VII), the metal complex and/or layer, the p-type charge generation layer, as well as the n-type charge generation layer, to the visible emission spectrum from an organic electronic device, such as OLED or display device, is less than 10 %, preferably less than 5 % relative to the visible emission spectrum. The visible emission spectrum is an emission spectrum with a wavelength of about ≥ 380 nm to about ≤ 780 nm.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electrons formed in the cathode may be easily injected into the emission layer and transported in the emission layer due to conductive characteristics according to a lowest unoccupied molecular orbital (LUMO) level.

The term "HOMO level" is understood to mean the highest occupied molecular orbital and is determined in eV (electron volt).

The term "HOMO level further away from vacuum level" is understood to mean that the absolute value of the HOMO level is higher than the absolute value of the HOMO level of the reference compound. For example, the term "further away from vacuum level than the HOMO level of N2,N2,N2',N2',N7,N7,N7',N7'-octakis(4-methoxyphenyl)-9,9'-spirobi [fluorene]-2,2',7,7'-tetraamine is understood to mean that the absolute value of the HOMO level of the organic matrix of the p-type charge generation layer is higher than the HOMO level of N2,N2,N2',N2', N7,N7, N7',N7'-octakis(4-methoxyphenyl)-9,9'-spirobi[fluorene]-2,2',7,7'-tetraamine.

The term "absolute value" is understood to mean the value without the "-" symbol. According to one embodiment of the present invention, the HOMO level of the organic matrix of the p-type charge generation layer may be calculated by quantum mechanical methods.

The work function of the first metal is measured in eV (electron volt). Tabulated values of work functions can be found for example in CRC Handbook of Chemistry and Physics version 2008, p. 12-114. Further, tabulated values of work functions can be found for example at https://en.wikipedia.org/wiki/Work_function#cite_note-12.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electrons formed in the cathode may be easily injected into the emission layer and transported in the emission layer due to conductive characteristics according to a lowest unoccupied molecular orbital (LUMO) level.

## Advantageous Effects

Surprisingly, it was found that the organic electronic device according to the invention solves the problem underlying the present invention by enabling devices in various aspects superior over the organic electroluminescent devices known in the art, in particular with respect to operating voltage over lifetime.

## Embodiments

According to one embodiment the benzo diphenyl fluorene compound is represented by formula (I):

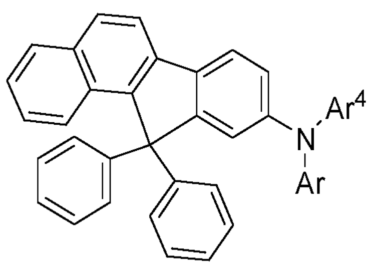

(I),

wherein $Ar^4$ is represented by formula (Ia) or (Ib),

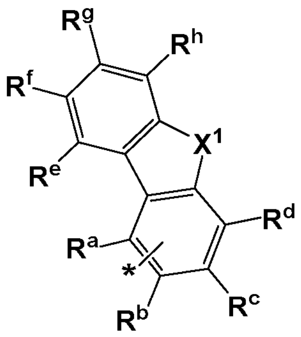

(Ia),

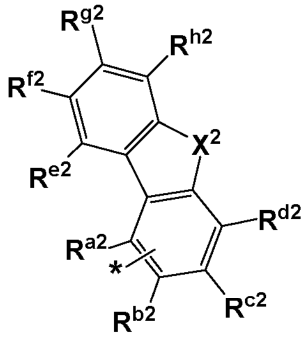

(Ib),

wherein the asterix "*" denotes the binding position of (Ia) and (Ib), and
wherein

| | |
|---|---|
| Ar =$Ar^1$ and $Ar^1$ | is selected from substituted or unsubstituted $C_6$ to $C_{24}$ aryl, or substituted or unsubstituted $C_5$ to $C_{25}$ heteroaryl, at least one substituent on $Ar^1$ is selected from $C_1$ to $C_6$ alkyl, $C_6$ to $C_{12}$ aryl, $C_5$ to $C_{12}$ heteroaryl; |
| $R^a$, $R^b$ , $R^c$ and $R^d$ | are independently selected from H, $C_1$ to $C_6$ alkyl, $C_6$ to $C_{18}$ aryl, and $C_2$ to $C_{18}$ heteroaryl, and one of the $R^a$, $R^b$, $R^c$ or $R^d$ represents a single bond that bonds to N of formula (I), optional two of adjacent substituents selected from the group of $R^a$, $R^b$, $R^c$ and $R^d$ form a substituted or unsubstituted condensed ring system, wherein at least one substituent on the condensed ring system is independently selected from $C_1$ to $C_6$ alkyl, $C_6$ to $C_{12}$ aryl or $C_2$ to $C_{12}$ heteroaryl; |
| $R^e$, $R^f$ , $R^g$ and $R^h$ | are independently selected from H, $C_1$ to $C_6$ alkyl, $C_6$ to $C_{18}$ aryl, and $C_2$ to $C_{18}$ heteroaryl; |
| $X^1$ | is selected from O, S, $NAr^{1a}$; |
| $Ar^{1a}$ | is selected from $C_6$ to $C_{12}$ aryl; |
| | or |
| Ar =$Ar^2$ and $Ar^2$ | is selected from $C_6$ to $C_{12}$ aryl, $C_5$ to $C_{25}$ heteroaryl or biphenyl; |
| $R^{a2}$, $R^{b2}$, $R^{c2}$ and $R^{d2}$ | are independently selected from H, $C_1$ to $C_6$ alkyl, $C_6$ to $C_{18}$ aryl, and $C_2$ to $C_{18}$ heteroaryl, and one of the $R^a$, $R^b$, $R^c$ or $R^d$ represents a single bond that bonds to N of formula (I), optional two of adjacent substituents selected from the group of $R^a$, $R^b$, $R^c$ and $R^d$ form a substituted or unsubstituted condensed ring system, wherein at least one substituent on the condensed ring system is independently selected from $C_1$ to $C_6$ alkyl, $C_6$ to $C_{12}$ aryl or $C_2$ to $C_{12}$ heteroaryl; |
| $R^{e2}$, $R^{f2}$, $R^{g2}$ and $R^{h2}$ | are independently selected from H, $C_1$ to $C_6$ alkyl, $C_6$ to $C_{18}$ aryl, and $C_2$ to $C_{18}$ heteroaryl; |
| $X^2$ | is selected from O, S, $NAr^{2b}$, $CR^{1b}R^{26}$, $SiR^{1b}R^{2}$ ; |
| $Ar^{2b}$ | is selected from $C_6$ to $C_{12}$ aryl; and |
| $R^{1b}$ and $R^{2b}$ | are independently selected from $C_1$ to $C_6$ alkyl, $C_6$ to $C_{12}$ aryl or $C_2$ to $C_{12}$ heteroaryl. |

According to one embodiment, wherein the benzo diphenyl fluorene compound is represented by formula (I):

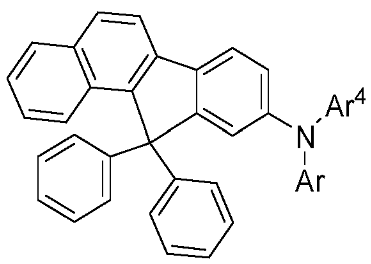

(I),

wherein $Ar^4$ is represented by formula (Ia) or (Ib),

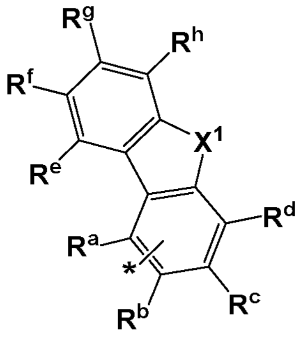 (Ia), 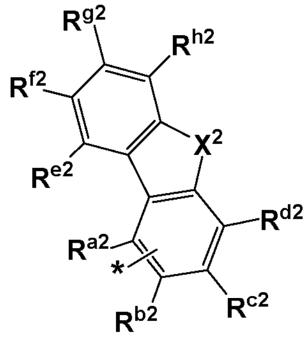 (Ib),

wherein the asterix "*" denotes the binding position of (Ia) and (Ib), and
wherein

| | |
|---|---|
| Ar =$Ar^1$ and $Ar^1$ | is selected from unsubstituted $C_6$ to $C_{24}$ aryl, or unsubstituted $C_5$ to $C_{25}$ heteroaryl; |
| $R^a$, $R^b$ , $R^c$ and $R^d$ | are independently selected from H, $C_1$ to $C_6$ alkyl, $C_6$ to $C_{18}$ aryl, and $C_2$ to $C_{18}$ heteroaryl, and one of the $R^a$, $R^b$, $R^c$ or $R^d$ represents a single bond that bonds to N of formula (I), optional two of adjacent substituents selected from the group of $R^a$, $R^b$, $R^c$ and $R^d$ form an unsubstituted condensed ring system; |
| $R^e$, $R^f$ , $R^g$, and $R^h$ | are independently selected from H, $C_1$ to $C_6$ alkyl, $C_6$ to $C_{18}$ aryl, and $C_2$ to $C_{18}$ heteroaryl; |
| $X^1$ | is selected from O, S, $NAr^{1a}$; |
| $Ar^{1a}$ | is selected from $C_6$ to $C_{12}$ aryl; |
| | or |
| Ar =$Ar^2$ and $Ar^2$ | is selected from $C_6$ to $C_{12}$ aryl, $C_5$ to $C_{25}$ heteroaryl or biphenyl; |
| $R^{a2}$, $R^{b2}$ , $R^{c2}$ and $R^{d2}$ | are independently selected from H, $C_1$ to $C_6$ alkyl, $C_6$ to $C_{18}$ aryl, and $C_2$ to $C_{18}$ heteroaryl, and one of the $R^a$, $R^b$, $R^c$ or $R^d$ represents a single bond that bonds to N of formula (I), optional two of adjacent substituents selected from the group of $R^a$, $R^b$, $R^c$ and $R^d$ form an unsubstituted condensed ring system; |
| $R^{e2}$, $R^{f2}$, $R^{g2}$ and $R^{h2}$ | are independently selected from H, $C_1$ to $C_6$ alkyl, $C_6$ to $C_{18}$ aryl, and $C_2$ to $C_{18}$ heteroaryl; |
| $X^2$ | is selected from O, S, $NAr^{2b}$, $CR^{1b}R^{26}$, $SiR^{1b}R^2$ ; |
| $Ar^{2b}$ | is selected from $C_6$ to $C_{12}$ aryl; and |
| $R^{1b}$ and $R^{2b}$ | are independently selected from $C_1$ to $C_6$ alkyl, $C_6$ to $C_{12}$ aryl or $C_2$ to $C_{12}$ heteroaryl. |

According to one embodiment, wherein the benzo diphenyl fluorene compound, represented by formula (I):

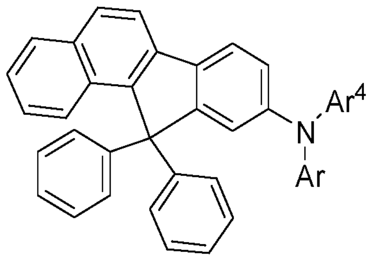

(I),

wherein $Ar^4$ is represented by formula (Ia) or (Ib),

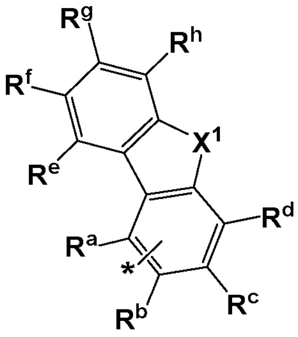 (Ia), 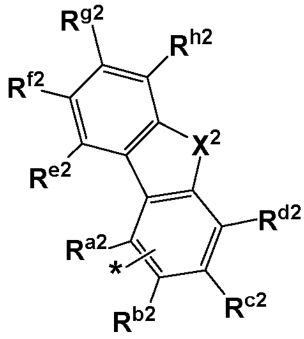 (Ib),

wherein the asterix "*" denotes the binding position of (Ia) and (Ib), and
wherein

| | |
|---|---|
| Ar =$Ar^1$ and $Ar^1$ | is selected from substituted or unsubstituted $C_6$ to $C_{24}$ aryl, or substituted or unsubstituted $C_5$ to $C_{25}$ heteroaryl,<br>at least one substituent on $Ar^1$ is selected from $C_1$ to $C_6$ alkyl, $C_6$ to $C_{12}$ aryl, $C_5$ to $C_{12}$ heteroaryl; |
| $R^a$, $R^b$, $R^c$ and $R^d$ | are independently selected from H, $C_1$ to $C_6$ alkyl, $C_6$ to $C_{18}$ aryl, and $C_2$ to $C_{18}$ heteroaryl, and one of the $R^a$, $R^b$, $R^c$ or $R^d$ represents a single bond that bonds to N of formula (I), optional two of adjacent substituents selected from the group of $R^a$, $R^b$, $R^c$ and $R^d$ form a substituted or unsubstituted condensed ring system, wherein<br>at least one substituent on the condensed ring system is independently selected from $C_1$ to $C_6$ alkyl, $C_6$ to $C_{12}$ aryl or $C_2$ to $C_{12}$ heteroaryl; |
| $R^e$, $R^f$ , $R^8$ and $R^h$ | are independently selected from H, $C_1$ to $C_6$ alkyl, $C_6$ to $C_{18}$ aryl, and $C_2$ to $C_{18}$ heteroaryl; |
| $X^1$ | is selected from O, S, $NAr^{1a}$; |
| $Ar^{1a}$ | is selected from $C_6$ to $C_{12}$ aryl;<br>or |
| Ar =$Ar^2$ and $Ar^2$ | is selected from $C_6$ to $C_{12}$ aryl, $C_5$ to $C_{25}$ heteroaryl or biphenyl; |
| $R^{a2}$, $R^{b2}$ , $R^{c2}$ and $R^{d2}$ | are independently selected from H, $C_1$ to $C_6$ alkyl, $C_6$ to $C_{18}$ aryl, and $C_2$ to $C_{18}$ heteroaryl, and one of the $R^a$, $R^b$, $R^c$ or $R^d$ represents a single bond that bonds to N of formula (I), optional two of adjacent substituents selected from the group of $R^a$, $R^b$, $R^c$ and $R^d$ form a substituted or unsubstituted condensed ring system, wherein<br>at least one substituent on the condensed ring system is independently selected from $C_1$ to $C_6$ alkyl, $C_6$ to $C_{12}$ aryl or $C_2$ to $C_{12}$ heteroaryl; |
| $R^{e2}$, $R^{f2}$, $R^{g2}$ and $R^{h2}$ | are independently selected from H, $C_1$ to $C_6$ alkyl, $C_6$ to $C_{18}$ aryl, and $C_2$ to $C_{18}$ heteroaryl; |
| $X^2$ | is selected from O, S, $NAr^{2b}$, $CR^{1b}R^{26}$, $SiR^{1b}R^2$ ; |
| $Ar^{2b}$ | is selected from biphenyl; and |
| $R^{1b}$ and $R^{2b}$ | are independently selected from $C_1$ to $C_6$ alkyl, $C_6$ to $C_{12}$ aryl or $C_2$ to $C_{12}$ heteroaryl. |

According to one embodiment, wherein the compound of formula (I) is represented by a compound of formula (Ic), (Id) or (Ie), wherein
formula (Ic) is:

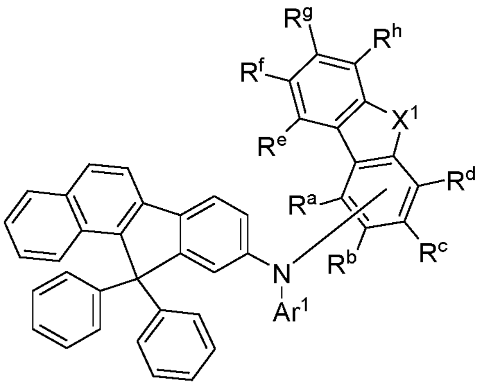

(Ic),

wherein

| | |
|---|---|
| $Ar^1$ | is selected from substituted or unsubstituted $C_6$ to $C_{24}$ aryl, or substituted or unsubstituted $C_5$ to $C_{25}$ heteroaryl, at least one substituent on $Ar^1$ is selected from $C_1$ to $C_6$ alkyl, $C_6$ to $C_{12}$ aryl, $C_5$ to $C_{12}$ heteroaryl; |
| $R^a$, $R^b$ , $R^c$ and $R^d$ | are independently selected from H, $C_1$ to $C_6$ alkyl, $C_6$ to $C_{18}$ aryl, and $C_2$ to $C_{18}$ heteroaryl, and one of the $R^a$, $R^b$, $R^c$ or $R^d$ represents a single bond that bonds to N of formula (I), optional two of adjacent substituents selected from the group of $R^a$, $R^b$, $R^c$ and $R^d$ form a substituted or unsubstituted condensed ring system, wherein at least one substituent on the condensed ring system is independently selected from $C_1$ to $C_6$ alkyl, $C_6$ to $C_{12}$ aryl or $C_2$ to $C_{12}$ heteroaryl; |
| $R^e$, $R^f$ , $R^g$, and $R^h$ | are independently selected from H, $C_1$ to $C_6$ alkyl, $C_6$ to $C_{18}$ aryl, and $C_2$ to $C_{18}$ heteroaryl; |
| $X^1$ | is selected from O, S, $NAr^{1a}$; |
| $Ar^{1a}$ | is selected from $C_6$ to $C_{12}$ aryl; or |

formula (Id) is:

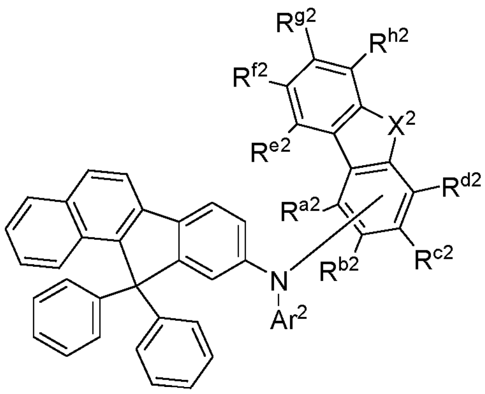

(Id),

wherein

| | |
|---|---|
| $Ar^2$ | is selected from $C_6$ to $C_{12}$ aryl or $C_5$ to $C_{25}$ heteroaryl; |
| $R^{a2}$, $R^{b2}$ , $R^{c2}$ and $R^{d2}$ | are independently selected from H, $C_1$ to $C_6$ alkyl, $C_6$ to $C_{18}$ aryl, and $C_2$ to $C_{18}$ heteroaryl, and one of the $R^{a2}$, $R^{b2}$, $R^{c2}$ or $R^{d2}$ represents a single bond that bonds to N of formula (I), optional two of adjacent substituents selected from the group of $R^{a2}$, $R^{b2}$, $R^{c2}$ and $R^{d2}$ form a substituted or unsubstituted condensed ring system, wherein at least one substituent on the condensed ring system is independently selected from $C_1$ to $C_6$ alkyl, $C_6$ to $C_{12}$ aryl or $C_2$ to $C_{12}$ heteroaryl; |
| $R^{e2}$, $R^{f2}$, $R^{g2}$, and $R^{h2}$ | are independently selected from H, $C_1$ to $C_6$ alkyl, $C_6$ to $C_{18}$ aryl, and $C_2$ to $C_{18}$ heteroaryl; |
| $X^2$ | is selected from O, S, $NAr^{2b}$, $CR^{1b}R^{26}$, $SiR^{1b}R^{2b}$; |
| $Ar^{2b}$ | is selected from $C_6$ to $C_{12}$ aryl; and |

$R^{1b}$ and $R^{2b}$ are independently selected from $C_1$ to $C_6$ alkyl, $C_6$ to $C_{12}$ aryl or $C_2$ to $C_{12}$ heteroaryl; or

formula (Ie) is:

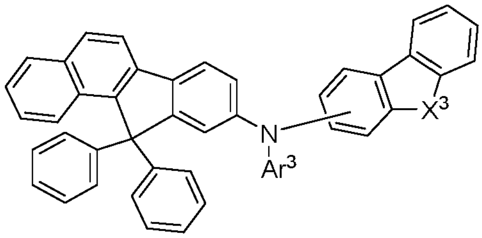

(Ie),

wherein

| | |
|---|---|
| $Ar^3$ | is selected from biphenyl, |
| $x^3$ | is selected from O, S, $NAr^{2b}$ , $CR^{1b}R^{2b}$; |
| $Ar^{2b}$ | is selected from $C_6$ to $C_{12}$ aryl; and |
| $R^{1b}$ and $R^{2b}$ | are independently selected from $C_1$ to $C_6$ alkyl, $C_6$ to $C_{12}$ aryl or $C_2$ to $C_{12}$ heteroaryl. |

According to one embodiment, wherein the compound of formula (I) is represented by a compound of formula (Ic), (Id) or (Ie), wherein
formula (Ic) is:

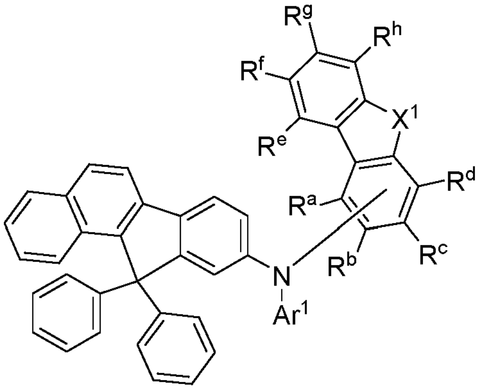

(Ic),

wherein

| | |
|---|---|
| $Ar^1$ | is selected from an unsubstituted $C_6$ to $C_{24}$ aryl, or unsubstituted $C_5$ to $C_{25}$ heteroaryl; |
| $R^a$, $R^b$ , $R^c$ and $R^d$ | are independently selected from H, $C_1$ to $C_6$ alkyl, $C_6$ to $C_{18}$ aryl, and $C_2$ to $C_{18}$ heteroaryl, and one of the $R^a$, $R^b$, $R^c$ or $R^d$ represents a single bond that bonds to N of formula (I), optional two of adjacent substituents selected from the group of $R^a$, $R^b$, $R^c$ and $R^d$ form an unsubstituted condensed ring system; |
| $R^e$, $R^f$, $R^g$, and $R^h$ | are independently selected from H, $C_1$ to $C_6$ alkyl, $C_6$ to $C_{18}$ aryl, and $C_2$ to $C_{18}$ heteroaryl; |
| $X^1$ | is selected from O, S, $Nar^{1a}$; |
| $Ar^{1a}$ | is selected from $C_6$ to $C_{12}$ aryl; or |

formula (Id) is:

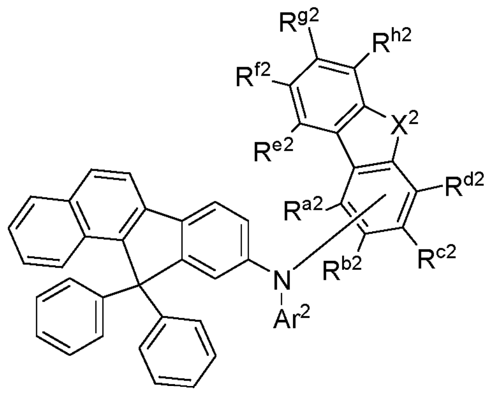

(Id),

wherein

| | |
|---|---|
| $Ar^2$ | is selected from $C_6$ to $C_{12}$ aryl or $C_5$ to $C_{25}$ heteroaryl; |
| $R^{a2}$, $R^{b2}$, $R^{c2}$ and $R^{d2}$ | are independently selected from H, $C_1$ to $C_6$ alkyl, $C_6$ to $C_{18}$ aryl, and $C_2$ to $C_{18}$ heteroaryl, and one of the $R^{a2}$, $R^{b2}$, $R^{c2}$ or $R^{d2}$ represents a single bond that bonds to N of formula (Ib), optional two of adjacent substituents selected from the group of $R^{a2}$, $R^{b2}$, $R^{c2}$ and $R^{d2}$ form an unsubstituted condensed ring system; |
| $R^{e2}$, $R^{f2}$, $R^{g2}$, and $R^{h2}$ | are independently selected from H, $C_1$ to $C_6$ alkyl, $C_6$ to $C_{18}$ aryl, and $C_2$ to $C_{18}$ heteroaryl; |
| $X^2$ | is selected from O, S, $NAr^{2b}$, $CR^{1b}R^{26}$, $SiR^{1b}R^{2b}$; |
| $Ar^{2b}$ | is selected from $C_6$ to $C_{12}$ aryl; and |
| $R^{1b}$ and $R^{2b}$ | are independently selected from $C_1$ to $C_6$ alkyl, $C_6$ to $C_{12}$ aryl or $C_2$ to $C_{12}$ heteroaryl; |

or formula (Ie) is:

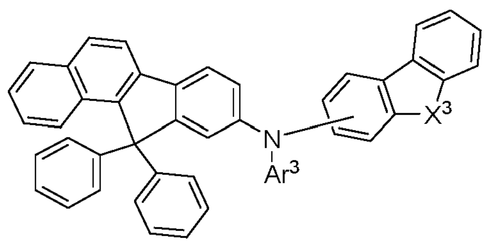

(Ie),

wherein

| | |
|---|---|
| $Ar^3$ | is selected from biphenyl, |
| $X^3$ | is selected from O, S, $NAr^{2b}$, $CR^{1b}R^2$ ; |
| $Ar^{2b}$ | is selected from $C_6$ to $C_{12}$ aryl; and |
| $R^{1b}$ and $R^{2b}$ | are independently selected from $C_1$ to $C_6$ alkyl, $C_6$ to $C_{12}$ aryl or $C_2$ to $C_{12}$ heteroaryl. |

**$Ar^1$**

According to an embodiment, $Ar^1$ can be same or individual of each other selected from $C_6$ to $C_{24}$ aryl, $C_6$ to $C_{22}$ aryl, $C_6$ to $C_{20}$ aryl, $C_6$ to $C_{18}$ aryl, $C_6$ to $C_{16}$ aryl, $C_6$ to $C_{14}$ aryl, or $C_6$ to $C_{13}$ aryl. According to an embodiment, $Ar^1$ can be same or individual of each other selected from $C_5$ to $C_{25}$ heteroaryl, $C_5$ to $C_{23}$ heteroaryl, $C_5$ to $C_{21}$ heteroaryl, $C_5$ to $C_{19}$ heteroaryl, $C_5$ to $C_{17}$ heteroaryl, $C_5$ to $C_{15}$ heteroaryl, $C_5$ to $C_{13}$ heteroaryl, or $C_5$ to $C_{12}$ heteroaryl. According to an embodiment, $Ar^1$ may be selected from substituted or unsubstituted $C_6$ to $C_{13}$ aryl, and substituted or unsubstituted $C_{12}$ heteroaryl. According to an embodiment, $Ar^1$ is selected from substituted $C_6$ to $C_{13}$ aryl, and substituted or unsubstituted $C_{12}$ heteroaryl. According to an embodiment, $Ar^1$ is selected from substituted $C_6$ to $C_{13}$ aryl, and substituted $C_{12}$ heteroaryl. According to an embodiment, $Ar^1$ is selected from substituted $C_6$ to $C_{13}$ aryl. According to another embodiment, the substituent on $Ar^1$ can be same or individual of each other selected from $C_1$ to $C_6$ alkyl, $C_1$ to $C_5$ alkyl, preferably $C_1$ to $C_4$ alkyl, more preferably $C_1$ to $C_3$ alkyl, still more preferably $C_1$ to $C_2$ alkyl, or most preferably $C_1$ alkyl. According to an embodiment, the at least one substituent on $Ar^1$ can be same or individual of each other selected from $C_6$ to $C_{12}$ aryl, $C_6$ to $C_{10}$ aryl or $C_6$ aryl. According to an embodiment, the at least one substituent on $Ar^1$ can be same or individual of each other

selected from $C_5$ to $C_{12}$ heteroaryl, $C_5$ to $C_{11}$ heteroaryl, $C_5$ to $C_{10}$ heteroaryl, or $C_5$ to $C_9$ heteroaryl. According to an embodiment, the at least one substituent on $Ar^1$ is selected from $C_1$ to $C_6$ alkyl.

**$Ar^2$**

According to an embodiment, wherein $Ar^2$ can be selected from $C_5$ to $C_{25}$ heteroaryl, $C_5$ to $C_{23}$ heteroaryl, $C_5$ to $C_{21}$ heteroaryl, $C_5$ to $C_{19}$ heteroaryl, $C_5$ to $C_{17}$ heteroaryl, $C_5$ to $C_{15}$ heteroaryl, $C_5$ to $C_{13}$ heteroaryl, or $C_5$ to $C_{12}$ heteroaryl.

According to an embodiment, wherein $Ar^2$ can be selected from $C_6$ to $C_{12}$ aryl, $C_{10}$ to $C_{12}$ aryl, and more preferably $C_{12}$ aryl. According to an embodiment, $Ar^2$ is selected from $C_6$ to $C_{10}$ aryl. According to an embodiment, $Ar^2$ is selected from $C_6$ aryl. According to an embodiment, $Ar^2$ can be selected from biphenyl, and napthyl. According to an embodiment, $Ar^2$ can be selected from biphenyl.

**$A_{r2b}$**

According to an embodiment, $Ar^{2b}$ is selected from $C_6$ to $C_{10}$ aryl. According to an embodiment, $Ar^{2b}$ is selected from $C_6$ aryl.

**$X^1$**

According to an embodiment, wherein $X^1$ is selected from O, S, $NAr^{1b}$. According to an embodiment, wherein $X^1$ is selected from O or $NAR^{1b}$. According to an embodiment, wherein $X^1$ is selected from O.

According to an embodiment, wherein $X^1$ is selected from O, S, $NAr^{2b}$, $CR^{1b}R^{2b}$ and $SiR^{1b}R^{2b}$. According to an embodiment, wherein $X^1$ is selected from O, S, $NAr^2$. According to an embodiment, wherein $X^1$ is selected from O or $NAR^2$. According to an embodiment, wherein X is selected from O.

**$X^2$**

According to an embodiment, wherein $X^2$ is selected from O, $NAR^{2b}$, $CR^{1b}R^{2b}$ and $SiR^{1b}R^{2b}$. According to an embodiment, wherein $X^2$ is selected from O, $NAR^{2b}$ and $CR^{1b}R^{2b}$. According to an embodiment, wherein $X^2$ is selected from O, and $CR^{1b}R^{2b}$. According to an embodiment, wherein $X^2$ is selected from $CR^{1b}R^{2b}$.

**$X^3$**

According to an embodiment, wherein $X^3$ is selected from O, S, $NAr^{2b}$, $CR^{1b}R^{2b}$. According to an embodiment, wherein $X^3$ is selected from O, and $CR^{1b}R^{2b}$. According to an embodiment, wherein $X^3$ is selected from $CR^{1b}R^{2b}$.

**$X^{1b}$**

According to an embodiment, wherein $X^2$ is selected from O, $NAR^{2b}$, $CR^{1b}R^{2b}$ and $SiR^{1b}R^{2b}$. According to an embodiment, wherein $X^2$ is selected from O, $NAR^{2b}$ and $CR^{1b}R^{2b}$. According to an embodiment, wherein $X^2$ is selected from O, and $CR^{1b}R^{2b}$. According to an embodiment, wherein $X^2$ is selected from $CR^{1b}R^{2b}$.

**$R^{1b}$ and $R^{2b}$**

According to an embodiment, $R^{1b}$ and $R^{2b}$ can be same or individual of each other selected from $C_1$ to $C_6$ alkyl, $C_1$ to $C_5$ alkyl, preferably $C_1$ to $C_4$ alkyl, more preferably $C_1$ to $C_3$ alkyl, still more preferably $C_1$ to $C_2$ alkyl, or most preferably $C_1$ alkyl. According to an embodiment, $R^{1b}$ and $R^{2b}$ can be same or individual of each other selected from $C_6$ to $C_{12}$ aryl, $C_6$ to $C_{10}$ aryl, and preferably $C_6$ alkyl. According to an embodiment, $R^{1b}$ and $R^{2b}$ can be same or individual of each other selected from $C_2$ to $C_{12}$ heteroaryl, $C_3$ to $C_{12}$ heteroaryl, $C_4$ to $C_{12}$ heteroaryl, $C_5$ to $C_{12}$ heteroaryl, $C_5$ to $C_{11}$ heteroaryl, $C_5$ to $C_{10}$ heteroaryl, or $C_5$ to $C_9$ heteroaryl. According to an embodiment, $R^{16}$ and $R^{2b}$ are independently selected from $C_1$ to $C_6$ alkyl, $C_1$ preferably to $C_5$ alkyl, more preferably $C_1$ to $C_4$ alkyl, more preferably $C_1$ to $C_3$ alkyl, still more preferably $C_1$ to $C_2$ alkyl, or most preferably $C_1$ alkyl.

**$R^a$, $R^b$, $R^c$ and $R^d$**

According to an embodiment, $R^a$, $R^b$, $R^c$ and $R^d$ can be same or individual of each other selected from $C_1$ to $C_6$ alkyl, $C_1$ to $C_5$ alkyl, $C_1$ to $C_4$ alkyl, $C_1$ to $C_3$ alkyl, $C_1$ to $C_2$ alkyl, or $C_1$ alkyl. According to an embodiment, $R^a$, $R^b$, $R^c$ and $R^d$ can be same or individual of each other selected from $C_6$ to $C_{18}$ aryl, $C_6$ to $C_{16}$ aryl, $C_6$ to $C_{14}$ aryl, $C_6$ to $C_{12}$ aryl, $C_6$ to $C_{10}$

aryl or $C_6$ aryl. According to an embodiment, $R^a$, $R^b$, $R^c$ and $R^d$ can be same or individual of each other selected from $C_2$ to $C_{18}$ heteroaryl, $C_3$ to $C_{18}$ heteroaryl, $C_4$ to $C_{18}$ heteroaryl, $C_5$ to $C_{18}$ heteroaryl, $C_5$ to $C_{17}$ heteroaryl, $C_5$ to $C_{16}$ heteroaryl, $C_5$ to $C_{15}$ heteroaryl, $C_5$ to $C_{14}$ heteroaryl, $C_5$ to $C_{13}$ heteroaryl, $C_5$ to $C_{12}$ heteroaryl, $C_5$ to $C_{11}$ heteroaryl, $C_5$ to $C_{10}$ heteroaryl, or $C_5$ to $C_{19}$ heteroaryl. According to an embodiment, $R^a$, $R^b$, $R^c$ and $R^d$ can be same or individual of each other selected from H, $C_1$ to $C_6$ alkyl, and $C_6$ to $C_{18}$ aryl.

**$R^e$, $R^f$, $R^g$, and $R^h$**

According to an embodiment, $R^e$, $R^f$, $R^g$, and $R^h$ can be same or individual of each other selected from $C_1$ to $C_6$ alkyl, $C_1$ to $C_5$ alkyl, $C_1$ to $C_4$ alkyl, $C_1$ to $C_3$ alkyl, $C_1$ to $C_2$ alkyl, or $C_1$ alkyl. According to an embodiment, $C_6$ to $C_{18}$ aryl of $R^e$, $R^f$, $R^g$, and $R^h$ can be same or individual of each other selected from $C_6$ to $C_{16}$ aryl, $C_6$ to $C_{14}$ aryl, $C_6$ to $C_{12}$ aryl, $C_6$ to $C_{10}$ aryl or $C_6$ aryl. According to an embodiment, $R^e$, $R^f$, $R^g$, and $R^h$ can be same or individual of each other selected from $C_2$ to $C_{18}$ heteroaryl, $C_3$ to $C_{18}$ heteroaryl, $C_4$ to $C_{18}$ heteroaryl, $C_5$ to $C_{18}$ heteroaryl, $C_5$ to $C_{17}$ heteroaryl, $C_5$ to $C_{16}$ heteroaryl, $C_5$ to $C_{15}$ heteroaryl, $C_5$ to $C_{14}$ heteroaryl, $C_5$ to $C_{13}$ heteroaryl, $C_5$ to $C_{12}$ heteroaryl, $C_5$ to $C_{11}$ heteroaryl, $C_5$ to $C_{10}$ heteroaryl, or $C_5$ to $C_{19}$ heteroaryl. According to an embodiment, $R^e$, $R^f$, $R^g$, and $R^h$ are independently selected from H, $C_6$ to $C_{18}$ aryl, and $C_2$ to $C_{18}$ heteroaryl. According to an embodiment, $R^e$, $R^f$, $R^g$, and $R^h$ are independently selected from H, and $C_6$ to $C_{18}$ aryl. According to an embodiment, $R^e$, $R^f$, $R^g$, and $R^h$ are independently selected from H, $C_1$ to $C_6$ alkyl, $C_6$ to $C_{18}$ aryl, and $C_2$ to $C_{18}$ heteroaryl.

**$R^{a2}$, $R^{b2}$, $R^{c2}$ and $R^{d2}$**

According to an embodiment, $R^{a2}$, $R^{b2}$, $R^{c2}$ and $R^{d2}$ can be same or individual of each other selected from $C_1$ to $C_6$ alkyl, $C_1$ to $C_5$ alkyl, $C_1$ to $C_4$ alkyl, $C_1$ to $C_3$ alkyl, $C_1$ to $C_2$ alkyl, or $C_1$ alkyl. According to an embodiment, $R^{a2}$, $R^{b2}$, $R^{c2}$ and $R^{d2}$ can be same or individual of each other selected from $C_6$ to $C_{18}$ aryl, $C_6$ to $C_{16}$ aryl, $C_6$ to $C_{14}$ aryl, $C_6$ to $C_{12}$ aryl, $C_6$ to $C_{10}$ aryl or $C_6$ aryl. According to an embodiment, $R^{a2}$, $R^{b2}$, $R^{c2}$ and $R^{d2}$ can be same or individual of each other selected from $C_2$ to $C_{18}$ heteroaryl, $C_3$ to $C_{18}$ heteroaryl, $C_4$ to $C_{18}$ heteroaryl, $C_5$ to $C_{18}$ heteroaryl, $C_5$ to $C_{17}$ heteroaryl, $C_5$ to $C_{16}$ heteroaryl, $C_5$ to $C_{15}$ heteroaryl, $C_5$ to $C_{14}$ heteroaryl, $C_5$ to $C_{13}$ heteroaryl, $C_5$ to $C_{12}$ heteroaryl, $C_5$ to $C_{11}$ heteroaryl, $C_5$ to $C_{10}$ heteroaryl, or $C_5$ to $C_{19}$ heteroaryl. According to an embodiment, $R^a$, $R^b$, $R^c$ and $R^d$ are independently selected from H, $C_1$ to $C_6$ alkyl, and $C_6$ to $C_{18}$ aryl.

**$R^{e2}$, $R^{f2}$, $R^{g2}$, and $R^{h2}$**

According to an embodiment, $R^{e2}$, $R^{f2}$, $R^{g2}$, and $R^{h2}$ can be same or individual of each other selected from $C_1$ to $C_6$ alkyl, $C_1$ to $C_5$ alkyl, $C_1$ to $C_4$ alkyl, $C_1$ to $C_3$ alkyl, $C_1$ to $C_2$ alkyl, or $C_1$ alkyl. According to an embodiment, $R^{e2}$, $R^{f2}$, $R^{g2}$, and $R^{h2}$ can be same or individual of each other selected from $C_6$ to $C_{18}$ aryl, $C_6$ to $C_{16}$ aryl, $C_6$ to $C_{14}$ aryl, $C_6$ to $C_{12}$ aryl, $C_6$ to $C_{10}$ aryl or $C_6$ aryl. According to an embodiment, $R^{e2}$, $R^{f2}$, $R^{g2}$, and $R^{h2}$ can be same or individual of each other selected from $C_2$ to $C_{18}$ heteroaryl, $C_3$ to $C_{18}$ heteroaryl, $C_4$ to $C_{18}$ heteroaryl, $C_5$ to $C_{18}$ heteroaryl, $C_5$ to $C_{17}$ heteroaryl, $C_5$ to $C_{16}$ heteroaryl, $C_5$ to $C_{15}$ heteroaryl, $C_5$ to $C_{14}$ heteroaryl, $C_5$ to $C_{13}$ heteroaryl, $C_5$ to $C_{12}$ heteroaryl, $C_5$ to $C_{11}$ heteroaryl, $C_5$ to $C_{10}$ heteroaryl, or $C_5$ to $C_{19}$ heteroaryl. According to an embodiment, $R^{e2}$, $R^{f2}$, $R^{g2}$, and $R^{h2}$ are independently selected from H, $C_6$ to $C_{18}$ aryl, and $C_2$ to $C_{18}$ heteroaryl. According to an embodiment, $R^{e2}$, $R^{f2}$, $R^{g2}$, and $R^{h2}$ are independently selected from H, and $C_6$ to $C_{18}$ aryl.

**Substituent on the condensed ring system**

According to an embodiment, the at least one substituent on the condensed ring system in case of formula (Ia), (Ib), (Ic), (Id), (If), (Ig), (Ih) and (Ik) can individual of each other selected from $C_1$ to $C_6$ alkyl, $C_1$ to $C_5$ alkyl, preferably $C_1$ to $C_4$ alkyl, more preferably $C_1$ to $C_3$ alkyl, still more preferably $C_1$ to $C_2$ alkyl, and most preferably $C_1$ alkyl. According to an embodiment, the at least one substituent on the condensed ring system is selected from $C_1$ to $C_6$ alkyl.

**Formulae (Ic), (Id), (Ie)**

According to an embodiment of the benzo diphenyl fluorene compound, wherein for formula (Ic) $X^1$ is selected from O or $NAR^2$, preferably O.

According to an embodiment of the benzo diphenyl fluorene compound, wherein for formula (Id) $X^2$ is selected from O, $NAR^{2b}$, $CR^{1b}R^{2b}$ and $SiR^{1b}R^{2b}$, preferably selected from O, and $CR^{1b}R^{2b}$, and in addition preferred selected from $CR^{1b}R^{2b}$.

According to an embodiment of the benzo diphenyl fluorene compound, wherein for formula (Ie) $X^3$ is selected from O, S and $SiR^{1b}R^{2b}$, preferably selected from O and S, and in addition preferred selected from O.

According to an embodiment of the benzo diphenyl fluorene compound, wherein for formula (Ic) $X^1$ is selected from O or $NAR^2$, preferably O; and for formula (Id) $X^2$ is selected from O, $NAR^{2b}$, $CR^{1b}R^{2b}$ and $SiR^{1b}R^{2b}$, preferably selected from O, and $CR^{1b}R^{2b}$, and in addition preferred selected from $CR^{1b}R^{2b}$; and for formula (Ie) $X^3$ is selected from O, S and $SiR^{1b}R^{2b}$, preferably selected from O and S, and in addition preferred selected from O.

According to an embodiment of the benzo diphenyl fluorene compound, wherein for formula (Ic) $Ar^1$ is selected from substituted or unsubstituted $C_6$ to $C_{13}$ aryl, and substituted or unsubstituted $C_{12}$ heteroaryl, preferably $Ar^1$ is selected from substituted $C_6$ to $C_{13}$ aryl.

According to an embodiment of the benzo diphenyl fluorene compound, wherein for formula (Id) $Ar^2$ is selected from $C_6$ to $C_{10}$ aryl, and preferably $Ar^2$ is selected from $C_6$ aryl.

According to an embodiment of the benzo diphenyl fluorene compound, wherein for formula (Ic) $Ar^1$ is selected from substituted or unsubstituted $C_6$ to $C_{13}$ aryl, and substituted or unsubstituted $C_{12}$ heteroaryl, preferably $Ar^1$ is selected from substituted $C_6$ to $C_{13}$ aryl; and for formula (Id) $Ar^2$ is selected from $C_6$ to $C_{10}$ aryl, and preferably $Ar^2$ is selected from $C_6$ aryl.

**Ar, $Ar^1$ and/or $Ar^2$**

According to an embodiment of the benzo diphenyl fluorene compound, wherein Ar, $Ar^1$ and/or $Ar^2$ are selected from the group of B1 to B13:

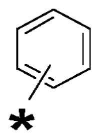 (B1), 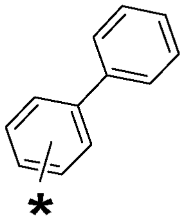 (B2), 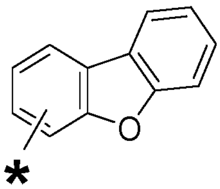 (B3), 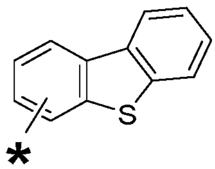 (B4),

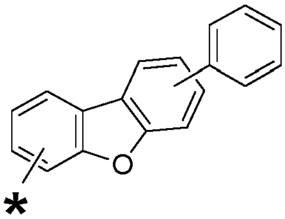 (B5), 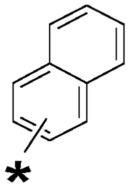 (B6), 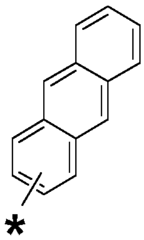 (B7),

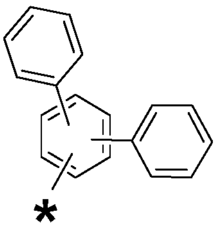 (B8), 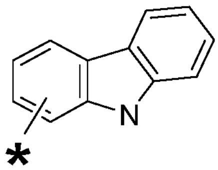 (B9), 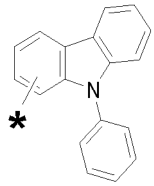 (B10),

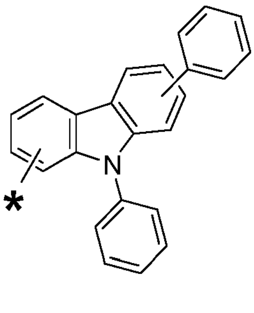 (B11), 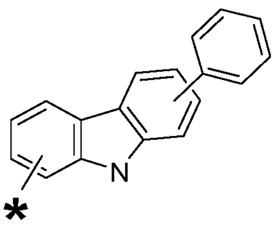 (B12);

wherein the asterix "*" denotes the binding position of Ar, $Ar^1$ and/or $Ar^2$.

According to an embodiment of the benzo diphenyl fluorene compound, wherein $Ar^1$ is selected from the group of B1 to B12, preferably selected from the group of B1, B2, B3, B4, B5, B6, and in addition preferred selected from the group of B1, B2, B3.

According to an embodiment of the benzo diphenyl fluorene compound, wherein $Ar^2$ is selected from the group of B1 to B12, preferably selected from the group of B1, B2, B3, B4, B5, B6, and in addition preferred selected from the group of B1, B2, B3.

According to an embodiment of the benzo diphenyl fluorene compound, wherein $Ar^1$ is selected from the group of B1 to B12, preferably selected from the group of B1, B2, B3, B4, B5, B6, and in addition preferred selected from the group of B1, B2, B3; and/or

$Ar^2$ is selected from the group of B1 to B12, preferably selected from the group of B1, B2, B3, B4, B5, B6, and in addition preferred selected from the group of B1, B2, B3.

**$Ar^4$**

According to an embodiment of the benzo diphenyl fluorene compound, wherein $Ar^4$ is selected from the group of D1 to D7:

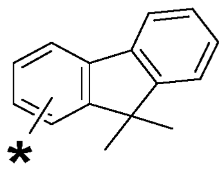
(D1),
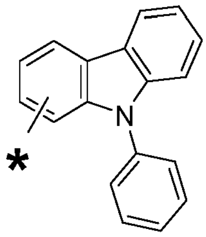
(D2),
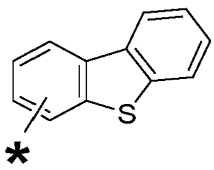
(D3),
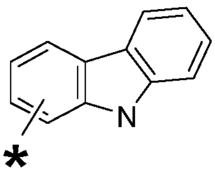
(D4),
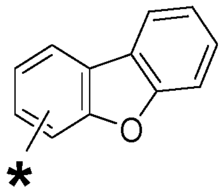
(D5),
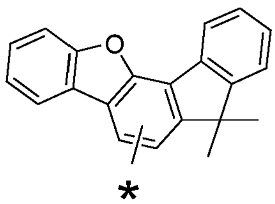
(D6),
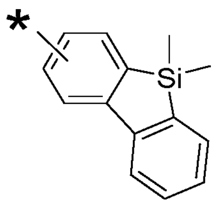
(D7);

wherein the asterix "*" denotes the binding position of $Ar^4$.

**Formula If**

According to an embodiment of the benzo diphenyl fluorene compound of formula I is represented by formula If:

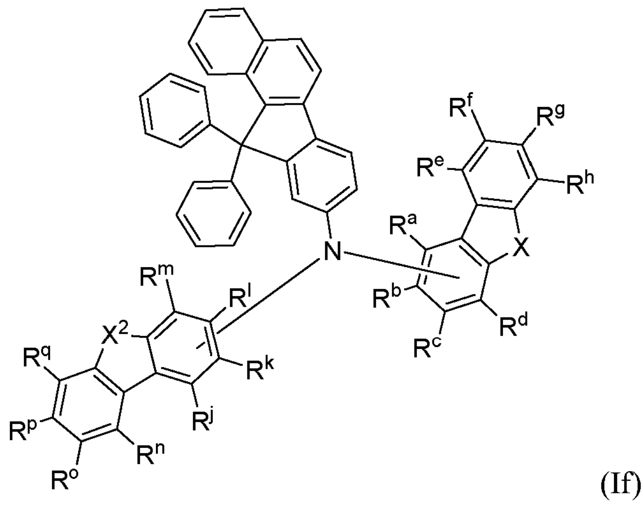

(If)

wherein

| | |
|---|---|
| $R^j$, $R^k$, $R^l$, $R^m$, $R^n$, $R^o$, $R^p$ and $R^q$ | are independently selected from H, $C_1$ to $C_6$ alkyl, $C_6$ to $C_{12}$ aryl, $C_5$ to $C_{12}$ heteroaryl; |
| $R^j$, $R^k$, $R^1$ and $R^m$ | are independently represent a single bond if N of formula (If) is bonded to $R^j$, $R^k$, $R^l$ and $R^m$, respectively; |
| $R^a$, $R^b$, $R^c$ and $R^d$ | are independently selected from H, $C_1$ to $C_6$ alkyl, $C_6$ to $C_{18}$ aryl, and $C_2$ to $C_{18}$ heteroaryl, |
| $R^a$, $R^b$, $R^c$ and $R^d$ | are independently represent a single bond if N of formula (If) is bonded to $R^a$, $R^b$, $R^c$ and $R^d$, respectively; |

wherein

two of $R^a$, $R^b$, $R^c$ and $R^d$ can form a substituted or unsubstituted condensed ring system when two of $R_a$, $R_b$, $R_c$ and $R_d$ are adjacent to each other;
at least one substituent on the condensed ring system is independently selected from $C_1$ to $C_6$ alkyl, $C_6$ to $C_{12}$ aryl or $C_2$ to $C_{12}$ heteroaryl;
$R^e$, $R^f$, $R^g$, and $R^h$ are independently selected from H, $C_1$ to $C_6$ alkyl, $C_6$ to $C_{18}$ aryl, and $C_2$ to $C_{18}$ heteroaryl;
X is selected from O, S, $NAr^2$;
$Ar^2$ is selected from $C_6$ to $C_{12}$ aryl;
$X^2$ is selected from O, S, $NAr^3$, $CR^3R^4$ and $SiR^3R^4$;
$R^3$ and $R^4$ are independently selected from $C_1$ to $C_6$ alkyl, $C_6$ to $C_{12}$ aryl or $C_2$ to $C_{12}$ heteroaryl; and
$Ar^3$ is selected from $C_6$ to $C_{12}$ aryl.

**$R^j$, $R^k$, $R^l$, $R^m$, $R^n$, $R^o$, $R^p$ and $R^q$ of formula (If)**

According to an embodiment, $C_1$ to $C_6$ alkyl of $R^j$, $R^k$, $R^l$, $R^m$, $R^n$, $R^o$, $R^p$ and $R^q$ can be $C_1$ to $C_5$ alkyl, $C_1$ to $C_4$ alkyl, $C_1$ to $C_3$ alkyl, $C_1$ to $C_2$ alkyl, or $C_1$ alkyl.
According to an embodiment, $C_6$ to $C_{18}$ aryl of $R^j$, $R^k$, $R^l$, $R^m$, $R^n$, $R^o$, $R^p$ and $R^q$ can be $C_6$ to $C_{16}$ aryl, $C_6$ to $C_{14}$ aryl, $C_6$ to $C_{12}$ aryl, $C_6$ to $C_{10}$ aryl or $C_6$ aryl.
According to an embodiment, $C_2$ to $C_{18}$ heteroaryl of $R^j$, $R^k$, $R^l$, $R^m$, $R^n$, $R^o$, $R^p$ and $R^q$ can be $C_3$ to $C_{18}$ heteroaryl, $C_4$ to $C_{18}$ heteroaryl, $C_5$ to $C_{18}$ heteroaryl, $C_5$ to $C_{17}$ heteroaryl, $C_5$ to $C_{16}$ heteroaryl, $C_5$ to $C_{15}$ heteroaryl, $C_5$ to $C_{14}$ heteroaryl, $C_5$ to $C_{13}$ heteroaryl, $C_5$ to $C_{12}$ heteroaryl, $C_5$ to $C_{11}$ heteroaryl, $C_5$ to $C_{10}$ heteroaryl, or $C_5$ to $C_{19}$ heteroaryl.
According to an embodiment, $R^j$, $R^k$, $R^l$, $R^m$, $R^n$, $R^o$, $R^p$ and $R^q$ are independently selected from H, $C_1$ to $C_6$ alkyl, and $C_6$ to $C_{18}$ aryl.

**X of formula (If)**

According to an embodiment, wherein X is selected from O, S, $NAr^2$.

**$X^2$ of formula (If)**

According to an embodiment, wherein $X^2$ is selected from O, $NAr^3$, $CR^3R^4$ and $SiR^3R^4$. According to an embodiment, wherein $X^2$ is selected from O, $CR^3R^4$ and $SiR^3R^4$. According to an embodiment, wherein $X^2$ is selected from $CR^3R^4$ and $SiR^3R^4$. According to an embodiment, wherein $X^2$ is selected from $CR^3R^4$

**$R^3$ and $R^4$ of formula (If)**

According to an embodiment, $C_1$ to $C_6$ alkyl of $R^3$ and $R^4$ can be $C_1$ to $C_5$ alkyl, preferably $C_1$ to $C_4$ alkyl, more preferably $C_1$ to $C_3$ alkyl, still more preferably $C_1$ to $C_2$ alkyl, or most preferably $C_1$ alkyl. According to an embodiment, $C_6$ to $C_{12}$ aryl of $R^3$ and $R^4$ can be $C_6$ to $C_{10}$ aryl, and preferably $C_6$ alkyl. According to an embodiment, $C_2$ to $C_{12}$ heteroaryl of $R^3$ and $R^4$ can be $C_3$ to $C_{12}$ heteroaryl, $C_4$ to $C_{12}$ heteroaryl, $C_5$ to $C_{12}$ heteroaryl, $C_5$ to $C_{11}$ heteroaryl, $C_5$ to $C_{10}$ heteroaryl, or $C_5$ to $C_9$ heteroaryl. According to an embodiment, $R^3$ and $R^4$ are independently selected from $C_1$ to $C_6$ alkyl, $C_1$ preferably to $C_5$ alkyl, more preferably $C_1$ to $C_4$ alkyl, more preferably $C_1$ to $C_3$ alkyl, still more preferably $C_1$ to $C_2$ alkyl, or most preferably $C_1$ alkyl.

**$Ar^3$ of formula (If)**

According to an embodiment, $Ar^3$ is selected from $C_6$ to $C_{10}$ aryl. According to an embodiment, $Ar^3$ is selected from $C_6$ aryl.

**Formula (Ig)**

According to an embodiment, the compound of formula I is represented by formula Ig:

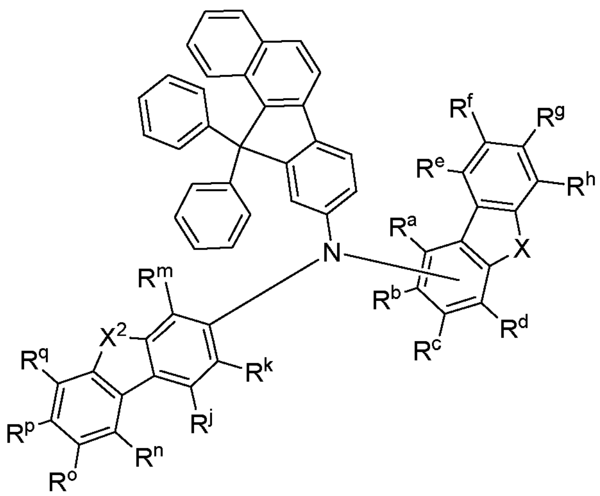

(Ig),

wherein

| | |
|---|---|
| $R^j$, $R^k$, $R^l$, $R^m$, $R^n$, $R^o$, $R^p$ and $R^q$ | are independently selected from H, $C_1$ to $C_6$ alkyl, $C_6$ to $C_{12}$ aryl, $C_5$ to $C_{12}$ heteroaryl; |
| $R^j$, $R^k$, $R^l$ and $R^m$ | are independently represent a single bond if N of formula (Ig) is bonded to $R^j$, $R^k$, $R^l$ and $R^m$, respectively; |
| $R^a$, $R^b$, $R^c$ and $R^d$ | are independently selected from H, $C_1$ to $C_6$ alkyl, $C_6$ to $C_{18}$ aryl, and $C_2$ to $C_{18}$ heteroaryl, |
| $R^a$, $R^b$, $R^c$ and $R^d$ | are independently represent a single bond if N of formula (Ig) is bonded to $R^a$, $R^b$, $R^c$ and $R^d$, respectively; |

two of $R^a$, $R^b$, $R^c$ and $R^d$ can form a substituted or unsubstituted condensed ring system when two of $R^a$, $R^b$, $R^c$ and $R_d$ are adjacent to each other;
the at least one substituent on the condensed ring system is independently selected from $C_1$ to $C_6$ alkyl, $C_6$ to $C_{12}$ aryl or $C_2$ to $C_{12}$ heteroaryl;

| | |
|---|---|
| $R^e$, $R^f$, $R^g$, and $R^h$ | are independently selected from H, $C_1$ to $C_6$ alkyl, $C_6$ to $C_{18}$ aryl, and $C_2$ to $C_{18}$ heteroaryl; |
| X | is selected from O, S, $NAr^2$; |
| $Ar^2$ | is selected from $C_6$ to $C_{12}$ aryl; |
| $X^2$ | is selected from O, S, $NAr^2$, $CR^3R^4$ and $SiR^3R^4$; |
| $R^3$ and $R^4$ | are independently selected from $C_1$ to $C_6$ alkyl, $C_6$ to $C_{12}$ aryl or $C_2$ to $C_{12}$ heteroaryl; and |
| $Ar^3$ | is selected from $C_6$ to $C_{12}$ aryl. |

**$R^j$, $R^k$, $R^l$, $R^m$, $R^n$, $R^o$, $R^p$ and $R^q$ of formula (Ig)**

According to an embodiment, $C_1$ to $C_6$ alkyl of $R^j$, $R^k$, $R^l$, $R^m$, $R^n$, $R^o$, $R^p$ and $R^q$ can be $C_1$ to $C_5$ alkyl, $C_1$ to $C_4$ alkyl, $C_1$ to $C_3$ alkyl, $C_1$ to $C_2$ alkyl, or $C_1$ alkyl.

According to an embodiment, $C_6$ to $C_{12}$ aryl of $R^j$, $R^k$, $R^l$, $R^m$, $R^n$, $R^o$, $R^p$ and $R^q$ can be $C_6$ to $C_{10}$aryl, $C_6$ to $C_8$ aryl, or $C_6$ aryl.

According to an embodiment, $C_5$ to $C_{12}$ heteroaryl of $R^j$, $R^k$, $R^l$, $R^m$, $R^n$, $R^o$, $R^p$ and $R^q$ can be $C_5$ to $C_{16}$ heteroaryl, $C_5$ to $C_{14}$ heteroaryl, $C_5$ to $C_{12}$ heteroaryl, $C_5$ to $C_{10}$ heteroaryl, $C_5$ to $C_8$, or $C_5$ heteroaryl.

According to an embodiment, $R^j$, $R^k$, $R^l$, $R^m$, $R^n$, $R^o$, $R^p$ and $R^q$ are independently selected from H, $C_1$ to $C_6$ alkyl, $C_6$ to $C_{12}$ aryl, $C_5$ to $C_{12}$ heteroaryl.

**X of formula (Ig)**

According to an embodiment, wherein X is selected from O, S, $NAr^2$.

**$X^2$ of formula (Ig)**

According to an embodiment, wherein $X^2$ is selected from O, $NAr^3$, $CR^3R^4$ and $SiR^3R^4$. According to an embodiment, wherein $X^2$ is selected from O, $CR^3R^4$ and $SiR^3R^4$. According to an embodiment, wherein $X^2$ is selected from $CR^3R^4$ and $SiR^3R^4$. According to an embodiment, wherein $X^2$ is selected from $CR^3R^4$

**$R^3$ and $R^4$ of formula (Ig)**

According to an embodiment, $C_1$ to $C_6$ alkyl of $R^3$ and $R^4$ can be $C_1$ to $C_5$ alkyl, preferably $C_1$ to $C_4$ alkyl, more preferably $C_1$ to $C_3$ alkyl, still more preferably $C_1$ to $C_2$ alkyl, or most preferably $C_1$ alkyl. According to an embodiment, $C_6$ to $C_{12}$ aryl of $R^3$ and $R^4$ can be $C_6$ to $C_{10}$ aryl, and preferably $C_6$ alkyl. According to an embodiment, $C_2$ to $C_{12}$ heteroaryl of $R^3$ and $R^4$ can be $C_3$ to $C_{12}$ heteroaryl, $C_4$ to $C_{12}$ heteroaryl, $C_5$ to $C_{12}$ heteroaryl, $C_5$ to $C_{11}$ heteroaryl, $C_5$ to $C_{10}$ heteroaryl, or $C_5$ to $C_9$ heteroaryl. According to an embodiment, $R^3$ and $R^4$ are independently selected from $C_1$ to $C_6$ alkyl, $C_1$ preferably to $C_5$ alkyl, more preferably $C_1$ to $C_4$ alkyl, more preferably $C_1$ to $C_3$ alkyl, still more preferably $C_1$ to $C_2$ alkyl, or most preferably $C_1$ alkyl.

**$Ar^3$ of formula (Ig)**

According to an embodiment, $Ar^3$ is selected from $C_6$ to $C_{10}$ aryl. According to an embodiment, $Ar^3$ is selected from $C_6$ aryl.

**Formula Ih**

According to an embodiment, the compound of formula I is represented by formula Ih:

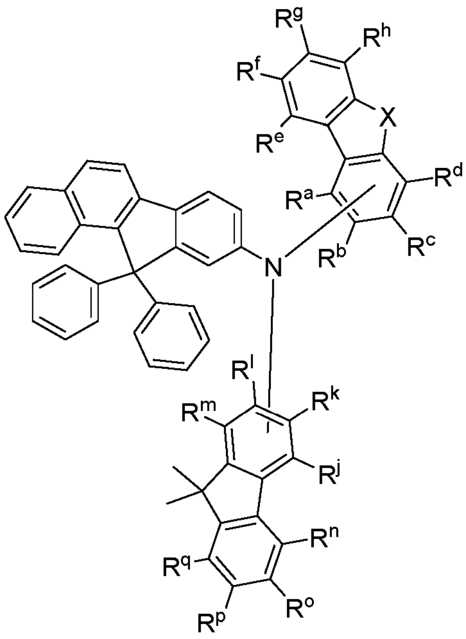

(Ih)

wherein

| | |
|---|---|
| $R^j$, $R^k$, $R^l$, $R^m$, $R^n$, $R^o$, $R^p$ and $R^q$ | are independently selected from H, $C_1$ to $C_6$ alkyl, $C_6$ to $C_{12}$ aryl, $C_5$ to $C_{12}$ heteroaryl; |
| $R^j$, $R^k$, $R^l$ and $R^m$ | are independently represent a single bond if N of formula (Ih) is bonded to $R^j$, $R^k$, $R^l$ and $R^m$, respectively; |
| $R^a$, $R^b$, $R^c$ and $R^d$ | are independently selected from H, $C_1$ to $C_6$ alkyl, $C_6$ to $C_{18}$ aryl, and $C_2$ to $C_{18}$ heteroaryl, |
| $R^a$, $R^b$, $R^c$ and $R^d$ | are independently represent a single bond if N of formula (Ih) is bonded to $R^a$, $R^b$, $R^c$ and $R^d$, respectively; |

two of $R^a$, $R^b$, $R^c$ and $R^d$ can form a substituted or unsubstituted condensed ring system when two of $R^a$, $R^b$, $R^c$ and $R^d$ are adjacent to each other;
the at least one substituent on the condensed ring system is independently selected from $C_1$ to $C_6$ alkyl, $C_6$ to $C_{12}$ aryl or $C_2$ to $C_{12}$ heteroaryl;

| | |
|---|---|
| $R^e$, $R^f$, $R^g$, and $R^h$ | are independently selected from H, $C_1$ to $C_6$ alkyl, $C_6$ to $C_{18}$ aryl, and $C_2$ to $C_{18}$ heteroaryl; |
| X | is selected from O, S, $NAr^2$; and |
| $Ar^2$ | is selected from $C_6$ to $C_{12}$ aryl. |

According to another embodiment, the compound of formula I is represented by formula (Ih),

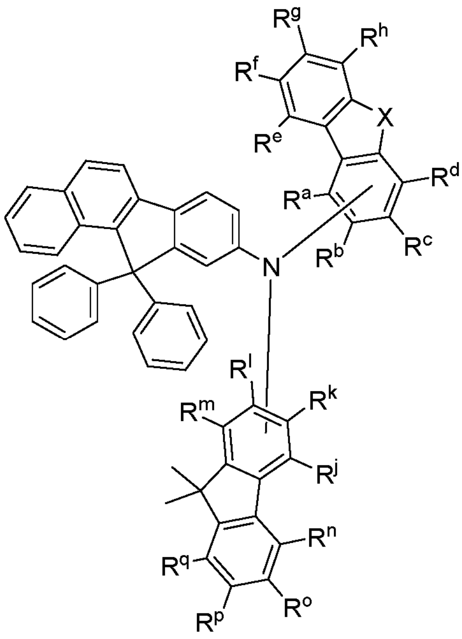

(Ih), wherein

| | |
|---|---|
| $R^j$, $R^k$, $R^l$, $R^m$, $R^n$, $R^o$, $R^p$ and $R^q$ | are independently selected from H, $C_1$ to $C_6$ alkyl, $C_6$ to $C_{12}$ aryl, $C_5$ to $C_{12}$ heteroaryl; |
| wherein $R^a$, $R^b$, $R^c$ and $R^d$ | are independently selected from H, $C_1$ to $C_6$ alkyl, $C_6$ to $C_{18}$ aryl, and $C_2$ to $C_{18}$ heteroaryl, |
| $R^a$, $R^b$, $R^c$ and $R^d$ | are independently represent a single bond if N of formula (Ih) is bonded to $R^a$, $R^b$, $R^c$ and $R^d$, respectively |

two of $R^a$, $R^b$, $R^c$ and $R^d$ can form a substituted or unsubstituted condensed ring system when two of $R^a$, $R^b$, $R^c$ and $R^d$ are adjacent to each other;
the at least one substituent on the condensed ring system is independently selected from $C_1$ to $C_6$ alkyl, $C_6$ to $C_{12}$ aryl or $C_2$ to $C_{12}$ heteroaryl;

| | |
|---|---|
| $R^e$, $R^f$, $R^g$, and $R^h$ | are independently selected from H, $C_1$ to $C_6$ alkyl, $C_6$ to $C_{18}$ aryl, and $C_2$ to $C_{18}$ heteroaryl; |
| X | is selected from O, S, $NAr^2$; and |
| $Ar^2$ | is selected from $C_6$ to $C_{12}$ aryl. |

**$R^j$, $R^k$, $R^l$, $R^m$, $R^n$, $R^o$, $R^p$ and $R^q$ of formula (Ih)**

According to an embodiment, $C_1$ to $C_6$ alkyl of $R^j$, $R^k$, $R^l$, $R^m$, $R^n$, $R^o$, $R^p$ and $R^q$ can be $C_1$ to $C_5$ alkyl, $C_1$ to $C_4$ alkyl, $C_1$ to $C_3$ alkyl, $C_1$ to $C_2$ alkyl, or $C_1$ alkyl.

According to an embodiment, $C_6$ to $C_{12}$ aryl of $R^j$, $R^k$, $R^l$, $R^m$, $R^n$, $R^o$, $R^p$ and $R^q$ can be $C_6$ to $C_{10}$aryl, $C_6$ to $C_8$ aryl, or $C_6$ aryl.

According to an embodiment, $C_5$ to $C_{12}$ heteroaryl of $R^j$, $R^k$, $R^l$, $R^m$, $R^n$, $R^o$, $R^p$ and $R^q$ can be $C_5$ to $C_{16}$ heteroaryl, $C_5$ to $C_{14}$ heteroaryl, $C_5$ to $C_{12}$ heteroaryl, $C_5$ to $C_{10}$ heteroaryl, $C_5$ to $C_8$, or $C_5$ heteroaryl.

According to an embodiment, $R^j$, $R^k$, $R^l$, $R^m$, $R^n$, $R^o$, $R^p$ and $R^q$ are independently selected from H, $C_1$ to $C_6$ alkyl, $C_6$ to $C_{12}$ aryl, $C_5$ to $C_{12}$ heteroaryl.

**X of formula (Ih)**

According to an embodiment, wherein X is selected from O, S, $NAr^2$.

**$Ar^3$ of formula (Ih)**

According to an embodiment, $Ar^3$ is selected from $C_6$ to $C_{12}$ aryl or $C_6$ to $C_{10}$ aryl. According to an embodiment, $Ar^3$ is selected from $C_6$ aryl.

**Formula (Ik)**

According to an embodiment, the compound of formula I is represented by formula Ik:

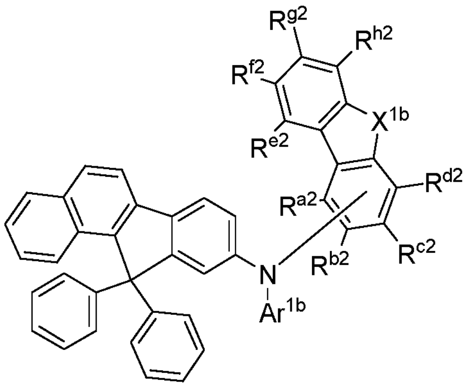

(Ik)

wherein

| | |
|---|---|
| $Ar^{1b}$ | is selected from $C_6$ to $C_{12}$ aryl or $C_5$ to $C_{25}$ heteroaryl; |
| $R^{a2}$, $R^{b2}$, $R^{c2}$ and $R^{d2}$ | are independently selected from H, $C_1$ to $C_6$ alkyl, $C_6$ to $C_{18}$ aryl, and $C_2$ to $C_{18}$ heteroaryl, |
| $R^{a2}$, $R^{b2}$, $R^{c2}$ and $R^{d2}$ | are independently represent a single bond if N of formula (Ik) is bonded to $R^{a2}$, $R^{b2}$, $R^{c2}$ and $R^{d2}$, respectively; |

two of $R^{a2}$, $R^{b2}$, $R^{c2}$ and $R^{d2}$ can form a substituted or unsubstituted condensed ring system when two of $R^{a2}$, $R^{b2}$, $R^{c2}$ and $R^{d2}$ are adjacent to each other;
the at least one substituent on the condensed ring system is independently selected from $C_1$ to $C_6$ alkyl, $C_6$ to $C_{12}$ aryl or $C_2$ to $C_{12}$ heteroaryl;

| | |
|---|---|
| $R^{e2}$, $R^{f2}$, $R^{g2}$, and $R^{h2}$ | are independently selected from H, $C_1$ to $C_6$ alkyl, $C_6$ to $C_{18}$ aryl, and $C_2$ to $C_{18}$ heteroaryl; |
| $X^{1b}$ | is selected from O, S, $NAr^{2b}$, $CR^{1b}R^{2b}$, $SiR^{1b}R^{2b}$; |
| $Ar^{2b}$ | is selected from $C_6$ to $C_{12}$ aryl; and |
| $R^{1b}$ and $R^{2b}$ | are independently selected from $C_1$ to $C_6$ alkyl, $C_6$ to $C_{12}$ aryl or $C_2$ to $C_{12}$ heteroaryl. |

According to an embodiment, the compound of formula I is represented by formula Ik:

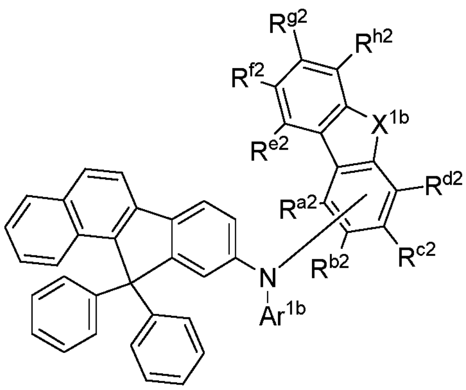

(Ik)

wherein

| | |
|---|---|
| $Ar^{1b}$ | is selected from $C_6$ to $C_{12}$ aryl or $C_5$ to $C_{25}$ heteroaryl; |
| $R^{a2}$, $R^{b2}$ , $R^{c2}$ and $R^{d2}$ | are independently selected from H, $C_1$ to $C_6$ alkyl, $C_6$ to $C_{18}$ aryl, and $C_2$ to $C_{18}$ heteroaryl, |
| $R^{a2}$, $Rb^2$ , $R^{c2}$ and $R^{d2}$ | are independently represent a single bond if N of formula (Ik) is bonded to $R^{a2}$, $R^{b2}$, $R^{c2}$ and $R^{d2}$, respectively; |

two of $R^{a2}$, $R^{b2}$, $R^{c2}$ and $R^{d2}$ can form a substituted or unsubstituted condensed ring system when two of $R^{a2}$, $R^{b2}$, $R^{c2}$ and $R^{d2}$ are adjacent to each other;
the at least one substituent on the condensed ring system is independently selected from $C_1$ to $C_6$ alkyl, $C_6$ to $C_{12}$ aryl or $C_2$ to $C_{12}$ heteroaryl;

| | |
|---|---|
| $R^{e2}$, $R^{f2}$, $R^{g2}$ , and $R^{h2}$ | are independently selected from H, $C_1$ to $C_6$ alkyl, $C_6$ to $C_{18}$ aryl, and $C_2$ to $C_{18}$ heteroaryl; |
| $X^{1b}$ | is selected from O, S, $NAr^{2b}$, and $CR^{1b}R^{2b}$; |
| $Ar^{2b}$ | is selected from $C_6$ to $C_{12}$ aryl; and |
| $R^{1b}$ and $R^{2b}$ | are independently selected from $C_1$ to $C_6$ alkyl, $C_6$ to $C_{12}$ aryl or $C_2$ to $C_{12}$ heteroaryl. |

According to an embodiment, the compound of formula I is represented by formula Ik:

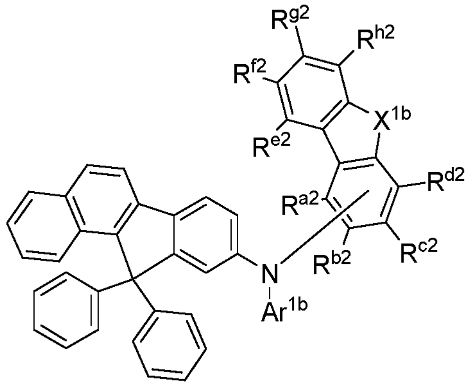

(Ik)

wherein

| | |
|---|---|
| $Ar^{1b}$ | is selected from biphenyl; |
| $X^{1b}$ | is selected from O, S, $NAr^{2b}$, $CR^{1b}R^{2b}$ and $SiR^{1b}R^{2b}$; |
| $Ar^{2b}$ | is selected from $C_6$ to $C_{12}$ aryl; and |
| $R^{1b}$ and $R^{2b}$ | are independently selected from $C_1$ to $C_6$ alkyl, $C_6$ to $C_{12}$ aryl or $C_2$ to $C_{12}$ heteroaryl. |

According to an embodiment, the compound of formula I is represented by formula Ik:

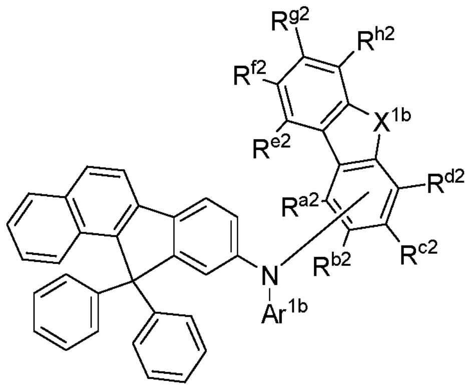

(Ik)

wherein

| | |
|---|---|
| $Ar^{1b}$ | is selected from biphenyl; |
| $X^{1b}$ | is selected from O, S, $NAr^{2b}$, and $CR^{1b}R^{2b}$; |
| $Ar^{2b}$ | is selected from $C_6$ to $C_{12}$ aryl; and |
| $R^{1b}$ and $R^{2b}$ | are independently selected from $C_1$ to $C_6$ alkyl, $C_6$ to $C_{12}$ aryl or $C_2$ to $C_{12}$ heteroaryl. |

According to an embodiment, the compound of formulae I, Ia, Ib, Ic, Id, Ie, If, Ig, Ih, Ik comprises 50 to 80 carbon atoms.

According to an embodiment, the compound of formulae I, Ia, Ib, Ic, Id, Ie, If, Ig, Ih, Ik comprises 50 to 75 carbon atoms.

According to an embodiment, the compound of formulae I, Ia, Ib, Ic, Id, Ie, If, Ig, Ih, Ik comprises 50 to 70 carbon atoms.

According to an embodiment, the compound of formulae I, Ia, Ib, Ic, Id, Ie, If, Ig, Ih, Ik comprises 55 to 65 carbon atoms.

According to an embodiment, the compound of formulae I, Ia, Ib, Ic, Id, Ie, If, Ig, Ih, Ik comprises 55 to 61 carbon atoms.

According to an embodiment, the compound of formulae I, Ia, Ib, Ic, Id, Ie, If, Ig, Ih, Ik comprises 10 to 13 rings.

According to an embodiment, the compound of formulae I, Ia, Ib, Ic, Id, Ie, If, Ig, Ih, Ik comprises 10 to 12 rings.

According to an embodiment, the compound of formulae I, Ia, Ib, Ic, Id, Ie, If, Ig, Ih, Ik comprises 11 to 12 rings.

According to an embodiment, the compound of formulae I, Ia, Ib, Ic, Id, Ie, If, Ig, Ih, Ik has a glass transition temperature Tg≥130°C.

According to an embodiment, the compound of formulae I, Ia, Ib, Ic, Id, Ie, If, Ig, Ih, Ik has a glass transition temperature Tg≥135°C.

According to an embodiment, the compound of formulae I, Ia, Ib, Ic, Id, Ie, If, Ig, Ih, Ik has a glass transition temperature Tg≥140°C.

**Compounds of formula I**

According to an embodiment of the present invention the benzo diphenyl fluorene compound of formula I, wherein the compound of formula I is selected from A1 to A35, A37; or the benzo diphenyl fluorene compound is represented by compound A36:

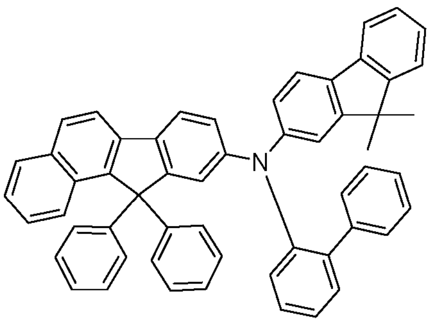
(A1),

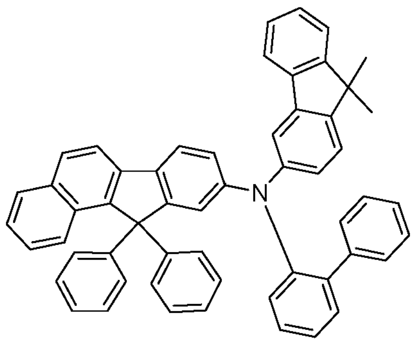
(A2),

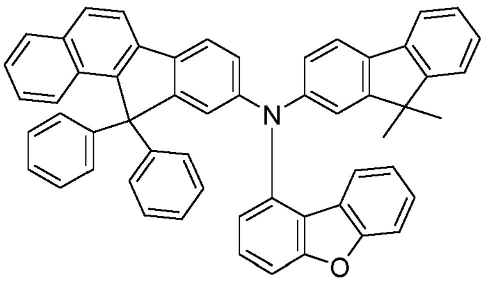
(A3),

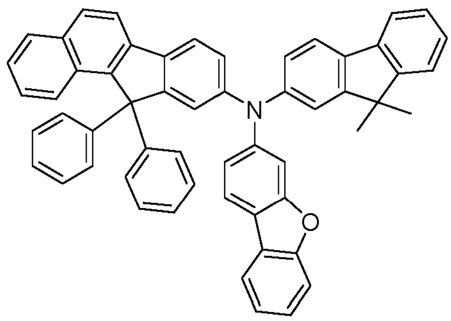
(A4),

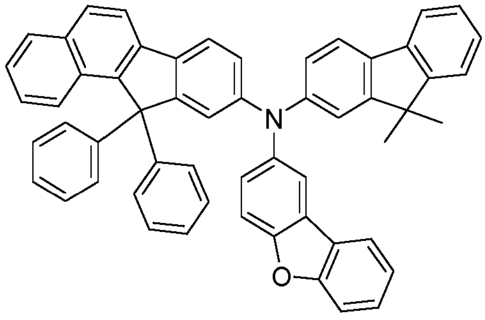 (A5), 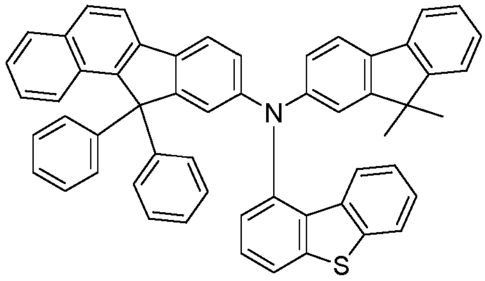 (A6),

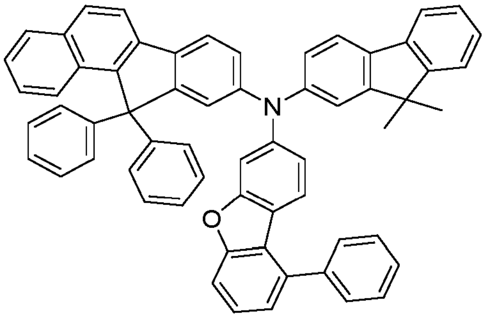 (A7), 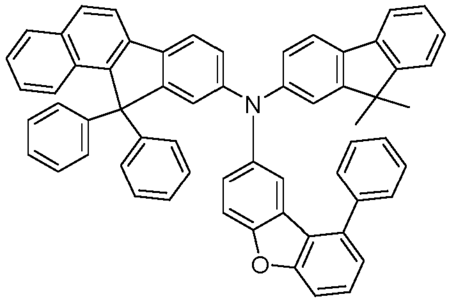 (A8),

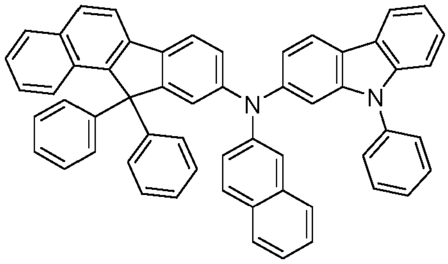 (A9), 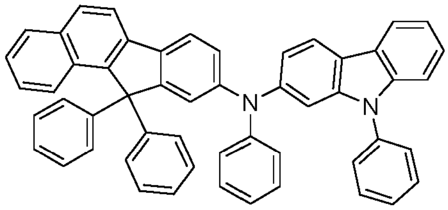 (A10),

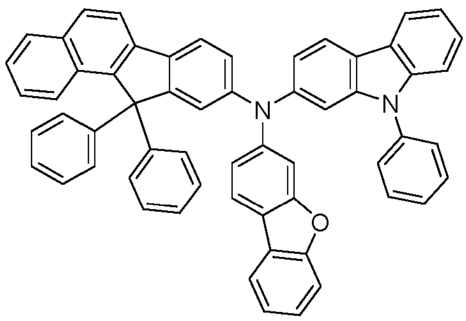 (A11), 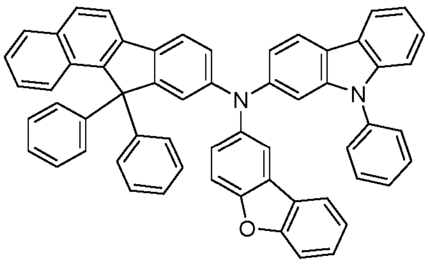 (A12),

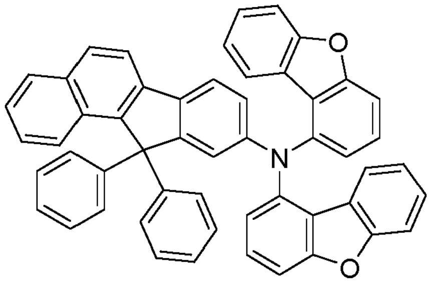 (A13), 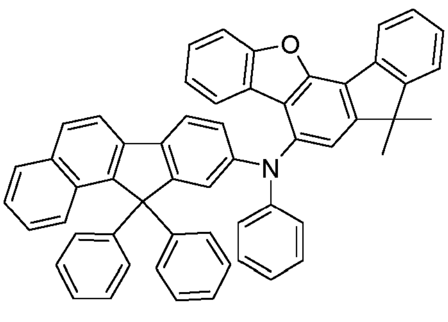 (A14),

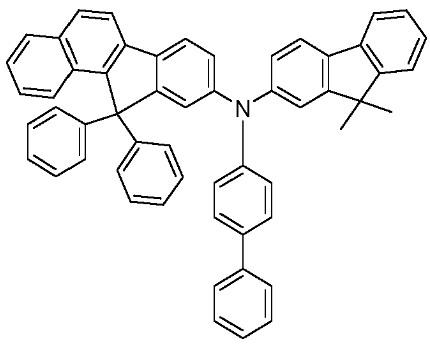
(A15),

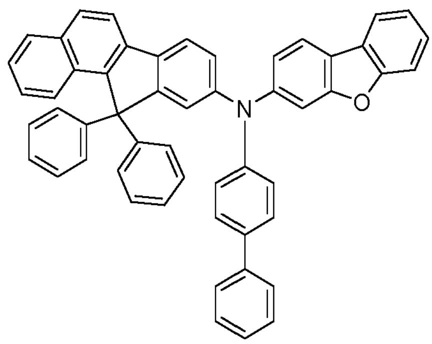
(A16),

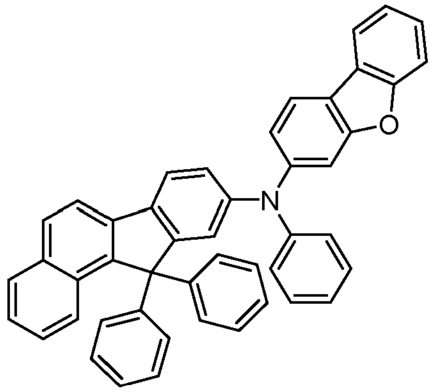
(A17),

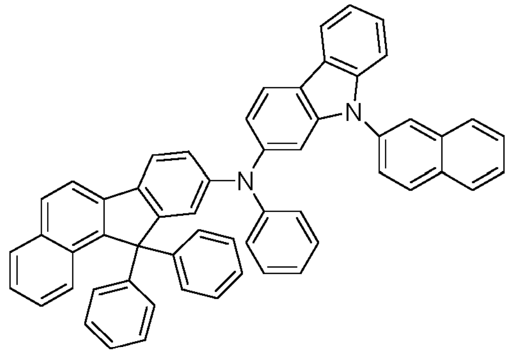
(A18),

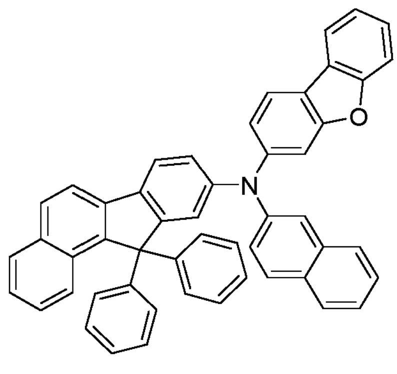
(A19),

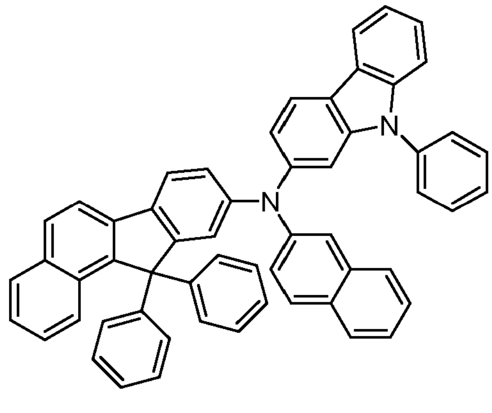
(A20),

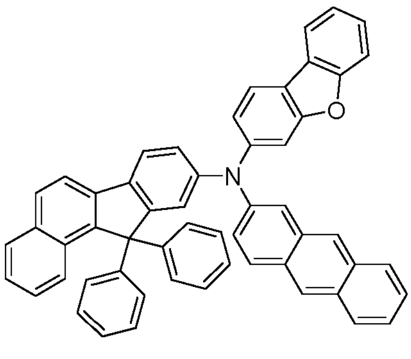
(A21),

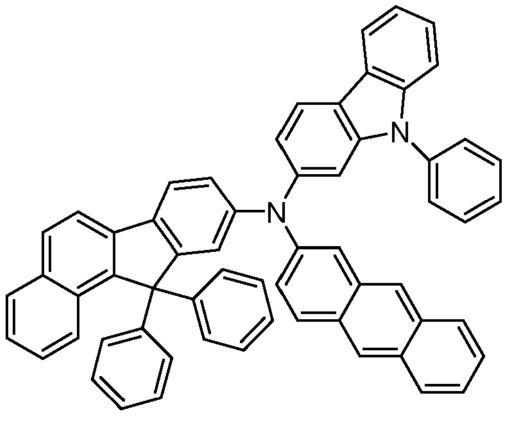
(A22),

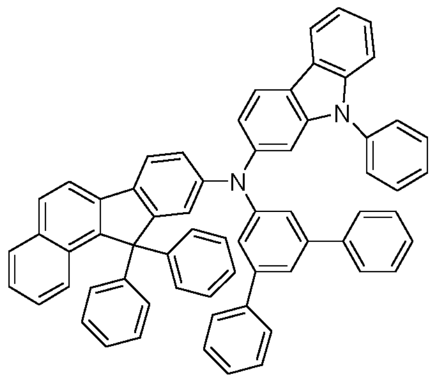
(A23),

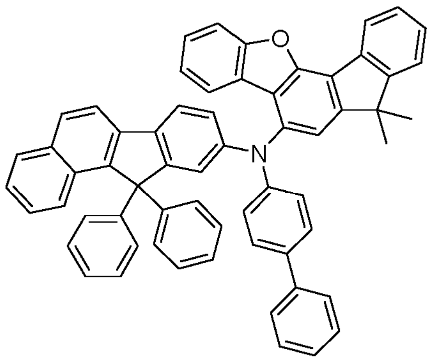
(A24),

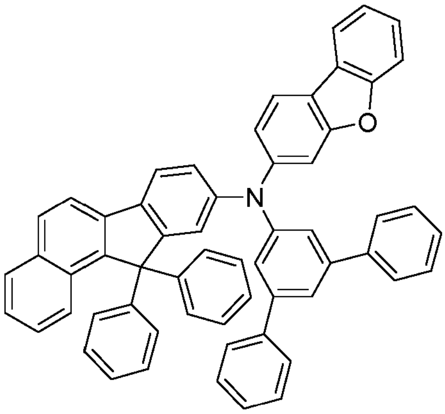
(A25),

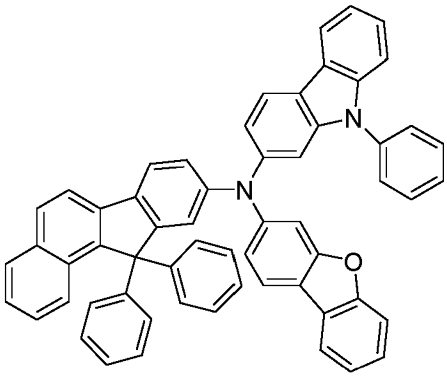
(A26),

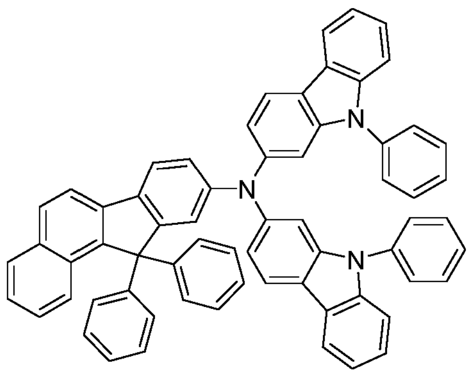
(A27),

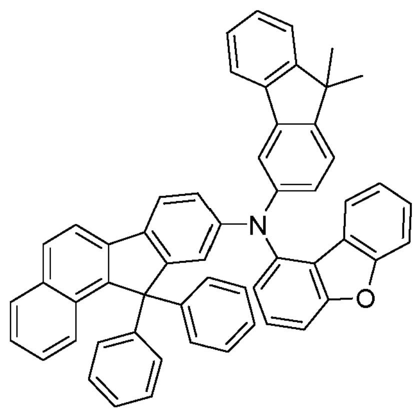
(A28),

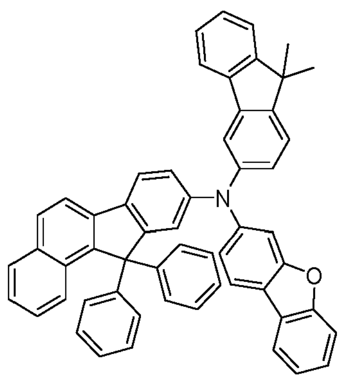
(A29),

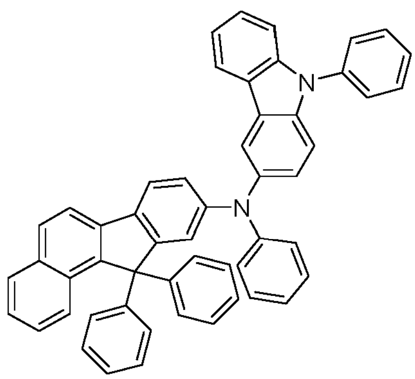
(A30),

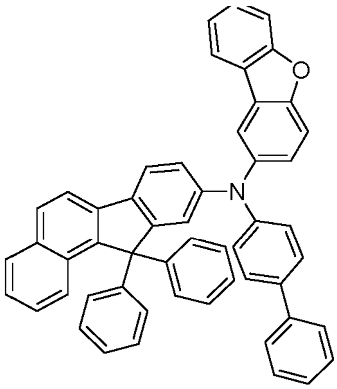
(A31),

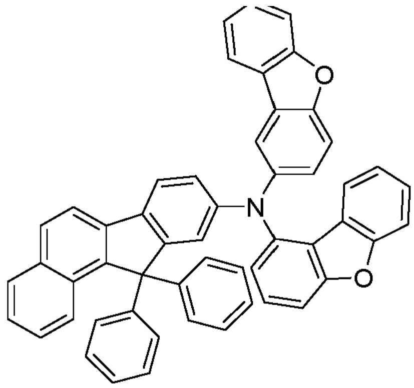
(A32),

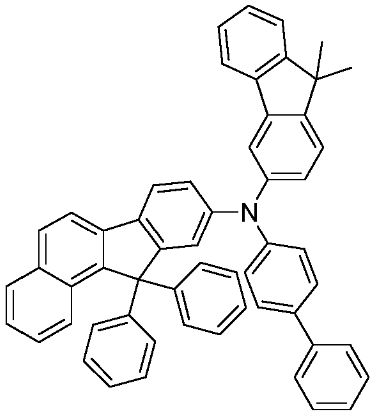
(A33),

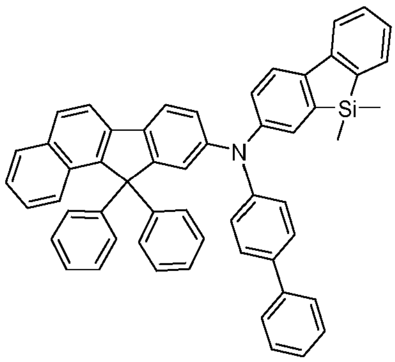
(A34),

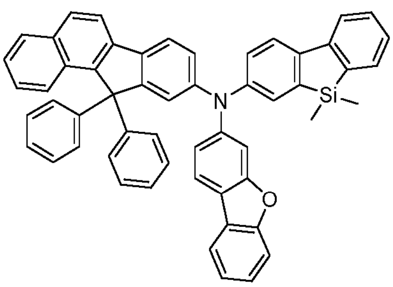
(A35),

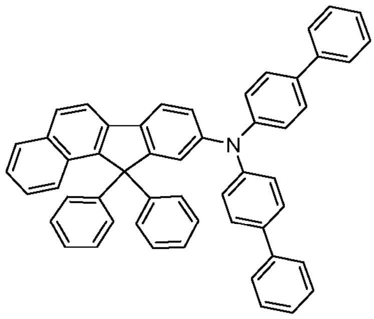
(A36),

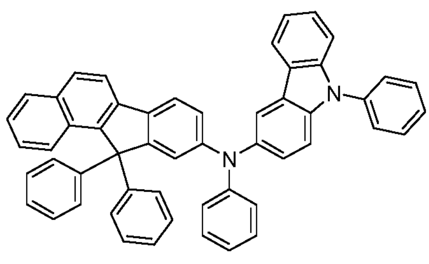
(A37).

According to an embodiment of the benzo diphenyl fluorene compound of formula I, wherein the compound of formula I is selected from A1 to A16 and A34 and A35 or A1 to A16 and A34 and A35 and A37. According to an embodiment of the benzo diphenyl fluorene compound of formula I, wherein the compound of formula I is selected from A1 to A14, A34, A35. According to an embodiment of the benzo diphenyl fluorene compound of formula I, wherein the compound of formula I is selected from A17 to A33. According to an embodiment of the benzo diphenyl fluorene compound of formula I, wherein the compound of formula I is selected from A17 to A35.

According to an embodiment of the benzo diphenyl fluorene compound of formula I, wherein the compound of formula I is selected from A1 to A4 and A10 and A16. According to an embodiment of the benzo diphenyl fluorene

compound of formula I, wherein the compound of formula I is selected from A1 to A14 and A35. According to an embodiment of the benzo diphenyl fluorene compound of formula I, wherein the compound of formula I is selected from A1 to A14. According to an embodiment of the benzo diphenyl fluorene compound of formula I, wherein the compound of formula I is selected from A1 to A8 and A11 to A14 and A35.

According to an embodiment of the benzo diphenyl fluorene compound of formula I, wherein the compound of formula I is selected from A1 to A8 and A11 to A14. According to an embodiment of the benzo diphenyl fluorene compound of formula I, wherein the compound of formula I is selected from A1 to A8 and A11 to A14. According to an embodiment of the benzo diphenyl fluorene compound of formula I, wherein the compound of formula I is selected from A3 to A8 and A14. According to an embodiment of the benzo diphenyl fluorene compound of formula I, wherein the compound of formula I is selected from A3 to A8. According to an embodiment of the benzo diphenyl fluorene compound of formula I, wherein the compound of formula I is selected from A1, A2, A9, A11, A14 and A34. According to an embodiment of the benzo diphenyl fluorene compound of formula I, wherein the compound of formula I is selected from A1, A2, A9, A11, A14. According to an embodiment of the benzo diphenyl fluorene compound of formula I, wherein the compound of formula I is selected from A1, A2, A14 and A34 and A35.

According to an embodiment of the benzo diphenyl fluorene compound of formula I, wherein the compound of formula I is selected from A1, A2, and A14.

**Semiconductor layer**

According to another embodiment, a semiconductor layer, preferably an organic semiconductor layer, comprises at least one benzo diphenyl fluorene compound selected from formulae I or selected from at least on benzo diphenyl fluorene compound selected from formulae Ia, Ib, Ic, Id, Ie, If, Ig, Ih, Ik according to the present invention.

According to another embodiment, wherein the organic semiconductor layer is a hole injection layer and/or hole transport layer. According to another embodiment, wherein the organic semiconductor layer is a hole injection layer. According to another embodiment, wherein the organic semiconductor layer is a hole transport layer.

According to another embodiment, wherein the organic semiconductor layer comprises in addition an organic p-dopant. According to another embodiment, wherein the organic semiconductor layer is a hole injection layer and/or hole transport layer and comprises in addition an organic p-dopant. According to another embodiment, wherein the organic semiconductor layer is a hole injection layer and comprises in addition an organic p-dopant. According to another embodiment, wherein the organic semiconductor layer is a hole transport layer and comprises in addition an organic p-dopant.

According to an embodiment, the organic semiconductor layer is arranged between the photoactive layer and anode.

According to an embodiment, the organic semiconductor layer is selected from a hole transport layer and a hole injection layer.

According to an embodiment, wherein the organic semiconductor layer is arranged between the photoactive layer and the anode, and wherein the organic semiconductor layer comprises an organic p-dopant.

According to an embodiment, the organic semiconductor layer is arranged between the photoactive layer and anode, and the organic semiconductor layer is selected from hole transport layer and a hole injection layer.

According to one embodiment, the thickness of the organic semiconductor layer may be in the range from about 1 nm to about 200 nm, and for example, from about 2 nm to about 175 nm, alternatively about 2 nm to about 150 nm.

According to one embodiment, the organic semiconductor layer may comprise:

- at least about $\geq$ 0.5 wt.-% to about $\leq$ 30 wt.-%, preferably about $\geq$ 0.5 wt.-% to about $\leq$ 20 wt.-%, and more preferred about $\geq$ 1 wt.-% to about $\leq$ 15 wt.-% of a compound of formula (II); and
- at least about $\geq$ 70 wt.-% to about $\leq$ 99.5 wt.-%, preferably about $\geq$ 80 wt.-% to about $\leq$ 99.5 wt.-%, and more preferred about $\geq$ 85 wt.-% to about $\leq$ 99 wt.-% of a compound of formula (I), (Ic), (Id), (Ie),(If),(Ig), (Ih) or (Ik); preferably the wt.-% of the compound of formula (II) is lower than the wt.-% of the compound of formula (I), (Ic), (Id), (Ie),(If),(Ig), (Ih) or (Ik); wherein the weight-% of the components are based on the total weight of the organic semiconductor layer.

According to one embodiment, the organic semiconductor layer is a hole injection layer having a thickness in the range from about 1 nm to about 100 nm, and for example, from about 1 nm to about 25 nm. When the thickness of the HIL is within this range, the HIL may have excellent hole injecting characteristics, without a substantial penalty in driving voltage.

According to one embodiment, the organic semiconductor layer is a hole transport layer having a thickness in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

**Organic electronic device**

According to an embodiment, the organic electronic device is an organic light emitting diode (OLED), a light emitting device, thin film transistor, a battery, a display device, an organic photovoltaic cell (OPV), a solar cell, preferably a perovskite solar cell, a photoconductor, a photodiode or a photodetector.
According to an embodiment, the organic electronic device is an electroluminescent device, a solar cell preferably a perovskite solar cell, an organic photovoltaic cell, a photoconductor, photodiode or a photodetector.
According to an embodiment, the organic electronic device is an organic light emitting diode (OLED), a light emitting device, thin film transistor, a battery, a display device, an organic photovoltaic cell (OPV).
According to an embodiment, the organic electronic device is an electroluminescent device, a solar cell preferably a perovskite solar cell, photoconductor, photodiode or a photodetector.
According to an embodiment, the organic electronic device is an electroluminescent device, a solar cell preferably a perovskite solar cell, a photodiode or a photodetector.
According to an embodiment, the electronic device is an electroluminescent device, a solar cell preferably a perovskite solar cell, or a photodetector.
According to an embodiment, the organic electronic device comprises an organic p-dopant.

**Radialene compound**

According to another embodiment not forming part of the present invention, wherein the organic p-dopant is a radialene compound.
According to an embodiment not forming part of the present invention, wherein the organic p-dopant is a radialene compound, wherein the radialene compound of formula (II)

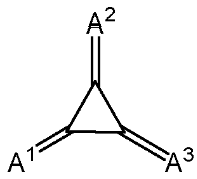 (II)

wherein in formula (II)

$A^1$ is independently selected from a group (1)

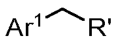 (1)

$Ar^1$ is independently selected from substituted or unsubstituted $C_6$ to $C_{36}$ aryl and substituted or unsubstituted $C_2$ to $C_{36}$ heteroaryl;
if $Ar^1$ is substituted, the one or more substituents are independently selected from an electron-withdrawing group, F, CN, partially perfluorinated or perfluorinated alkyl, $-NO_2$;
$A^2$ and $A^3$ are independently selected from a group (2)

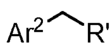

$Ar^2$ and $Ar^3$ are independently selected from substituted or unsubstituted $C_6$ to $C_{36}$ aryl and substituted or unsubstituted $C_2$ to $C_{36}$ heteroaryl; and
if $Ar^2$ and $Ar^3$ are substituted, the one or more substituents are independently selected from an electron-withdrawing group, F, CN, partially perfluorinated or perfluorinated alkyl, $-NO_2$;
each R' is independently selected from an electron-withdrawing group.

According to an embodiment, $C_6$ to $C_{36}$ aryl of $Ar^1$ can be selected from $C_6$ to $C_{36}$ aryl, $C_6$ to $C_{30}$ aryl, $C_6$ to $C_{26}$ aryl, $C_6$ to $C_{34}$ aryl, $C_6$ to $C_{22}$ aryl, $C_6$ to $C_{20}$ aryl, $C_6$ to $C_{18}$ aryl, $C_6$ to $C_{16}$ aryl, $C_6$ to $C_{14}$ aryl, $C_6$ to $C_{12}$ aryl, or $C_6$ to $C_{10}$ aryl.
According to an embodiment, $C_6$ to $C_{36}$ aryl of $Ar^1$ can be selected from $C_6$ to $C_{36}$ heteroaryl, $C_6$ to $C_{30}$ heteroaryl, $C_6$ to $C_{26}$ heteroaryl, $C_6$ to $C_{34}$ heteroaryl, $C_6$ to $C_{22}$ heteroaryl, $C_6$ to $C_{20}$ heteroaryl, $C_6$ to $C_{18}$ heteroaryl, $C_6$ to $C_{16}$ heteroaryl, $C_6$ to $C_{14}$ heteroaryl, $C_6$ to $C_{12}$ heteroaryl, or $C_6$ to $C_{10}$ heteroaryl.
According to an embodiment, $Ar^1$ is independently selected from substituted or unsubstituted $C_6$ to $C_{24}$ aryl and

substituted or unsubstituted $C_2$ to $C_{24}$ heteroaryl.

According to an embodiment, $Ar^1$ is independently selected from substituted or unsubstituted $C_6$ to $C_{18}$ aryl and substituted or unsubstituted $C_2$ to $C_{18}$ heteroaryl.

According to an embodiment, $Ar^1$ is independently selected from substituted or unsubstituted $C_6$ to $C_{12}$ aryl and substituted or unsubstituted $C_2$ to $C_{12}$ heteroaryl.

According to an embodiment, the one or more substituents of $Ar^1$ can be selected from F, CN, partially perfluorinated or perfluorinated alkyl or $-NO_2$.

According to an embodiment, the one or more substituents of $Ar^1$ can be selected from F, CN, or partially perfluorinated or perfluorinated alkyl.

According to an embodiment, the one or more substituents of $Ar^1$ can be selected from F, CN, or $CF_3$.

According to an embodiment, $C_6$ to $C_{36}$ aryl of $Ar^2$ can be selected independently from $C_6$ to $C_{36}$ aryl, $C_6$ to $C_{30}$ aryl, $C_6$ to $C_{26}$ aryl, $C_6$ to $C_{34}$ aryl, $C_6$ to $C_{22}$ aryl, $C_6$ to $C_{20}$ aryl, $C_6$ to $C_{18}$ aryl, $C_6$ to $C_{16}$ aryl, $C_6$ to $C_{14}$ aryl, $C_6$ to $C_{12}$ aryl, or $C_6$ to $C_{10}$ aryl.

According to an embodiment, $C_6$ to $C_{36}$ aryl of $Ar^2$ can be selected independently from $C_6$ to $C_{36}$ heteroaryl, $C_6$ to $C_{30}$ heteroaryl, $C_6$ to $C_{26}$ heteroaryl, $C_6$ to $C_{34}$ heteroaryl, $C_6$ to $C_{22}$ heteroaryl, $C_6$ to $C_{20}$ heteroaryl, $C_6$ to $C_{18}$ heteroaryl, $C_6$ to $C_{16}$ heteroaryl, $C_6$ to $C_{14}$ heteroaryl, $C_6$ to $C_{12}$ heteroaryl, or $C_6$ to $C_{10}$ heteroaryl.

According to an embodiment, $Ar^2$ is independently selected from substituted or unsubstituted $C_6$ to $C_{24}$ aryl and substituted or unsubstituted $C_2$ to $C_{24}$ heteroaryl.

According to an embodiment, $Ar^2$ is independently selected from substituted or unsubstituted $C_6$ to $C_{18}$ aryl and substituted or unsubstituted $C_2$ to $C_{18}$ heteroaryl.

According to an embodiment, $Ar^2$ is independently selected from substituted or unsubstituted $C_6$ to $C_{12}$ aryl and substituted or unsubstituted $C_2$ to $C_{12}$ heteroaryl.

According to an embodiment, the one or more substituents of $Ar^2$ can be selected independently from F, CN, partially perfluorinated or perfluorinated alkyl or $-NO_2$.

According to an embodiment, the one or more substituents of $Ar^2$ can be selected independently from F, CN, or partially perfluorinated or perfluorinated alkyl.

According to an embodiment, the one or more substituents of $Ar^2$ can be selected independently from F, CN, or $CF_3$.

According to an embodiment, the electron-withdrawing group of R' can be selected from CN, partially fluorinated or perfluorinated $C_1$ to $C_6$ alkyl preferably from $CF_3$, $NO_2$, or F.

According to an embodiment, the electron-withdrawing group of R' can be selected from $CF_3$, F, or CN.

According to an embodiment, the electron-withdrawing group of R' can be selected from $CF_3$ or CN.

According to an embodiment, the electron-withdrawing group of R' can be selected from CN.

According to an embodiment, the compound of formula is selected from a compound of formula (III)

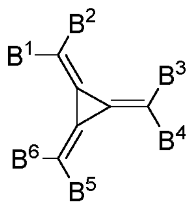

(III)

whereby $B^1$ is selected from $Ar^1$

whereby $B^3$ and $B^5$ are independently selected from $Ar^2$.

According to one embodiment not forming part of the present invention, $B^3$ and $B^5$ are Ar and Ar is selected from substituted or unsubstituted $C_6$ to $C_{12}$ aryl and substituted or unsubstituted $C_3$ to $C_{12}$ heteroaryl, wherein the substituents on Ar are independently selected from CN, partially or perfluorinated $C_1$ to $C_4$ alkyl, halogen, F; preferably Ar is selected from substituted phenyl, pyridyl, pyrimidyl or triazinyl, wherein the substituents on Ar are independently selected from CN, $CF_3$ or F.

According to one embodiment of the present invention, $B^2$, $B^4$ and $B^6$ are R" and R" is selected from CN, partially or fully fluorinated $C_1$ to $C_4$ alkyl, $-NO_2$, partially or fully fluorinated $C_1$ to $C_4$ alkoxy, substituted or unsubstituted $C_6$ to $C_{12}$ aryl or $C_3$ to $C_{12}$ heteroaryl, wherein the substituents are selected from halogen, F, Cl, CN, partially or fully fluorinated $C_1$ to $C_4$ alkyl, partially or fully fluorinated $C_1$ to $C_4$ alkoxy; more preferred $R^3$ is selected from CN, $CF_3$, $OCF_3$ or F, most preferred CN.

According to one embodiment, the organic semiconductor layer comprises a composition comprising a compound of formula (V) and at least one compound of formula (Va) to (Vd)

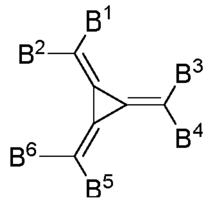

(Va)

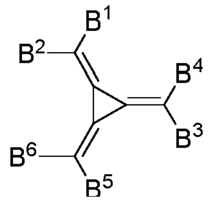

(Vb)

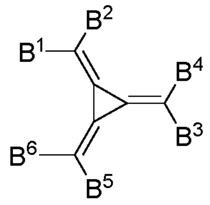

(Vc)

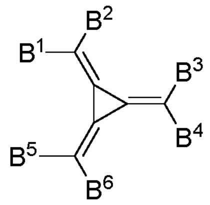

(Vd)

According to one embodiment, the compound of formula (III) comprises less than nine cyano moieties.

According to one embodiment, the compound of formula (III) comprises between 3 and 8 cyano moieties.

According to one embodiment, the compound of formula (III) comprises between 6 and 8 cyano moieties.

According to one embodiment of the present invention, $Ar^1$ is selected from formula (IVa)

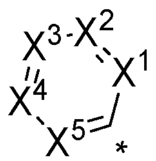

(IVa)

wherein in formula (IVa)

$X^1$ is selected from $CR^1$ or N;

$X^2$ is selected from $CR^2$ or N;
$X^3$ is selected from $CR^3$ or N;
$X^4$ is selected from $CR^4$ or N;
$X^5$ is selected from $CR^5$ or N;
$R^1$, $R^2$, $R^3$, $R^4$ and $R^5$ (if present) are independently selected from electron-withdrawing group, CN, partially fluorinated $C_1$ to $C_8$ alkyl, perfluorinated $C_1$ to $C_8$ alkyl, $-NO_2$, halogen, Cl, F, D or H, whereby when any of $R^1$, $R^2$, $R^3$, $R^4$ and $R^5$ is present, then the corresponding $X^1$, $X^2$, $X^3$, $X^4$ and $X^5$ is not N;

wherein the asterisk "*" denotes the binding position.

According to one embodiment of the present invention, $Ar^2$ and $Ar^3$ are independently selected from formula (IVb)

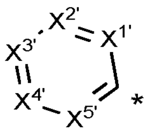

(IVb)

wherein in formula (IVb)
$X^{1'}$ is selected from $CR^{1'}$ or N;
$X^{2'}$ is selected from $CR^{2'}$ or N;
$X^{3'}$ is selected from $CR^{3'}$ or N;
$X^{4'}$ is selected from $CR^{4'}$ or N;
$X^{5'}$ is selected from $CR^{5'}$ or N;
$R^{1'}$, $R^{2'}$, $R^{3'}$, $R^{4'}$ and $R^{5'}$ (if present) are independently selected from electron-withdrawing group, CN, partially fluorinated $C_1$ to $C_8$ alkyl, perfluorinated $C_1$ to $C_8$ alkyl, $-NO_2$, halogen, Cl, F, D or H, whereby when any of $R^{1'}$, $R^{2'}$, $R^{3'}$, $R^{4'}$ and $R^{5'}$ is present, then the corresponding $X^{1'}$, $X^{2'}$, $X^{3'}$, $X^{4'}$ and $X^{5'}$ is not N;

wherein the asterisk "*" denotes the binding position.

According to one embodiment, $Ar^1$ is selected from

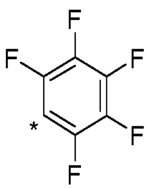 (G1), 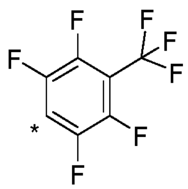 (G2, 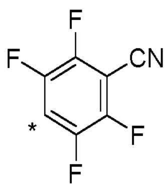 (G3), 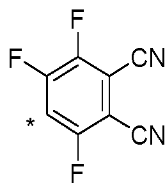 (G4), 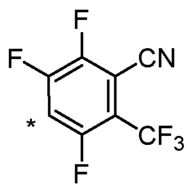 (G5),

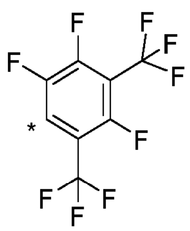 (G6), 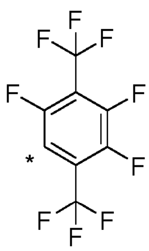 (G7), 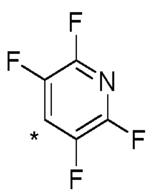 (G8), 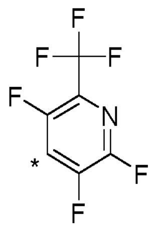 (G9), 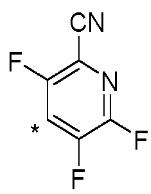 (G10),

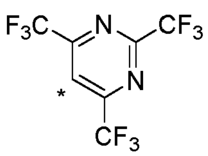 (G11), 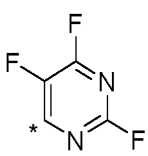 (G12), 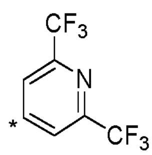 (G13), 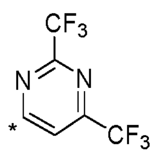 (G14),

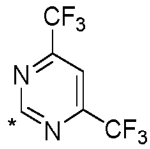 (G15), 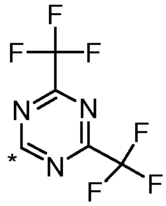 (G16), 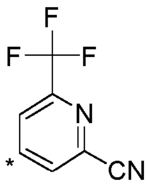 (G17), 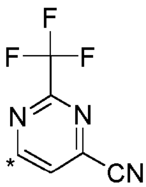 (G18), 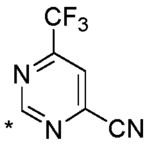 (G19),

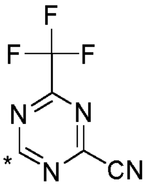 (G20), 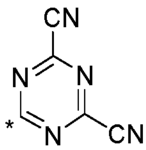 (G21), 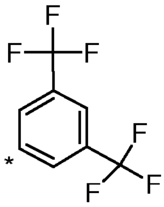 (G22), 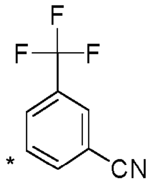 (G23) , 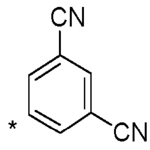 (G24),

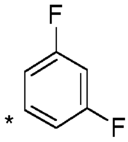 (G25), 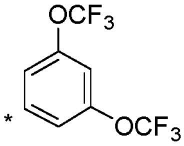 (G26), 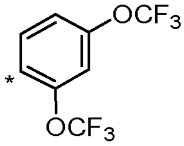 (G27), 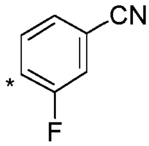 (G28),

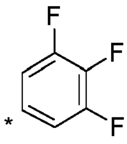 (G29) , 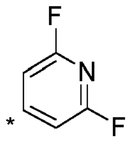 (G30) , 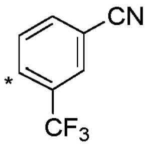 (G31), 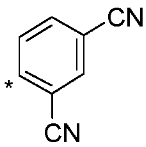 (G32), 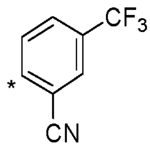 (G33),

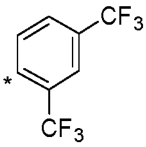 (G34), 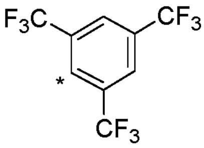 (G35), 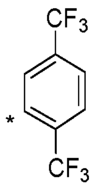 (G36), 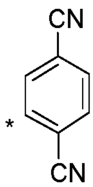 (G37), 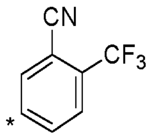 (G38),

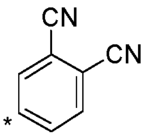 (G39), 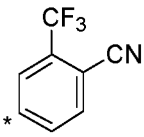 (G40), 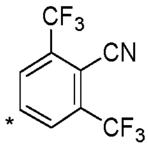 (G41), 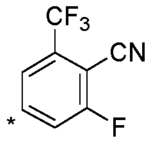 (G42), 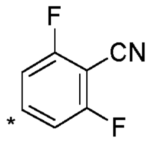 (G43),

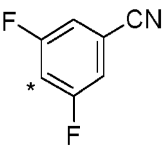 (G44), 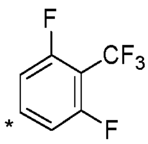 (G45), 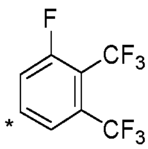 (G46), 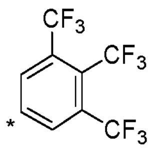 (G47), 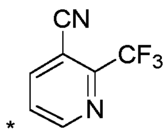 (G48),

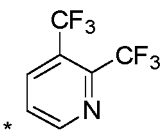 (G49), 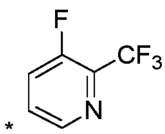 (G50), 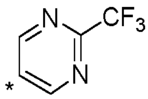 (G51), 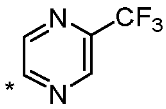 (G52), 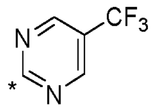 8G53),

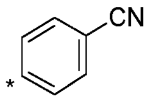 (G54), and 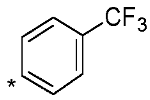 (G55);

wherein the asterisk "*" denotes the binding position.

According to one embodiment, $Ar^1$, $Ar^2$, and $Ar^3$ are independently selected from G1 to G55.

According to an embodiment, the organic semiconductor layer, the stack of organic layers, and/or the organic electronic device does not contain Dipyrazino[2,3-f:2',3'-h]quinoxaline-2,3,6,7,10,11-hexacarbonitrile (HATCN) or there like. According to an embodiment, the organic semiconductor layer, the stack of organic layers, and/or the organic electronic device does not contain Dipyrazino[2,3-f:2',3'-h]quinoxaline-2,3,6,7,10,11-hexacarbonitrile (HATCN).

According to an embodiment, the organic semiconductor layer, the stack of organic layers, and/or the organic electronic device does not contain Dipyrazino[2,3-f:2',3'-h]quinoxaline-2,3,6,7,10,11-hexacarbonitrile (HATCN) or there like 2,2'-(2,5-cyclohexadiene-1,4-diylidene)bismalononitrile (TCNQ) or 2,2'-(perfluorocyclohexa-2,5-diene-1,4-diylidene)dimalononitrile (F4-TCNQ). According to an embodiment, the organic semiconductor layer, the stack of organic layers, and/or the organic electronic device does not contain Dipyrazino[2,3-f:2',3'-h]quinoxaline-2,3,6,7,10,11-hexacarbonitrile (HATCN), 2,2'-(2,5-cyclohexadiene-1,4-diylidene)bismalononitrile (TCNQ) or 2,2'-(perfluorocyclohexa-2,5-diene-1,4-diylidene)dimalononitrile (F4-TCNQ).

According to an embodiment, the organic semiconductor layer, the stack of organic layers, and/or the organic electronic device does not contain Dipyrazino[2,3-f:2',3'-h]quinoxaline-2,3,6,7,10,11-hexacarbonitrile (HATCN) or there like 2,2'-(2,5-cyclohexadiene-1,4-diylidene)bismalononitrile (TCNQ), 2,2'-(perfluorocyclohexa-2,5-diene-1,4-diylidene)dimalononitrile (F4-TCNQ), or 2,2'-(1,3,4,5,7,8-Hexafluoro-2,6-naphthalenediylidene)bis[propanedinitrile] (F6-TCNNQ). According to an embodiment, the organic semiconductor layer, the stack of organic layers, and/or the organic electronic device does not contain Dipyrazino[2,3-f:2',3'-h]quinoxaline-2,3,6,7,10,11-hexacarbonitrile (HATCN), 2,2'-(2,5-cyclohexadiene-1,4-diylidene)bismalononitrile (TCNQ), 2,2'-(perfluorocyclohexa-2,5-diene-1,4-diylidene)dimalononitrile (F4-TCNQ) or 2,2'-(1,3,4,5,7,8-Hexafluoro-2,6-naphthalenediylidene)bis[propanedinitrile] (F6-TCNNQ).

Any specifications of formula (I) as described above in the context of the organic electronic device apply *mutatis mutandis.*

## Organic semiconductor layer

According to an embodiment, wherein the organic semiconductor layer is arranged between the photoactive layer and the anode, and wherein the organic electronic device comprises a further organic semiconductor layer preferably comprising an organic p-dopant and more preferably an organic p-dopant and a matrix compound.

According to an embodiment, wherein the organic semiconductor layer is arranged between the photoactive layer and the anode, and wherein the organic electronic device further comprises a hole injection layer preferably comprising an organic p-dopant, and more preferably an organic p-dopant and a matrix compound.

According to an embodiment, the organic semiconductor layer is a hole transport layer and the organic electronic device comprises a further organic semiconductor layer preferably comprising an organic p-dopant and more preferably an organic p-dopant and a matrix compound.

According to an embodiment, the organic semiconductor layer is a hole transport layer and the organic electronic device further comprises a hole injection layer preferably comprising an organic p-dopant and more preferably an organic p-dopant and a matrix compound.

According to an embodiment, wherein the organic semiconductor layer is arranged between the photoactive layer and the anode, wherein the organic semiconductor layer is a hole transport layer, and wherein the organic electronic device comprises a further organic semiconductor layer preferably comprising an organic p-dopant and more preferably an organic p-dopant and a matrix compound.

According to an embodiment, wherein the organic semiconductor layer is arranged between the photoactive layer and the anode, wherein the organic semiconductor layer is a hole transport layer, and wherein the organic electronic device further comprises a hole injection layer preferably comprising an organic p-dopant and more preferably an organic p-dopant and a matrix compound.

According to an embodiment, wherein the organic semiconductor layer is arranged between the photoactive layer and the anode, wherein the organic electronic device further comprises a hole injection layer preferably comprising an organic p-dopant, and wherein the hole injection layer is adjacent to the anode.

According to an embodiment, wherein the organic semiconductor layer is a hole transport layer, wherein the organic electronic device further comprises a hole injection layer preferably comprising an organic p-dopant and more preferably an organic p-dopant and a matrix compound, and wherein the hole injection layer is adjacent to the anode.

According to an embodiment, wherein the organic semiconductor layer is arranged between the photoactive

layer and the anode, wherein the organic semiconductor layer is a hole transport layer, and wherein the organic electronic device comprises a further organic semiconductor layer preferably comprising an organic p-dopant and more preferably an organic p-dopant and a matrix compound, and wherein the hole injection layer is adjacent to the anode.

According to an embodiment, wherein the organic semiconductor layer is arranged between the photoactive layer and the anode, wherein the organic semiconductor layer is a hole transport layer, and wherein the organic electronic device further comprises a hole injection layer preferably comprising an organic p-dopant and more preferably an organic p-dopant and a matrix compound, and wherein the hole injection layer is adjacent to the anode.

According to an embodiment, wherein the organic semiconductor layer is arranged between the photoactive layer and the anode, wherein the organic electronic device further comprises a hole injection layer preferably comprising an organic p-dopant and more preferably an organic p-dopant and a matrix compound, and wherein the hole injection layer is in direct contact to the anode.

According to an embodiment, wherein the organic semiconductor layer is a hole transport layer, wherein the organic electronic device further comprises a hole injection layer preferably comprising an organic p-dopant and more preferably an organic p-dopant and a matrix compound, and wherein the hole injection layer is in direct contact to the anode.

According to an embodiment, wherein the organic semiconductor layer is arranged between the photoactive layer and the anode, wherein the organic semiconductor layer is a hole transport layer, and wherein the organic electronic device further comprises a hole injection layer preferably comprising an organic p-dopant and more preferably an organic p-dopant and a matrix compound, and wherein the hole injection layer is in direct contact to the anode.

Preferably the matrix compound is a hole transport material.

According to an embodiment, the organic electronic device comprises a hole injection layer, wherein the hole injection layer comprises a compound of formulae (I) or (Ia), (Ib), (Ic), (Id), (Ie),(If),(Ig), (Ih) and (Ik).

According to an embodiment, the organic electronic device further comprises a hole injection layer, wherein the hole injection layer comprises a compound of formulae (I) or (Ia), (Ib), (Ic), (Id), (Ie),(If),(Ig), (Ih) and (Ik) and an organic p-dopant.

According to an embodiment, wherein the organic semiconductor layer is arranged between the photoactive layer and the anode, wherein the organic semiconductor layer is a hole injection layer, and wherein the organic semiconductor layer comprises an organic p-dopant.

A hole injection layer (HIL) may be formed on the anode layer by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of $10^{-8}$ to $10^{-3}$ Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

A HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecyl benzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

A HIL may comprise or consist of p-type dopant and the p-type dopant may be selected from tetrafluoro-tetracyanoquinonedimethane (F4TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto. The HIL may be selected from a hole-transporting matrix compound doped with a p-type dopant. Typical examples of known doped hole transport materials are: copper phthalocyanine (CuPc), which HOMO level is approximately -5.2 eV, doped with tetrafluoro-tetracyanoquinonedimethane (F4TCNQ), which LUMO level is about -5.2 eV; zinc phthalocyanine (ZnPc) (HOMO = -5.2 eV) doped with F4TCNQ; α-NPD (N,N'-Bis(naphthalen-1-yl)-N,N'-bis(phenyl)-benzidine) doped with F4TCNQ. α-NPD doped with 2,2'-(perfluoronaphthalen-2,6-diylidene) dimalononitrile. The p-type dopant concentrations can be selected from 1 to 20 wt.-%, more preferably from 3 wt.-% to 10 wt.-%.

The thickness of a HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

Substantially covalent matrix compound

The organic semiconductor layer or the organic electronic device may further comprises a covalent matrix

compound also named "substantially covalent matrix compound". Preferably, the covalent matrix compound is a hole transport compound preferably used in a hole injection layer and/or hole transport layer.

According to one embodiment the substantially covalent matrix compound may be selected from at least one organic compound. The substantially covalent matrix may consists substantially from covalently bound C, H, O, N, S, which optionally comprise in addition covalently bound B, P, As and/or Se.

According to one embodiment of the organic electronic device, the organic semiconductor layer further comprises a substantially covalent matrix compound, wherein the substantially covalent matrix compound may be selected from organic compounds consisting substantially from covalently bound C, H, O, N, S, which optionally comprise in addition covalently bound B, P, As and/or Se.

Organometallic compounds comprising covalent bonds carbon-metal, metal complexes comprising organic ligands and metal salts of organic acids are further examples of organic compounds that may serve as substantially covalent matrix compounds of the hole injection layer.

In one embodiment, the substantially covalent matrix compound lacks metal atoms and majority of its skeletal atoms may be selected from C, O, S, N. Alternatively, the substantially covalent matrix compound lacks metal atoms and majority of its skeletal atoms may be selected from C and N.

According to one embodiment, the substantially covalent matrix compound may have a molecular weight Mw of $\geq$ 400 and $\leq$ 2000 g/mol, preferably a molecular weight Mw of $\geq$ 450 and $\leq$ 1500 g/mol, further preferred a molecular weight Mw of $\geq$ 500 and $\leq$ 1000 g/mol, in addition preferred a molecular weight Mw of $\geq$ 550 and $\leq$ 900 g/mol, also preferred a molecular weight Mw of $\geq$ 600 and $\leq$ 800 g/mol.

Preferably, the substantially covalent matrix compound comprises at least one arylamine moiety, alternatively a diarylamine moiety, alternatively a triarylamine moiety.

Preferably, the substantially covalent matrix compound is free of metals and/or ionic bonds.

Compound of formula (VI) or a compound of formula (VII)

According to another aspect of the present disclosure, at least one matrix compound, also referred to as "substantially covalent matrix compound", may comprises at least one arylamine compound, diarylamine compound, triarylamine compound, a compound of formula (VI) or a compound of formula (VII):

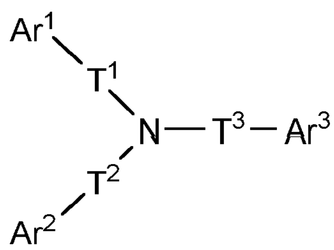
(VI),

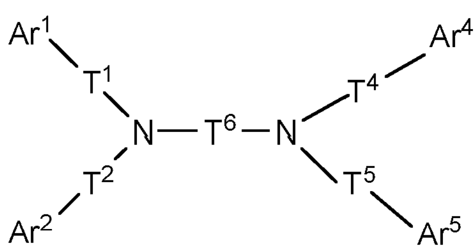
(VII),

wherein:

$T^1$, $T^2$, $T^3$, $T^4$ and $T^5$ are independently selected from a single bond, phenylene, biphenylene, terphenylene or naphthenylene, preferably a single bond or phenylene;

$T^6$ is phenylene, biphenylene, terphenylene or naphthenylene;

$Ar^1$, $Ar^2$, $Ar^3$, $Ar^4$ and $Ar^5$ are independently selected from substituted or unsubstituted $C_6$ to $C_{20}$ aryl, or substituted or unsubstituted $C_3$ to $C_{20}$ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle,

(iv) 6-member ring of an aromatic non-heterocycle;
wherein the substituents of $Ar^1$, $Ar^2$, $Ar^3$, $Ar^4$ and $Ar^5$ are selected the same or different from the group comprising H, D, F, $C(-O)R^2$, CN, $Si(R^2)_3$, $P(-O)(R^2)_2$, $OR^2$, $S(-O)R^2$, $S(-O)_2R^2$, substituted or unsubstituted straight-chain alkyl having 1 to 20 carbon atoms, substituted or unsubstituted branched alkyl having 1 to 20 carbon atoms, substituted or unsubstituted cyclic alkyl having 3 to 20 carbon atoms, substituted or unsubstituted alkenyl or alkynyl groups having 2 to 20 carbon atoms, substituted or unsubstituted alkoxy groups having 1 to 20 carbon atoms, substituted or unsubstituted aromatic ring systems having 6 to 40 aromatic ring atoms, and substituted or unsubstituted heteroaromatic ring systems having 5 to 40 aromatic ring atoms, unsubstituted $C_6$ to $C_{18}$ aryl, unsubstituted $C_3$ to $C_{18}$ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle,
wherein $R^2$ may be selected from H, D, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, $C_6$ to $C_{18}$ aryl or $C_3$ to $C_{18}$ heteroaryl.

According to an embodiment wherein $T^1$, $T^2$, $T^3$, $T^4$ and $T^5$ may be independently selected from a single bond, phenylene, biphenylene or terphenylene. According to an embodiment wherein $T^1$, $T^2$, $T^3$, $T^4$ and $T^5$ may be independently selected from phenylene, biphenylene or terphenylene and one of $T^1$, $T^2$, $T^3$, $T^4$ and $T^5$ are a single bond. According to an embodiment wherein $T^1$, $T^2$, $T^3$, $T^4$ and $T^5$ may be independently selected from phenylene or biphenylene and one of $T^1$, $T^2$, $T^3$, $T^4$ and $T^5$ are a single bond. According to an embodiment wherein $T^1$, $T^2$, $T^3$, $T^4$ and $T^5$ may be independently selected from phenylene or biphenylene and two of $T^1$, $T^2$, $T^3$, $T^4$ and $T^5$ are a single bond.

According to an embodiment wherein $T^1$, $T^2$ and $T^3$ may be independently selected from phenylene and one of $T^1$, $T^2$ and $T^3$ are a single bond. According to an embodiment wherein $T^1$, $T^2$ and $T^3$ may be independently selected from phenylene and two of $T^1$, $T^2$ and $T^3$ are a single bond.

According to an embodiment wherein $T^6$ may be phenylene, biphenylene, terphenylene. According to an embodiment wherein $T^6$ may be phenylene. According to an embodiment wherein $T^6$ may be biphenylene. According to an embodiment wherein $T^6$ may be terphenylene.

According to an embodiment wherein $Ar^1$, $Ar^2$, $Ar^3$, $Ar^4$ and $Ar^5$ may be independently selected from D1 to D16:

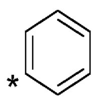 (D1), 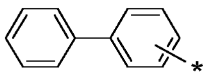 (D2), 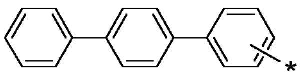 (D3), 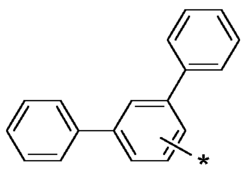 (D4),

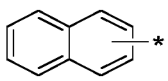 (D5), 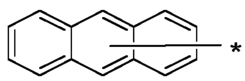 (D6), 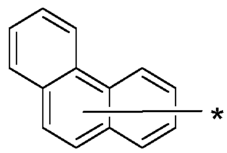 (D7), 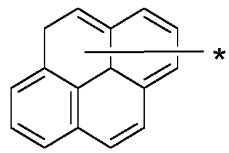 (D8),

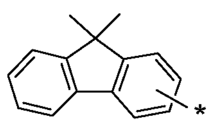 (D9), 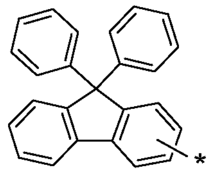 (D10), 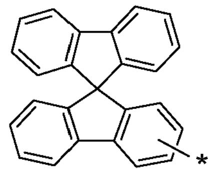 (D11),

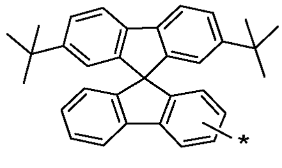 (D12), 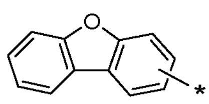 (D13), 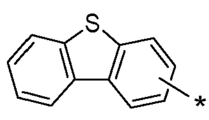 (D14),

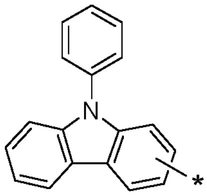(D15), 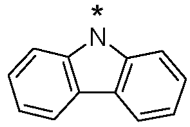(D16),

wherein the asterix "*" denotes the binding position.

According to an embodiment, wherein $Ar^1$, $Ar^2$, $Ar^3$, $Ar^4$ and $Ar^5$ may be independently selected from D1 to D15; alternatively selected from D1 to D10 and D13 to D15.

According to an embodiment, wherein $Ar^1$, $Ar^2$, $Ar^3$, $Ar^4$ and $Ar^5$ may be independently selected from the group consisting of D1, D2, D5, D7, D9, D10, D13 to D16.

The rate onset temperature may be in a range particularly suited to mass production, when $Ar^1$, $Ar^2$, $Ar^3$, $Ar^4$ and $Ar^5$ are selected in this range.

The "matrix compound of formula (VI) or formula (VII)" may be also referred to as "hole transport compound".

According to one embodiment, the substantially covalent matrix compound comprises at least one naphthyl group, carbazole group, dibenzofuran group, dibenzothiophene group and/or substituted fluorenyl group, wherein the substituents are independently selected from methyl, phenyl or fluorenyl.

According to an embodiment of the electronic device, wherein the matrix compound of formula (VI) or formula (VII) are selected from F1 to F18:

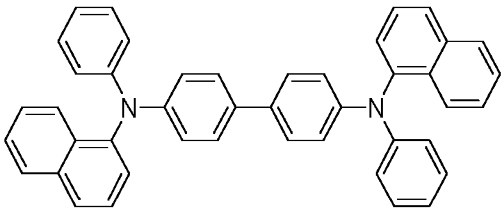(F1), 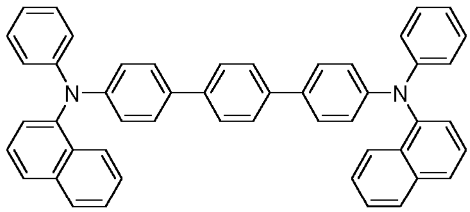(F2),

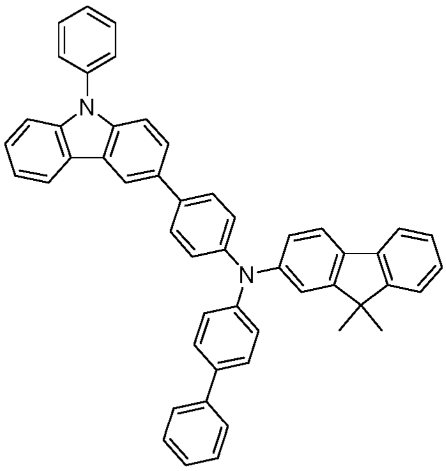(F3), 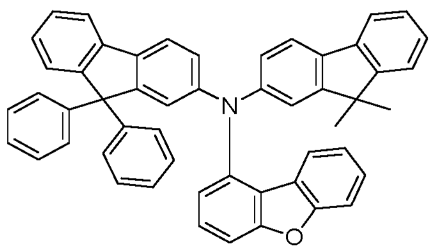(F4),

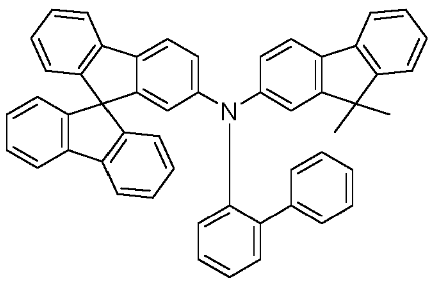(F5), 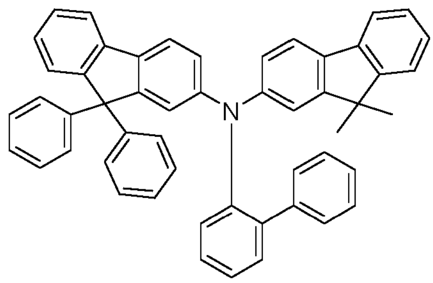(F6),

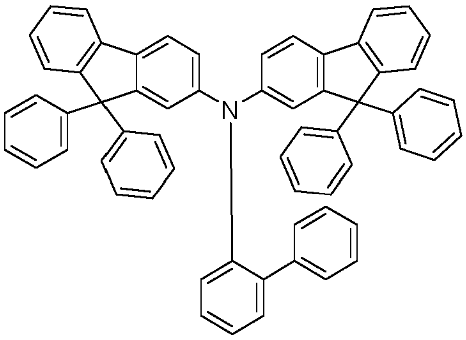
(F7),

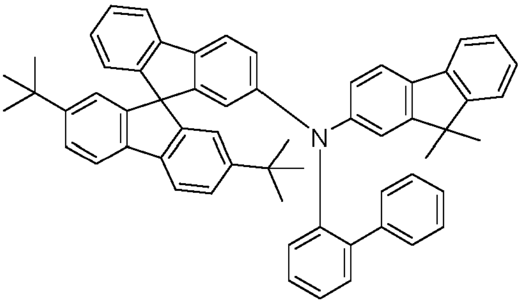
(F8),

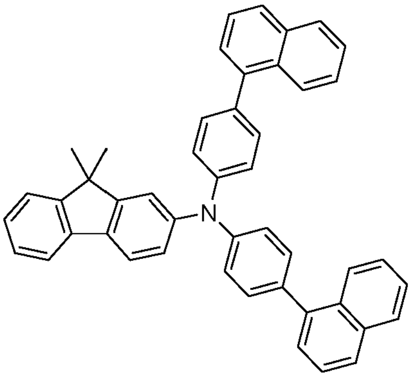
(F9),

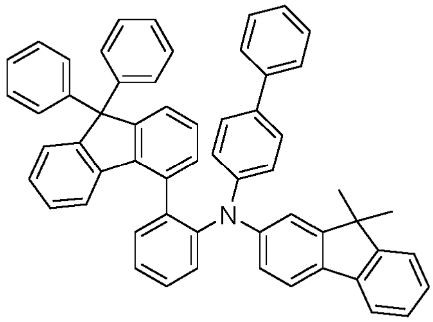
(F10),

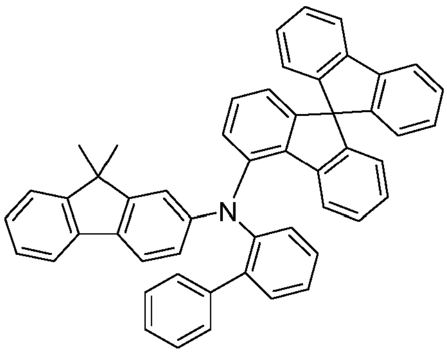
(F11),

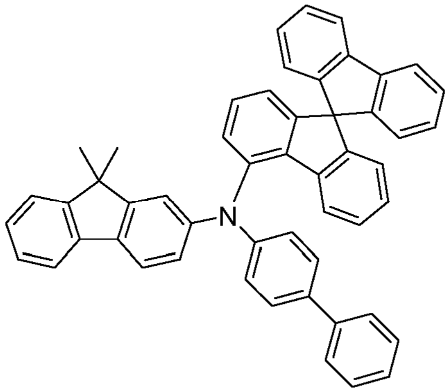
(F12),

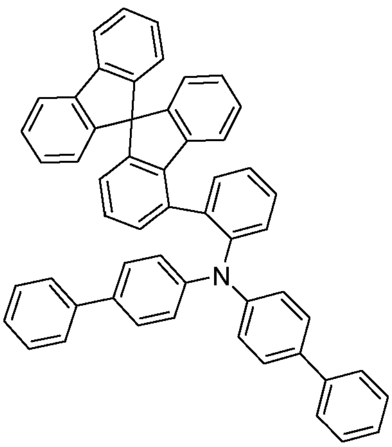
(F13),

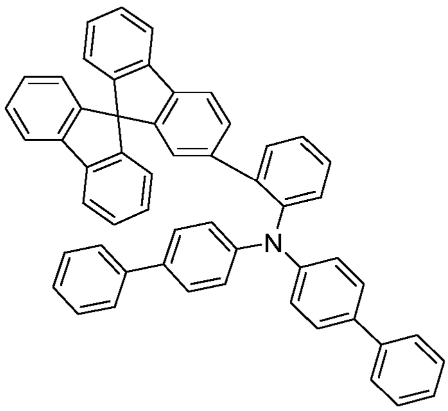
(F14),

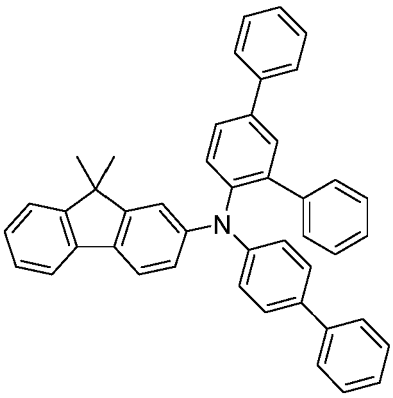
(F15),

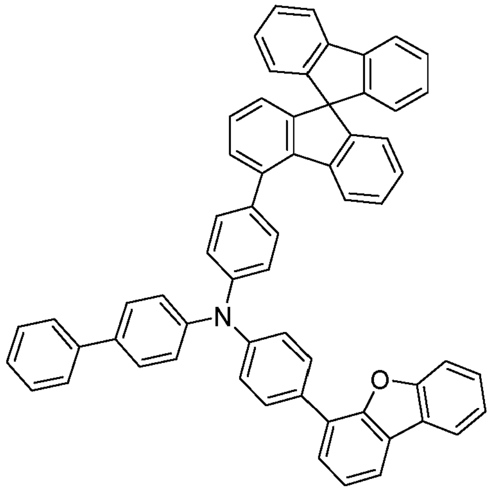
(F16),

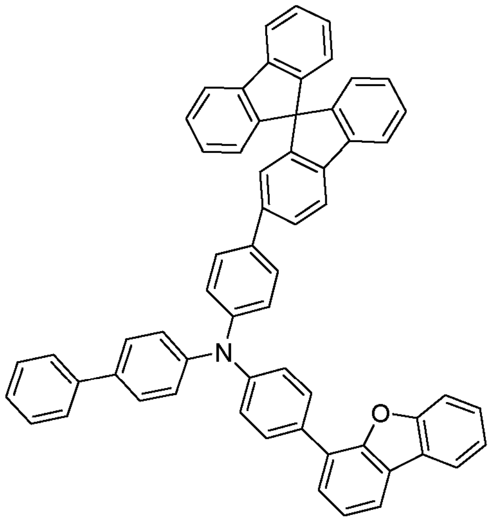
(F17)

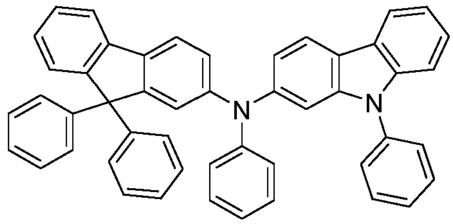
(F18).

According to one embodiment the electronic organic device is an electroluminescent device, preferably an organic light emitting diode.

According to a preferred embodiment, the electronic organic device is an electroluminescent device, preferably an organic light emitting diode, wherein light is emitted through the cathode layer.

The present invention furthermore relates to a display device comprising an organic electronic device according to the present invention.

According to a preferred embodiment, the display device comprising an organic electronic device according to the present invention, wherein the cathode layer is transparent.

**Further layers**

In accordance with the invention, the organic electronic device may comprise, besides the layers already mentioned above, further layers. Exemplary embodiments of respective layers are described in the following:

**Substrate**

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate, a silicon substrate or a backplane.

## Electrode

In an embodiment the first electrode is transparent. In another embodiment the second electrode is transparent. In a further embodiment the transparent electrode material is a thin conductive oxide (TCO). In another embodiment the transparent electrode material is selected from the group of indium-tin-oxide (ITO), fluorine doped thin oxide (FTO), aluminum-zinc-oxide (AZO), indium-gallium-zinc-oxide (IGZO), indium-zink-oxide (IZO), molybdenum-zink-oxide (MZO) and indium-molybdenum-oxide (IMO).

In another embodiment the transparent electrode material is selected from the group of magnesium (Mg), aluminum (Al), aluminum-lithium (Al-Li), calcium (Ca), magnesium-indium (Mg-In), magnesium-silver (Mg-Ag), silver (Ag), gold (Au), or the like.
According to an embodiment, wherein the second electrode comprises at least a metal, wherein the metal is selected from Al, Ag, Au, Ti, Pt, Cr, Zn, Sn, Sr, In, Sc, Hf or a mixture thereof.

According to an embodiment, the first electrode is the anode and the second electrode is the cathode. According to an embodiment, the first electrode is the cathode and the second electrode is the anode.

## Anode layer

The anode layer may be formed by depositing or sputtering a material that is used to form the anode layer. The material used to form the anode layer may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode electrode. The anode layer may also be formed using metals, typically silver (Ag), gold (Au), or metal alloys.

## p-type charge generation layer

A p-type charge generation layer may be formed on the anode layer or cathode layer by vacuum deposition, spin coating, printing, casting, slot-die coating, Langmuir-Blodgett (LB) deposition, or the like. When the a p-type charge generation layer is formed using vacuum deposition, the deposition conditions may vary according to the compound(s) that are used to form the layer, and the desired structure and thermal properties of the layer. In general, however, conditions for vacuum deposition may include a deposition temperature of 100° C to 350° C, a pressure of $10^{-8}$ to $10^{-3}$ Torr (1 Torr equals 133.322 Pa), and a deposition rate of 0.1 to 10 nm/sec.

When the a p-type charge generation layer is formed using spin coating or printing, coating conditions may vary according to the compound(s) that are used to form the layer, and the desired structure and thermal properties of the organic semiconductor layer. For example, the coating conditions may include a coating speed of about 2000 rpm to about 5000 rpm, and a thermal treatment temperature of about 80° C to about 200° C. Thermal treatment removes a solvent after the coating is performed.

## n-type charge generation layer

The organic electronic device may comprise a n-type charge generation layer.

According to an embodiment, the n-type charge generation layer may comprise an n-CGL matrix compound, preferably comprising at least one $C_2$ to $C_{24}$ N-heteroaryl or P=X group, with X being O, P, Se, with P=O especially preferred.

According to an embodiment, at least one $C_2$ to $C_{24}$ N-heteroaryl may be selected from a compound comprising at least one azine group, preferably at least two azine groups, also preferred three azine groups.

According to an embodiment, the n-type charge generation layer may comprise an n-CGL matrix compound comprising at least one group selected from the list consisting of pyridine, pyrimidine, triazine, imidazole, benzimidazole, benzoxazole, quinone, benzoquinone, quinoxaline, benzo quinoxaline, acridine, phenanthroline, benzoacridine, dibenzoacridine.

According to an embodiment, the n-type charge generation layer may comprise an n-CGL matrix compound comprising at least one phenanthroline group, preferably two phenanthroline groups.

According to an embodiment, the n-type charge generation layer may comprise metal a dopant, wherein the metal dopant may be a metal selected from Li, Na, Cs, Mg, Ca, Sr, S or Yb, preferably from Li or Yb.

## Hole injection layer

According to an embodiment, wherein the organic electronic device further comprises a hole injection layer in

direct contact to the anode, wherein the organic semiconductor layer is arranged between the hole injection layer and the light-emitting layer.

According to an embodiment, wherein the organic electronic device further comprises a hole injection layer in direct contact to the anode, wherein the organic semiconductor layer is arranged between the hole injection layer and the light-emitting layer, and wherein the hole transport layer is in direct contact to the hole injection layer.

A hole injection layer (HIL) may be formed on the anode layer by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of $10^{-8}$ to $10^{-3}$ Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecyl benzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of p-type dopant and the p-type dopant may be selected from tetrafluoro-tetracyanoquinonedimethane (F4TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto.

Preferably, the p-type dopant is selected from a radialene compound, for example 2,2',2"-(cyclopropane-1,2,3-triylidene)tris(2-(p-cyanotetrafluorophenyl)acetonitrile) (CC3).

The HIL may comprise a substantially covalent matrix compound and a p-type dopant.

The p-type dopant concentrations can be selected from 1 to 20 wt.-%, more preferably from 3 wt.-% to 10 wt.-%.

The p-type dopant concentrations can be selected from 1 to 20 vol.-%, more preferably from 3 vol.-% to 10 vol.-%.

According to a preferred embodiment, however, the HIL comprises a compound of formula (I) or (IV) as described above.

According to a preferred embodiment, the HIL may comprises the same compound of formula (I) and/or (IV) as in the p-type charge generation layer.

According to a preferred embodiment, the HIL may comprises a substantially covalent matrix compound as described above.

According to a preferred embodiment, the HIL may comprises a compound of formula (I) or (IV), as described above, and a compound of formula (VI) or (VII), as described above.

According to a preferred embodiment, the p-type charge generation layer and the hole injection layer may comprise an identical substantially covalent matrix compound.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

**Hole transport layer**

According to an embodiment, wherein the semiconductor layer, preferably organic semiconductor layer, is a hole transport layer, wherein the organic semiconductor layer comprises an organic p-dopant.

According to an embodiment, wherein the organic semiconductor layer is arranged between the photoactive layer and the anode, wherein the organic semiconductor layer is a hole transport layer and wherein the organic semiconductor layer comprises an organic p-dopant.

According to an embodiment, wherein the organic semiconductor layer is arranged between the photoactive layer and the anode, wherein the organic semiconductor layer is a hole transport layer, and wherein the organic electronic device comprises an organic p-dopant.

A hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

According to one embodiment, the hole transport layer may comprise a substantially covalent matrix compound as described above.

According to a preferred embodiment, the hole injection layer and the hole transport layer may comprise an identical substantially covalent matrix compound as described above.

According to one embodiment, the hole transport layer may comprise a compound of formula (VI) or (VII) as described above.

According to a preferred embodiment, the hole injection layer and the hole transport layer may comprise an identical compound of formula (VI) or (VII) as described above.

According to a preferred embodiment, the p-type charge generation layer, the hole injection layer and the hole transport layer may comprise an identical substantially covalent matrix compound.

According to a preferred embodiment, the p-type charge generation layer, the hole injection layer and the hole transport layer may comprise an identical compound of formula (VI) or (VII) as described above.

According to a preferred embodiment, the hole injection layer, the hole transport layer and the p-type charge generation layer may comprise an identical compound of formula (I) or (Ia), (Ib), (Ic), (Id), (Ie),(If),(Ig), (Ih) and (Ik), as described above.

According to one embodiment of the present invention, the hole transport layer may comprise the same substantially covalent matrix compound as the Organic semiconductor layer.

The thickness of a HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics, without a substantial penalty in driving voltage.

**Electron blocking layer**

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

**Photoactive layer (PAL)**

The photoactive layer converts an electrical current into photons or photons into an electrical current.

The PAL may be formed on the HTL by vacuum deposition, spin coating, slot-die coating, printing, casting, LB deposition, or the like. When the PAL is formed using vacuum deposition or spin coating, the conditions for deposition and coating may be similar to those for the formation of the HIL. However, the conditions for deposition and coating may vary, according to the compound that is used to form the PAL.

According to an embodiment, the organic electronic device may further comprise a photoactive layer, wherein the photoactive layer is arranged between the anode layer and the cathode layer.

The photoactive layer converts an electrical current into photons or photons into an electrical current.

The PAL may be formed on the HTL by vacuum deposition, spin coating, slot-die coating, printing, casting, LB deposition, or the like. When the PAL is formed using vacuum deposition or spin coating, the conditions for deposition and coating may be similar to those for the formation of the HIL. However, the conditions for deposition and coating may vary, according to the compound that is used to form the PAL.

According to one embodiment, the photoactive layer does not comprise the compound of formula (I).

The photoactive layer may be a light-emitting layer or a light-absorbing layer.

**Emission layer (EML)**

According to an embodiment, the organic electronic device may further comprise an emission layer, wherein the emission layer is arranged between the anode layer and the cathode layer.

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

According to one embodiment not forming part of the present invention, the emission layer does not comprise the compound of formula (I).

The emission layer (EML) may be formed of a combination of a host and an emitter dopant. Example of the host are Alq3, 4,4'-N,N'-dicarbazole-biphenyl (CBP), poly(n-vinyl carbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters, however, fluorescent red emitter dopants could also be used.

Examples of phosphorescent green emitter dopants are Ir(ppy)3 (ppy = phenylpyridine), Ir(ppy)2(acac), Ir(mpyp) 3.

Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

**Hole blocking layer (HBL)**

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, phenanthroline derivatives and azine derivatives, preferably triazine or pyrimidine derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

**Electron transport layer (ETL)**

The organic electronic device according to the present disclosure may further comprise an electron transport layer (ETL).

According to another embodiment not forming part of the present invention, the electron transport layer may further comprise an azine compound, preferably a triazine compound or a pyrimidine compound.

In one embodiment, the electron transport layer may further comprise a dopant selected from an alkali organic complex, preferably LiQ.

The thickness of the ETL may be in the range from about 15 nm to about 50 nm, for example, in the range from about 20 nm to about 40 nm. When the thickness of the EIL is within this range, the ETL may have satisfactory electron-injecting properties, without a substantial penalty in driving voltage.

According to another embodiment of the present invention, the organic electronic device may further comprise a hole blocking layer and an electron transport layer, wherein the hole blocking layer and the electron transport layer comprise an azine compound. Preferably, the azine compound is a triazine compound.

## Electron injection layer (EIL)

An optional EIL, which may facilitates injection of electrons from the cathode, may be formed on the ETL, preferably directly on the electron transport layer. Examples of materials for forming the EIL include lithium 8-hydroxyquinolinolate (LiQ), LiF, NaCl, CsF, Li2O, BaO, Ca, Ba, Yb, Mg which are known in the art. Deposition and coating conditions for forming the EIL are similar to those for formation of the HIL, although the deposition and coating conditions may vary, according to the material that is used to form the EIL.
The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

## Cathode layer

The cathode layer is formed on the ETL or optional EIL. The cathode layer may be formed of a metal, an alloy, an electrically conductive compound, or a mixture thereof. The cathode electrode may have a low work function. For example, the cathode layer may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode electrode may be formed of a transparent conductive oxide, such as ITO or IZO.
The thickness of the cathode layer may be in the range from about 5 nm to about 1000 nm, for example, in the range from about 10 nm to about 100 nm. When the thickness of the cathode layer is in the range from about 5 nm to about 50 nm, the cathode layer may be transparent or semitransparent even if formed from a metal or metal alloy.
In a preferred embodiment, the cathode layer comprises a metal or metal alloy and is transparent.
It is to be understood that the cathode layer is not part of an electron injection layer or the electron transport layer.

## Organic light-emitting diode (OLED)

The organic electronic device according to the invention may be an organic light-emitting device.
According to one aspect of the present invention, there is provided an organic light-emitting diode (OLED) comprising: a substrate; an anode electrode formed on the substrate; an organic semiconductor layer comprising a compound of formula (I) , a hole transport layer, an emission layer, an electron transport layer and a cathode electrode.
According to another aspect of the present invention, there is provided an OLED comprising: a substrate; an anode electrode formed on the substrate; an organic semiconductor layer comprising a compound of formula (I), a hole transport layer, an electron blocking layer, an emission layer, a hole blocking layer, an electron transport layer and a cathode electrode.
According to another aspect of the present invention, there is provided an OLED comprising: a substrate; an anode electrode formed on the substrate; an organic semiconductor layer comprising a compound of formula (I), a hole transport layer, an electron blocking layer, an emission layer, a hole blocking layer, an electron transport layer, an electron injection layer, and a cathode electrode.
According to various embodiments of the present invention, there may be provided OLEDs layers arranged between the above mentioned layers, on the substrate or on the top electrode.
According to one aspect of the present invention, there is provided an organic light-emitting diode (OLED) comprising: a substrate; an anode layer formed on the substrate; a hole injection layer which may comprise a compound of formula (I), a hole transport layer, an emission layer, an electron transport layer, a n-type charge generation layer, a p-type charge generation layer comprising a compound of formula (I), a hole transport layer, an optional electron injection layer and a cathode layer.
According to various embodiments of the present invention, there may be provided OLEDs layers arranged between the above mentioned layers, on the substrate or on the top electrode.
According to one aspect, the OLED may comprise a layer structure of a substrate that is adjacent arranged to an anode electrode, the anode electrode is adjacent arranged to a first hole injection layer, the first hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to a first electron transport layer, the first electron transport layer is adjacent arranged to an n-type charge generation layer, the n-type charge generation layer is adjacent arranged to a p-type charge generating layer, the p-type charge generating layer is adjacent arranged to a second hole transport layer, the second hole transport layer is adjacent

arranged to a second electron blocking layer, the second electron blocking layer is adjacent arranged to a second emission layer, between the second emission layer and the cathode electrode an optional electron transport layer and/or an optional electron injection layer are arranged.

The Organic semiconductor layer according to the invention may be the first hole injection layer and/or the hole transport layer.

**Organic electronic device**

According to one embodiment the organic electronic device may comprises at least one semiconductor layer comprising a compound of formula I or formula Ia, Ib, Ic, Id, Ie, If, Ig, Ih, Ik according to the invention.

The organic electronic device may comprises an anode layer, a cathode layer and at least one organic semiconductor layer, wherein at least one organic semiconductor layer is arranged between the anode layer and the cathode layer, wherein at least one semiconductor layer comprises a compound of formula I or formula Ia, Ib, Ic, Id, Ie, If, Ig, Ih, Ik according to the invention..

The organic electronic device according to the invention may be the organic electronic device is an organic light emitting diode (OLED), a light emitting device, thin film transistor, a battery, a display device or an organic photovoltaic cell (OPV).

According to another aspect not forming part of the present invention, there is provided a method of manufacturing an organic electronic device, the method using:

- at least one deposition source, preferably two deposition sources and more preferred at least three deposition sources.

The methods for deposition that can be suitable comprise:

- deposition via vacuum thermal evaporation;
- deposition via solution processing, preferably the processing is selected from spin-coating, printing, casting; and/or
- slot-die coating.

According to various embodiments not forming part of the present invention, there is provided a method using:

- a first deposition source to release the compound of formula (I) according to the invention, and
- a second deposition source to release the substantially covalent matrix compound;

the method comprising the steps of forming the organic semiconductor layer; whereby for an organic light-emitting diode (OLED):

- the organic semiconductor layer is formed by releasing the compound of formula (I) according to the invention from the first deposition source and the substantially covalent matrix compound from the second deposition source.

According to various embodiments not forming part of the present invention, the method may further include forming on the anode electrode, at least one layer selected from the group consisting of forming a hole transport layer or forming a hole blocking layer, and an emission layer between the anode electrode and the first electron transport layer.

According to various embodiments not forming part of the present invention, the method may further include the steps for forming an organic light-emitting diode (OLED), wherein

- on a substrate an anode electrode is formed,
- on the anode electrode an organic semiconductor layer comprising a compound of formula (I) is formed,
- on the organic semiconductor layer comprising a compound of formula (I) a hole transport layer is formed,
- on the hole transport layer an emission layer is formed,
- on the emission layer an electron transport layer is formed, optionally a hole blocking layer is formed on the emission layer,
- and finally a cathode electrode is formed,
- optional a hole blocking layer is formed in that order between the first anode electrode and the emission layer,
- optional an electron injection layer is formed between the electron transport layer and the cathode electrode.

According to various embodiments not forming part of the present invention, there is provided a method using:

- a first deposition source to release the compound of formula (I) according to the invention, and
- a second deposition source to release the substantially covalent matrix compound;
- a third deposition source to release the n-CGL matrix compound;
- a fourth deposition source to release the n-CGL dopant;

the method comprising the steps of forming the p-type charge generation layer; whereby for an organic light-emitting diode (OLED):

- the p-type charge generation layer is formed by releasing the compound of formula (I) according to the invention from the first deposition source and the substantially covalent matrix compound from the second deposition source;

the method comprising the steps of forming the n-type charge generation layer; whereby for an organic light-emitting diode (OLED):

- the n-type charge generation layer is formed by releasing the n-CGL matrix compound according to the invention from the third deposition source and n-CGL dopant from the fourth deposition source.

According to various embodiments not forming part of the present invention, the method may further include forming on the anode electrode, at least one layer selected from the group consisting of forming a hole transport layer or forming a hole blocking layer, an emission layer and a n-type charge generation layer between the anode electrode and the cathode layer.

According to various embodiments, the OLED may have the following layer structure, wherein the layers having the following order:
anode, organic semiconductor layer comprising a compound of formula (I) according to the invention, first hole transport layer, second hole transport layer, emission layer, optional hole blocking layer, electron transport layer, optional electron injection layer, and cathode.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

Hereinafter, the embodiments are illustrated in more detail with reference to examples. However, the present disclosure is not limited to the following examples. Reference will now be made in detail to the exemplary aspects.

**Description of the Drawings**

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.

Fig. 1 shows a simplified schematic illustration of an organic electronic device without substrate according to the present invention;

Fig. 2 shows a simplified schematic illustration of an organic electronic device with substrate according to the present invention;

Fig. 3 shows a simplified schematic illustration of an organic electronic device with substrate according to the present invention;

Fig. 4 shows a simplified schematic illustration of an organic electronic device with substrate according to the present invention.

Fig. 5 shows the power conversion efficiency of a perovskite solar cell comprising the inventive compound A3 (N-(9,9-dimethyl-9H-fluoren-2-yl)-N-(11,11-diphenyl-11H-benzo[a]fluoren-9-yl)dibenzo[b,d]furan-1-amine) or N-([1,1'-biphenyl]-4-yl)-9,9-dimethyl-N-(4-(9-phenyl-9H-carbazol-3-yl)phenyl)-9H-fluoren-2-amine as a comparative compound at 23±2°C after 0h and after ageing at 85°C at 450h.

Hereinafter, the figures are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

Fig. 1 shows a simplified schematic illustration of an organic electronic device without substrate according to the present invention. The organic electronic device **1** is a solar cell and comprises a first electrode **2.** On the first electrode **2** an organic semiconductor layer is applied. On the top of the organic semiconductor layer **4** second electrode **7** is deposited

Fig. 2 shows a simplified schematic illustration of an organic electronic device with substrate according to the present invention. The organic electronic device **1** is a solar cell and comprises a first electrode **2,** which is arranged on a substrate **3.** On the first electrode **2** an organic semiconductor layer is applied. On the top of the organic semiconductor layer **4** second electrode **7** is deposited

Fig. 3 shows a simplified schematic illustration of an organic electronic device with substrate according to the present invention. The organic electronic device **1** is a solar cell and comprises a first electrode **2.** On the first electrode **2** a hole transport layer (HTL) **4** is deposited. The hole transport layer (HTL) **4.** On the hole transport layer (HTL) **4** an photoactive layer **5** is deposited. On the photoactive layer an electron transport layer **6** is deposited. The electron transport layer (ETL) **6.** On the top of the electron transport layer **6** a second electrode **7** is deposited.

Fig. 4 shows a simplified schematic illustration of an organic electronic device with substrate according to the present invention. The organic electronic device **1** is a solar cell and comprises a first electrode **2,** which is arranged on a substrate **3.** On the first electrode **2** a hole transport layer (HTL) **4** is deposited. The hole transport layer (HTL) **4.** On the hole transport layer (HTL) **4** an photoactive layer **5** is deposited. On the photoactive layer an electron transport layer **6** is deposited. The electron transport layer (ETL) **6.** On the top of the electron transport layer **6** a second electrode 7 is deposited.

Fig. 5 shows the power conversion efficiency in percent over the lifetime (ageing time) of a perovskite solar cell comprising the inventive A3 (N-(9,9-dimethyl-9H-fluoren-2-yl)-N-(11,11-diphenyl-11H-benzo[a]fluoren-9-yl)dibenzo[b,d]furan-1-amine) according to invention or a comparative compound according to Table 8 (N-([1,1'-biphenyl]-4-yl)-9,9-dimethyl-N-(4-(9-phenyl-9H-carbazol-3-yl)phenyl)-9H-fluoren-2-amine) at 23±2°C after 0h and after ageing at 85°C at 450h.

Hereinafter, one or more exemplary embodiments of the present invention will be described in detail with, reference to the following examples. However, these examples are not intended to limit the purpose and scope of the one or more exemplary embodiments of the present invention.

**Experimental data/Synthesis of compounds/Synthesis of Intermediates**

**Synthesis of methyl 5-chloro-2-(naphthalen-2-yl)benzoate**

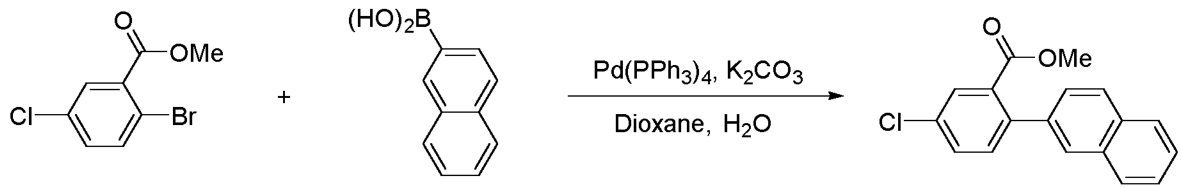


A flask was flushed with nitrogen and charge with methyl 2-bromo-5-chlorobenzoate (50g, 0.20mol), naphthalen-2-ylboronic acid (34.4g, 0.20mol), $Pd(PPh_3)_4$ (11.6g, 0.01mol), $K_2CO_3$ (82.9g, 0.60mol), 1000mL of dioxane and 300mL of distilled water and stirred for 12hours at 120°C. After the reaction was terminated, it was cooled down to room temperature. The organic layer was decanted and dried over $MgSO_4$. The drying agent was filtered off, and the solvent in the organic phase were evaporated under vacuum, and purified by column chromatography. (methyl 5-chloro-2-(naphthalen-2-yl)benzoate was obtained. (47.5g, yield 80%)

**Synthesis of (5-chloro-2-(naphthalen-2-yl)phenyl)diphenylmethanol**

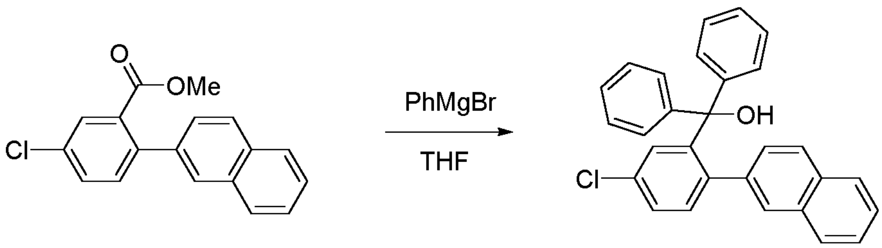


A flask was flushed with nitrogen and charge with 5-chloro-2-(naphthalen-2-yl)benzoate (50g, 0.17mol) and 1000mL of THF. After cooling temperature at -78°C, phenyl magnesium bromide 1M in THF solution 430mL (0.43mol) was adding slowly and stirred for 1 hour at same temperature. After that, stirred for 24hours at room temperature. After the reaction was terminated, the reaction was quenched by 500mL of distilled water. The organic layer was decanted and dried over $MgSO_4$. The drying agent was filtered off, and the solvent in the organic phase were evaporated under vacuum, and purified by column chromatography. ((5-chloro-2-(naphthalen-2-yl)phenyl)diphenylmethanol was obtained. (57.2g, yield 80%)

**Synthesis of 9-chloro-11,11-diphenyl-11H-benzo[a]fluorene**

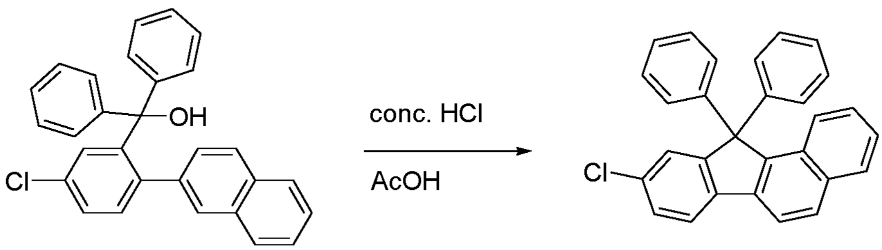


A flask was flushed with nitrogen and charge with (5-chloro-2-(naphthalen-2-yl)phenyl)diphenylmethanol (50g, 0.12mol), 5.2mL of conc. HCl and 780mL of AcOH and stirred for 6hour at 110°C. After the reaction was terminated, it was cooled down to room temperature. After the reaction was terminated, the reaction was quenched by 500mL of distilled water. The organic layer was decanted and dried over $MgSO_4$. The drying agent was filtered off, and the solvent in the organic phase were evaporated under vacuum, and purified by column chromatography. 9-chloro-11,11-diphenyl-11H-benzo[a]fluorene was obtained. (19.3g, yield 40%)

**Synthesis of N-([1,1'-biphenyl]-2-yl)-9,9-dimethyl-9H-fluoren-2-amine**

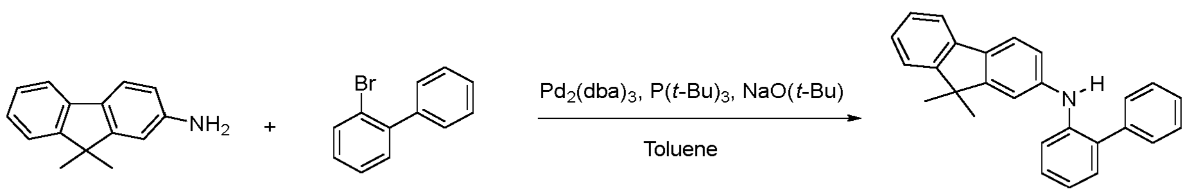


A flask was flushed with nitrogen and charge with 2-bromo-1,1'-biphenyl (11.7g, 1eq, 50mmmol), 9,9-dimethyl-9H-fluoren-2-amine (12.6g, 1.2eq, 60mmol), $Pd_2(dba)_3$ (1.3g, 0.03eq, 1.5mmol), $P(t\text{-}Bu)_3$ (1.01g, 0.1eq, 5mmol), NaO(*t*-Bu) (14.42g, 3eq, 150mmol) and toluene (400 mL) and stirred for 12 hours at 80°C. After the reaction was terminated, it was cooled down to room temperature. The organic layer was decanted and dried over $MgSO_4$. The drying agent was filtered off, and the solvent in the organic phase were evaporated under vacuum, and purified by column chromatography. N-([1,1'-biphenyl]-2-yl)-9,9-dimethyl-9H-fluoren-2-amine; CAS 1198395-24-2, was obtained. (14.5g, yield 80%)

**Synthesis of N-([1,1'-biphenyl]-2-yl)-9,9-dimethyl-9H-fluoren-3-amine**

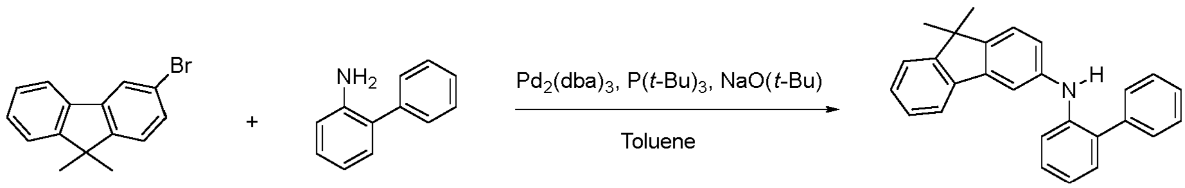


A flask was flushed with nitrogen and charge with 3-bromo-9,9-dimethyl-9H-fluorene (13.7g, 1eq, 50mmmol), [1,1'-biphenyl]-2-amine (10.2g, 1.2eq, 60mmol), $Pd_2(dba)_3$ (1.3g, 0.03eq, 1.5mmol), P(*t*-Bu)$_3$ (1.01g, 0.1eq, 5mmol), NaO(t-Bu) (14.42g, 3eq, 150mmol) and toluene (400 mL) and stirred for 12 hours at 80°C. After the reaction was terminated, it was cooled down to room temperature. The organic layer was decanted and dried over $MgSO_4$. The drying agent was filtered off, and the solvent in the organic phase were evaporated under vacuum, and purified by column chromatography. N-([1,1'-biphenyl]-2-yl)-9,9-dimethyl-9H-fluoren-3-amine; CAS 1421789-39-0, was obtained. (13.6g, yield 75%)

**Synthesis of N-(9,9-dimethyl-9H-fluoren-2-yl)dibenzo[b,d]furan-1-amine**

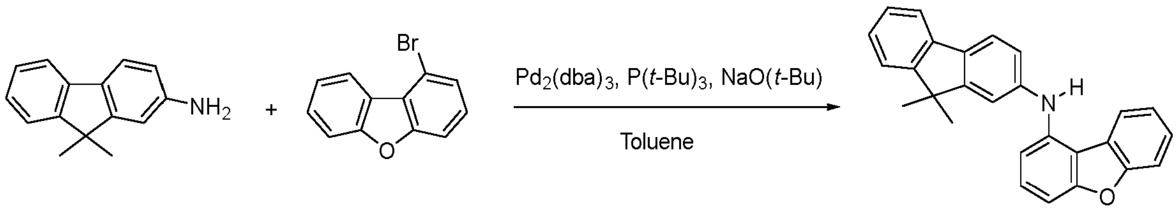


A flask was flushed with nitrogen and charge with 1-bromodibenzo[b,d]furan (12.4g, 1eq, 50mmmol), 9,9-dimethyl-9H-fluoren-2-amine (12.6g, 1.2eq, 60mmol), $Pd_2(dba)_3$ (1.3g, 0.03eq, 1.5mmol), P(*t*-Bu)$_3$ (1.01g, 0.1eq, 5mmol), NaO(*t*-Bu) (14.42g, 3eq, 150mmol) and toluene (400 mL) and stirred for 12 hours at 80°C. After the reaction was terminated, it was cooled down to room temperature. The organic layer was decanted and dried over $MgSO_4$. The drying agent was filtered off, and the solvent in the organic phase were evaporated under vacuum, and purified by column chromatography. N-(9,9-dimethyl-9H-fluoren-2-yl)dibenzo[b,d]furan-1-amine; CAS 2225845-23-6, was obtained. (15.4g, yield 82%)

**Synthesis of N-(9,9-dimethyl-9H-fluoren-2-yl)dibenzo[b,d]furan-3-amine**

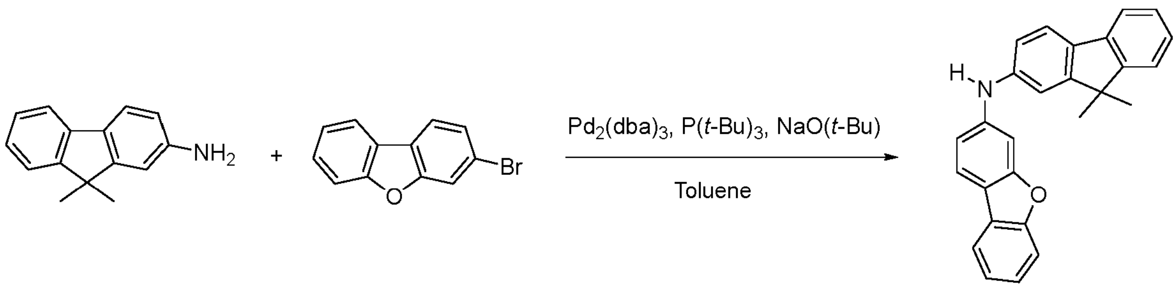


A flask was flushed with nitrogen and charge with 3-bromodibenzo[b,d]furan (12.4g, 1eq, 50mmmol), 9,9-dimethyl-9H-fluoren-2-amine (12.6g, 1.2eq, 60mmol), $Pd_2(dba)_3$ (1.3g, 0.03eq, 1.5mmol), P(*t*-Bu)$_3$ (1.01g, 0.1eq, 5mmol), NaO(*t*-Bu) (14.42g, 3eq, 150mmol) and toluene (400 mL) and stirred for 12 hours at 80°C. After the reaction was terminated, it was cooled down to room temperature. The organic layer was decanted and dried over $MgSO_4$. The drying agent was filtered off, and the solvent in the organic phase were evaporated under vacuum, and purified by column

chromatography. N-(9,9-dimethyl-9H-fluoren-2-yl)dibenzo[b,d]furan-3-amine; CAS 1427556-50-0, was obtained. (16.0g, yield 85%)

**Synthesis of N,9-diphenyl-9H-carbazol-2-amine**

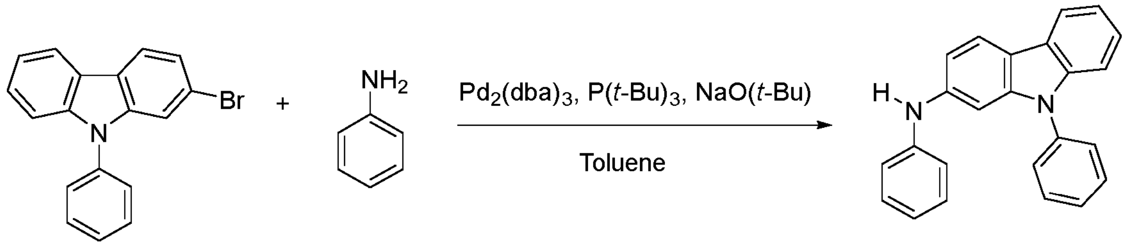


A flask was flushed with nitrogen and charge with 2-bromo-9-phenyl-9H-carbazole (16.1g, 1eq, 50mmmol), aniline (5.6g, 1.2eq, 60mmol), $Pd_2(dba)_3$ (1.3g, 0.03eq, 1.5mmol), P(*t*-Bu)$_3$ (1.01g, 0.1eq, 5mmol), NaO(*t*-Bu) (14.42g, 3eq, 150mmol) and toluene (400 mL) and stirred for 3 hours at 60°C. After the reaction was terminated, it was cooled down to room temperature. The organic layer was decanted and dried over $MgSO_4$. The drying agent was filtered off, and the solvent in the organic phase were evaporated under vacuum, and purified by column chromatography. N,9-diphenyl-9H-carbazol-2-amine; CAS 1427316-55-9, was obtained. (11.7g, yield 70%)

**Synthesis of 7,7-dimethyl-N-phenyl-7H-fluoreno[4,3-b]benzofuran-5-amine**

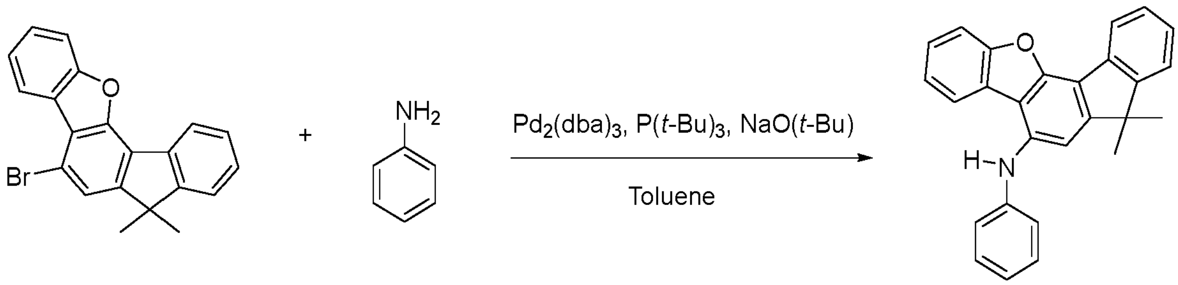


A flask was flushed with nitrogen and charge with 5-bromo-7,7-dimethyl-7H-fluoreno[4,3-b]benzofuran (18.2g, 1eq, 50mmmol), aniline (5.6g, 1.2eq, 60mmol), $Pd_2(dba)_3$ (1.3g, 0.03eq, 1.5mmol), P(*t*-Bu)$_3$ (1.01g, 0.1eq, 5mmol), NaO(t-Bu) (14.42g, 3eq, 150mmol) and toluene (400 mL) and stirred for 3 hours at 60°C. After the reaction was terminated, it was cooled down to room temperature. The organic layer was decanted and dried over $MgSO_4$. The drying agent was filtered off, and the solvent in the organic phase were evaporated under vacuum, and purified by column chromatography. 7,7-dimethyl-N-phenyl-7H-fluoreno[4,3-b]benzofuran-5-amine was obtained. (13.5g, yield 72%)

**Synthesis N-([1,1'-biphenyl]-2-yl)-N-(9,9-dimethyl-9H-fluoren-2-yl)-11,11-diphenyl-11H-benzo[a]fluoren-9-amine (A2)**

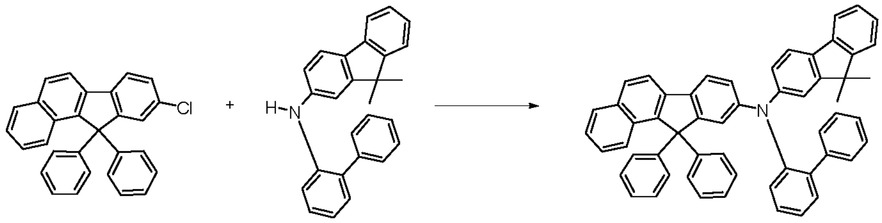

A2

A flask was flushed with nitrogen and charge with 9-chloro-11,11-diphenyl-11H-benzo[a]fluorine (20.15g, 1.0eq, 50mmol), N-([1,1'-biphenyl]-2-yl)-9,9-dimethyl-9H-fluoren-2-amine (18.07g, 1.0eq, 50mmol; CAS 1198395-24-2), $Pd_2(dba)_3$ (1.3g, 0.03eq, 1.5mmol), P(*t*-Bu)$_3$ (1.01g, 0.1eq, 5mmol), NaO(*t*-Bu) (14.42g, 3eq, 150mmol) and toluene (500 mL) and stirred for 12 hours at 110°C. After the reaction was terminated, it was cooled down to room temperature. The organic layer was decanted and dried over $MgSO_4$. The drying agent was filtered off, and the solvent in the organic phase were evaporated under vacuum. The crude product was dissolved in toluene, and then the resulting solution was filtered over silica using toluene as eluent. The organic solution was evaporated under vacuum, and acetone was added. And then the suspension was stirred at room temperature till precipitation. The solid was filtered, rinsed with acetone and dried overnight at 60°C under vacuum, 18.78g of crude product was obtained. Further purification was achieved by means of gradient sublimation. (Sublimation yield 91%, HPLC purity 100% after sublimation)

**Synthesis N-([1,1'-biphenyl]-2-yl)-N-(9,9-dimethyl-9H-fluoren-3-yl)-11,11-diphenyl-11H-benzo[a]fluoren-9-amine (A3)**

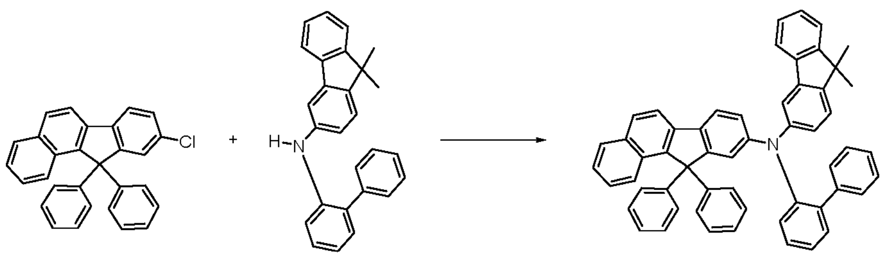

A3

A flask was flushed with nitrogen and charge with 9-chloro-11,11-diphenyl-11H-benzo[a]fluorine (20.65g, 1.025eq, 51.25mmol), N-([1,1'-biphenyl]-2-yl)-9,9-dimethyl-9H-fluoren-3-amine (18.07g, 1.0eq, 50mmol; CAS 1421789-39-0), $Pd_2(dba)_3$ (1.3g, 0.03eq, 1.5mmol), P(*t*-Bu)$_3$ (1.01g, 0.1eq, 5mmol), NaO(t-Bu) (14.42g, 3eq, 150mmol) and toluene (400 mL) and stirred for 12 hours at 110°C. After the reaction was terminated, it was cooled down to room temperature. The organic layer was decanted and dried over $MgSO_4$. The drying agent was filtered off, and the solvent in the organic phase were evaporated under vacuum. The crude product was dissolved in toluene, and then the resulting solution was filtered over silica using toluene as eluent. The organic solution was evaporated under vacuum, and acetone was added. And then the suspension was stirred at room temperature till precipitation. The solid was filtered, rinsed with acetone and dried overnight at 60°C under vacuum, 22.70g of crude product was obtained. Further purification was achieved by means of gradient sublimation. (Sublimation yield 90%, HPLC purity 99.97% after sublimation)

**Synthesis N-(9,9-dimethyl-9H-fluoren-2-yl)-N-(11,11-diphenyl-11H-benzo[a]fluoren-9-yl)dibenzo[b,d]furan-1-amine (A5)**

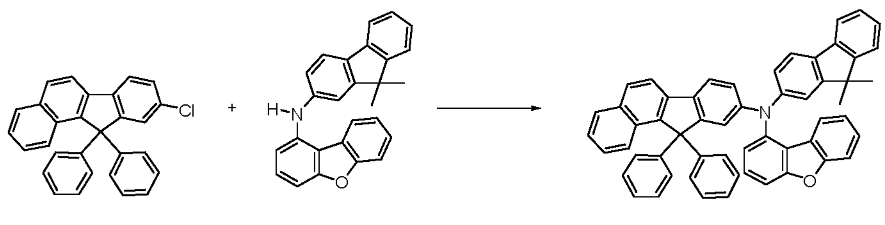

A5

A flask was flushed with nitrogen and charge with 9-chloro-11,11-diphenyl-11H-benzo [a] fluorine (20.15g, 1.0eq, 50mmol), N-(9,9-Dimethyl-9H-fluoren-2-yl)-1-dibenzofuranamine (18.77g, 1.0eq, 50mmol; CAS 2225845-23-6), $Pd_2(dba)_3$ (1.3g, 0.03eq, 1.5mmol), P(*t*-Bu)$_3$ (1.01g, 0.1eq, 5mmol), NaO(*t*-Bu) (14.42g, 3eq, 150mmol) and toluene (400 mL) and stirred for 12 hours at 110°C. After the reaction was terminated, it was cooled down to room temperature. The organic layer was decanted and dried over $MgSO_4$. The drying agent was filtered off, and the solvent in the organic phase were evaporated under vacuum. The crude product was dissolved in toluene, and then the resulting solution was filtered

over silica using toluene as eluent. The organic solution was evaporated under vacuum, and acetone was added. And then the suspension was stirred at room temperature till precipitation. The solid was filtered, rinsed with acetone and dried overnight at 60°C under vacuum, 17.30g of crude product was obtained. Further purification was achieved by means of gradient sublimation. (Sublimation yield 94%, HPLC purity 100% after sublimation)

**Synthesis N-(9,9-dimethyl-9H-fluoren-2-yl)-N-(11,11-diphenyl-11H-benzo[a]fluoren-9-yl)dibenzo[b,d]furan-3-amine (A6)**

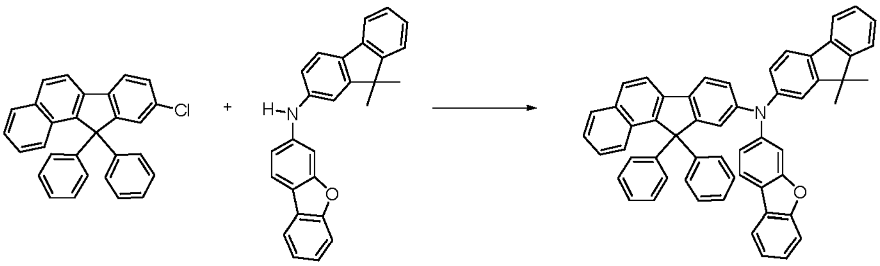

A6

A flask was flushed with nitrogen and charge with 9-chloro-11,11-diphenyl-11H-benzo[a]fluorine (20.65g, 1.025eq, 51.25mmol), N-(9,9-dimethyl-9H-fluoren-2-yl)dibenzo[b,d]furan-3-amine (18.77g, 1.0eq, 50mmol; CAS 1427556-50-0), $Pd_2(dba)_3$ (1.3g, 0.03eq, 1.5mmol), $P(t\text{-}Bu)_3$ (1.01g, 0.1eq, 5mmol), NaO(*t*-Bu) (14.42g, 3eq, 150mmol) and toluene (400 mL) and stirred for 12 hours at 110°C. After the reaction was terminated, it was cooled down to room temperature. The organic layer was decanted and dried over $MgSO_4$. The drying agent was filtered off, and the solvent in the organic phase were evaporated under vacuum. The crude product was dissolved in toluene, and then the resulting solution was filtered over silica using toluene as eluent. The organic solution was evaporated under vacuum, and acetone was added. And then the suspension was stirred at room temperature till precipitation. The solid was filtered, rinsed with acetone and dried overnight at 60°C under vacuum, 26.87g of crude product was obtained. Further purification was achieved by means of gradient sublimation. (Sublimation yield 96%, HPLC purity 99.88% after sublimation)

**Synthesis N-(11,11-diphenyl-11H-benzo[a]fluoren-9-yl)-N,9-diphenyl-9H-carbazol-2-amine (A12)**

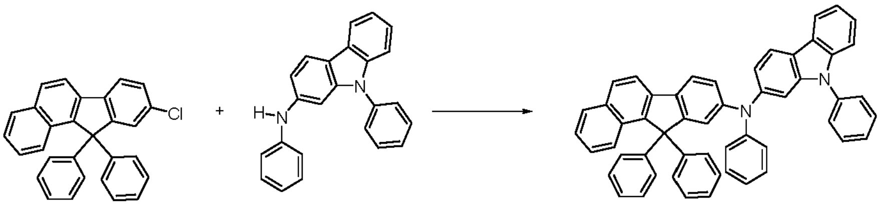

A12

A flask was flushed with nitrogen and charge with 9-chloro-11,11-diphenyl-11H-benzo[a]fluorine (20.65g, 1.025eq, 51.25mmol), N,9-diphenyl-9H-carbazol-2-amine (16.72g, 1.0eq, 50mmol; CAS 1427316-55-9), $Pd_2(dba)_3$ (1.3g, 0.03eq, 1.5mmol), $P(t\text{-}Bu)_3$ (1.01g, 0.1eq, 5mmol), NaO(*t*-Bu) (14.42g, 3eq, 150mmol) and toluene (400 mL) and stirred for 12 hours at 110°C. After the reaction was terminated, it was cooled down to room temperature. The organic layer was decanted and dried over $MgSO_4$. The drying agent was filtered off, and the solvent in the organic phase were evaporated under vacuum. The crude product was dissolved in toluene, and then the resulting solution was filtered over silica using toluene as eluent. The organic solution was evaporated under vacuum, and acetone was added. And then the suspension was stirred at room temperature till precipitation. The solid was filtered, rinsed with acetone and dried overnight at 60°C under vacuum, 28.61g of crude product was obtained. Further purification was achieved by means of gradient sublimation. (Sublimation yield 94%, HPLC purity 99.96% after sublimation)

**Synthesis N-(11,11-diphenyl-11H-benzo[a]fluoren-9-yl)-7,7-dimethyl-N-phenyl-7H-fluoreno[4,3-b]benzofuran-5-amine (A16)**

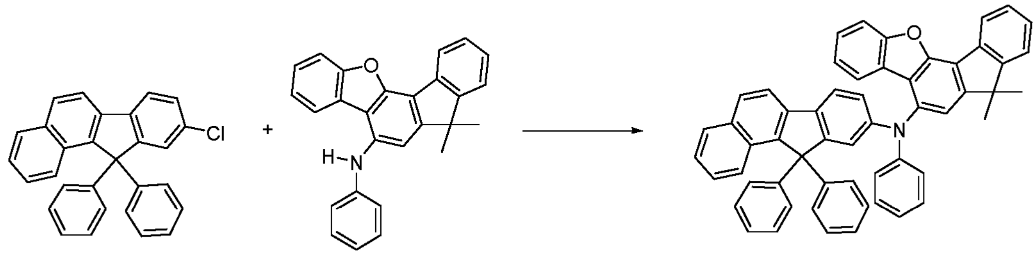

A16

A flask was flushed with nitrogen and charge with 9-chloro-11,11-diphenyl-11H-benzo[a]fluorine (20.65g, 1.025eq, 51.25mmol), 7,7-dimethyl-N-phenyl-7H-fluoreno[4,3-b]benzofuran-5-amine (18.77g, 1.0eq, 50mmol), $Pd_2(dba)_3$ (1.3g, 0.03eq, 1.5mmol), P(*t*-Bu)$_3$ (1.01g, 0.1eq, 5mmol), NaO(t-Bu) (14.42g, 3eq, 150mmol) and toluene (400 mL) and stirred for 12 hours at 110°C. After the reaction was terminated, it was cooled down to room temperature. The organic layer was decanted and dried over $MgSO_4$. The drying agent was filtered off, and the solvent in the organic phase were evaporated under vacuum. The crude product was dissolved in toluene, and then the resulting solution was filtered over silica using toluene as eluent. The organic solution was evaporated under vacuum, and acetone was added. And then the suspension was stirred at room temperature till precipitation. The solid was filtered, rinsed with acetone and dried overnight at 60°C under vacuum, 24.50g of crude product was obtained. Further purification was achieved by means of gradient sublimation. (Sublimation yield 94%, HPLC purity 99.73% after sublimation)

**Detailed description**

The invention is furthermore illustrated by the following examples which are illustrative only and non-binding.

HOMO and LUMO

The HOMO and LUMO are calculated with the program package TURBOMOLE V6.5 (TURBOMOLE GmbH, Litzenhardtstrasse 19, 76135 Karlsruhe, Germany). The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected.

Thermogravimetric analysis

The term "TGA5%" denotes the temperature at which 5 % weight loss occurs during thermogravimetric analysis and is measured in °C.

The TGA5% value may be determined by heating a 9-11 mg sample in a thermogravimetric analyzer at a heating rate of 10 K/min in an open 100 μL aluminum pan, under a stream of nitrogen at a flow rate of 20 mL/min in the balance area and of 30 mL/min in the oven area.

The TGA5% value may provide an indirect measure of the volatility and/or decomposition temperature of a compound. In first approximation, the higher the TGA5% value the lower is the volatility of a compound and/or the higher the decomposition temperature.

According to one embodiment, the TGA5% value of compound of formula (I) is selected in the range of ≥ 270 °C and ≤ 450 °C; preferably of ≥ 280 °C and ≤ 440 °C, also preferred of ≥ 295 °C and ≤ 430 °C.

Glass transition temperature

The glass transition temperature, also named Tg, is measured in °C and determined by Differential Scanning Calorimetry (DSC).

The glass transition temperature is measured under nitrogen and using a heating rate of 10 K per min in a Mettler Toledo DSC 822e differential scanning calorimeter as described in DIN EN ISO 11357, published in March 2010.

General procedure for fabrication of OLEDs

A 15Ω /cm$^2$ glass substrate with 90 nm ITO (available from Corning Co.) was cut to a size of 50 mm x 50 mm x 0.7 mm, ultrasonically washed with isopropyl alcohol for 5 minutes and then with pure water for 5 minutes, and washed again with UV ozone for 30 minutes, to prepare the anode.

Then, compound of N-([1,1'-biphenyl]-4-yl)-9,9-dimethyl-N-(4-(9-phenyl-9H-carbazol-3-yl)phenyl)-9H-fluoren-2-amine (F3) or compound of formula (Ia) according to Table 3 and 4,4',4"-((1E,1'E,1"E)-cyclopropane-1,2,3-triylidenetris(cyanomethanylylidene))tris(2,3,5,6-tetrafluorobenzonitrile) were co-deposited in vacuum on the anode, to form a HIL having a thickness of 10 nm. The composition of the HIL can be seen in Table 3.

Then, compound of N-([1,1'-biphenyl]-4-yl)-9,9-dimethyl-N-(4-(9-phenyl-9H-carbazol-3-yl)phenyl)-9H-fluoren-2-amine or a compound of formula (I) according to Table 3 was vacuum deposited on the HIL, to form a first HTL having a thickness of 128 nm.

Then N-(4-(dibenzo[b,d]furan-4-yl)phenyl)-N-(4-(9-phenyl-9H-fluoren-9-yl)-phenyl)-[1,1'-biphenyl]-4-amine (CAS 1824678-59-2) was vacuum deposited on the HTL, to form an electron blocking layer (EBL) having a thickness of 5 nm.

Then 97 vol.-% H09 (H09 tradename and available by Sun Fine Chemicals, Korea) as EML host and 3 vol.-% BD200 (BD200 tradename and available by Sun Fine Chemicals, Korea) as fluorescent blue dopant were co-deposited on the EBL, to form a first blue-emitting EML with a thickness of 20 nm.

Then the hole blocking layer (HBL) is formed with a thickness of 5 nm by depositing 2-(3'-(9,9-dimethyl-9H-fluoren-2-yl)-[1,1'-biphenyl]-3-yl)-4,6-diphenyl-1,3,5-triazine on the emission layer.

Then, the electron transporting layer (ETL) having a thickness of 31 nm is formed on the hole blocking layer by co-depositing 50 wt.-% 4'-(4-(4-(4,6-diphenyl-1,3,5-triazin-2-yl)phenyl)naphthalen-1-yl)-[1,1'-biphenyl]-4-carbonitrile and 50 wt.-% LiQ.

Then, the cathode having a thickness of 100 nm is formed on the ETL by depositing A1 at a rate of 0.01 to 1 Å/s at $10^{-7}$ mbar.

The OLED stack is protected from ambient conditions by encapsulation of the device with a glass slide. Thereby, a cavity is formed, which includes a getter material for further protection.

To assess the performance of the inventive examples compared to the prior art, the current efficiency is measured at 20°C. The current-voltage characteristic is determined using a Keithley 2635 source measure unit, by sourcing a voltage U in V and measuring the current in mA flowing through the device under test. The voltage applied to the device is varied in steps of 0.1V in the range between 0V and 10V. Likewise, the luminance-voltage characteristics and CIE coordinates are determined by measuring the luminance in cd/m$^2$ using an Instrument Systems CAS-140CT array spectrometer (calibrated by Deutsche Akkreditierungsstelle (DAkkS)) for each of the voltage values. The cd/A efficiency at 10 mA/cm2 is determined by interpolating the luminance-voltage and current-voltage characteristics, respectively.

In bottom emission devices, the emission is predominately Lambertian and quantified in percent external quantum efficiency (EQE). To determine the efficiency EQE in % the light output of the device is measured using a calibrated photodiode at 10 mA/cm2.

In top emission devices, the emission is forward directed, non-Lambertian and also highly dependent on the micro-cavity. Therefore, the external quantum <efficiency EQE will be higher compared to bottom emission devices. To determine the efficiency EQE in % the light output of the device is measured using a calibrated photodiode at 10 mA/cm$^2$.

Lifetime LT of the device is measured at ambient conditions (20°C) and 30 mA/cm$^2$, using a Keithley 2400 source meter, and recorded in hours.

The brightness of the device is measured using a calibrated photo diode. The lifetime LT is defined as the time till the brightness of the device is reduced to 97 % of its initial value.

The increase in operating voltage ΔU is used as a measure of the operational voltage stability of the device. This increase is determined during the LT measurement and by subtracting the operating voltage after 1 hour after the start of operation of the device from the operating voltage after 100 hours.

$$\Delta U=[U100\ h)-\ U(1h)]$$

The smaller the value of ΔU the better is the operating voltage stability.

**General procedure for fabrication of vacuum-processed perovskite solar cells**

ITO coated glass substrates are patterned by photolithography to limit the active area of the solar cell and allow for easy contacting of the top electrode.

Materials used are: p-type dopant 4,4',4"-((1E,1',1"E)-cyclopropane-1,2,3-triylidenetris(cyanomethanylylidene))tris(2,3,5,6-tetrafluorobenzonitrile), the hole transport material as shown in Table 8 and n-type dopant N1,N4-bis(tri-p-tolylphosphoranylidene)benzene-1,4-diamine (PhIm). The electron transport material is fullerene (C60). The precursor materials for the perovskite light absorbing layer are PbI2 and CH3NH3I (MAI).

With regard to characterization of the embodiments prepared, grazing incident X-ray diffraction (GIXRD) pattern are collected at room temperature on an Empyrean PANanalytical powder diffractometer using the Cu K$\alpha$1 radiation. Typically, three consecutive measurements are collected and averaged into single spectra. The surface morphology of the thin films is analyzed using atomic force microscopy (AFM, Multimode SPM, Veeco, USA). Scanning Electron Microscopy (SEM) images is performed on a Hitachi S-4800 microscope operating at an accelerating voltage of 2 kV over Platinum - metallized samples. Absorption spectra are collected using a fiber optics based Avantes Avaspec2048 Spectrometer.

Characterization of the solar cells is performed as follows: The external quantum efficiency (EQE) is estimated using the cell response at different wavelength (measured with a white light halogen lamp in combination with band-pass filters), where the solar spectrum mismatch is corrected using a calibrated Silicon reference cell (MiniSun simulator by ECN, the Netherlands).

The current density-voltage (J-V) characteristics are obtained using a Keithley 2400 source measure unit and under white light illumination, and the short circuit current density is corrected taking into account the device EQE. The electrical characterization was validated using a solar simulator by Abet Technologies (Model 10500 with an AM1.5G xenon lamp as the light source). Before each measurement, the exact light intensity is determined using a calibrated Si reference diode equipped with an infrared cut-off filter (KG-3, Schott). The J-V curves are recorded between -0.2 and 1.2 V with 0.01V steps, integrating the signal for 20 ms after a 10 ms delay. This corresponds to a speed of about 0.3 V s-1.

For ageing the solar cell at 85°C samples are measured at 0h and then put on a hot plate (Stuart SD160) of 85°C for 450h. Samples are characterized after sample has reached room temperature.

The device layout used for the solar cells configurations consists in four equal pixels (area of 0.06 cm2, defined as the overlap between the patterned ITO and the top metal contact) measured through a shadow masks with 0.01 cm2 aperture. For hysteresis study, different scan rates (0.1, 0.5 and 1 Vs-1) are used, biasing the device from -0.2 to 1.2 V with 0.01 V steps and vice versa. Light intensity dependence measurements are done by placing 0.1, 1, 10, 20, 50% neutral density filters (LOT-QuantumDesign GmbH) between the light source and the device.

Further, with regard to device preparation, ITO-coated glass substrates are subsequently cleaned with soap, water and isopropanol in an ultrasonic bath, followed by UV-ozone treatment. They are transferred to a vacuum chamber integrated into a nitrogen-filled glovebox (MBraun, H2O and O2 < 0.1 ppm) and evacuated to a pressure of 1·10-6 mbar. The vacuum chamber is equipped with six temperature controlled evaporation sources (Creaphys) fitted with ceramic crucibles. The sources are directed upwards with an angle of approximately 90° with respect to the bottom of the evaporator. The substrate holder to evaporation sources distance is approximately 20 cm. Three quartz crystal microbalance (QCM) sensors are used, two monitoring the deposition rate of each evaporation source and a third one close to the substrate holder monitoring the total deposition rate.

For thickness calibration, firstly the materials hole transport materials according to Table 8 and 4,4',4"-((1E,1'E,1"E)-cyclopropane-1,2,3-triylidenetris(cyanomethanylylidene))tris(2,3,5,6-tetrafluorobenzonitrile), C60 and PhIm are individually sublimed. A calibration factor is obtained by comparing the thickness inferred from the QCM sensors with that measured with a mechanical profilometer (Ambios XP1). Then these materials are co-sublimed at temperatures ranging from 150°C-190 °C for 4,4',4"-((1E,1'E,1"E)-cyclopropane-1,2,3-triylidenetris(cyanomethanylylidene))tris(2,3,5,6-tetrafluorobenzonitrile) to 250°C-315°C for the hole transport material according to Table 8 and C60, and the evaporation rate is controlled by separate QCM sensors and adjusted to obtain the desired doping concentration. In general, the deposition rate for hole transport material according to Table 8 and C60 is kept constant at 0.8 Å s-1 or 0.5 Å s-1. 4,4',4''-((1E,1'E,1''E)-cyclopropane-1,2,3-triylidenetris(cyanomethanylylidene))tris(2,3,5,6-tetrafluorobenzonitrile) was deposited at a rate of 0.08 Å s-1. Phim was deposited at a rate of 0.15 Å s-1. Undoped hole transport materials according to Table 8 were deposited at a rate of 0.8 Å s-1 and undoped C60 layers are deposited at a rate of 0.5 Å s-1.

An ITO-coated glass substrates are subsequently cleaned with soap, water and isopropanol in an ultrasonic bath, followed by UV-ozone treatment to prepare the anode.

Then 10 weight% of 4,4' 4"-((1E,1'E,1"E)-cyclopropane-1,2,3-triylidenetris(cyanomethanylylidene))tris(2,3,5,6-tetrafluorobenzonitrile) and 90 weight% of a hole transport material according Table 8 is co-deposited in vacuum on the anode to form a doped hole transport layer having a thickness of 40 nm, wherein the hole transport material is deposited at 250-315°C at 8x10-6 mbar.

Then the same hole transport material as for the doped hole transport layer (Table 8) is deposited in vacuum on the doped hole transport layer to form an undoped hole transport layer having a thickness of 10nm, , wherein the hole transport material is deposited at 250-315°C at 8x10-6 mbar.

Once the deposition on the ITO is completed, the chamber is vented with dry N2 to replace the crucibles with those containing the precursor materials for the perovskite light absorbing layer deposition, PbI2 and CH3NH3I. The vacuum chamber is evacuated again to a pressure of 10-6 mbar, and the perovskite films (light absorbing layer) are then

obtained by codeposition of the two precursors.

The calibration of the deposition rate for the CH3NH3I is difficult due to non-uniform layers and the soft nature of the material which impedes accurate thickness measurements. Hence, the source temperature of the CH3NH3I is kept constant at 70 °C and the CH3NH3I:PbI2 ratio is controlled off line using grazing incident x-ray diffraction by adjusting the PbI2 deposition temperature. The optimum deposition temperatures are 250 °C for the PbI2 and 70 °C for the CH3NH3I. After deposition of a 500 nm thick perovskite film, the chamber is vented and the crucibles replaced with those containing C60 and PhIm, and evacuated again to a pressure of 10-6 mbar. This process of exchanging crucibles is done to minimize possible crosscontamination between the organic materials and the perovskite precursors. A light-absorption layer (perovskite layer) is formed.

Then pure C60 is deposited at 8x10-6 mbar and 420°C on the light-absorption layer (perovskite layer) to form an undoped electron transport layer having a thickness of 10nm.

Then 30 weight% of Phim and 70 weight% of C60 is co-deposited on the undoped electron transport layer having a thickness of 40nm to from a doped electron transport layer, wherein C60 is deposited at 8x10-6 mbar and 420°C and Phim at 150-190°C at 8x10-6.

In a single evaporation run five substrates (3 by 3 cm) are prepared, each substrate containing four cells. Generally, one substrate is reserved for a reference configuration. Finally the substrates are transferred to a second vacuum chamber where the silver electrode (100 nm thick) is deposited at a rate of 0.7 Å s-1 for 20 min and 1.8 to 2.4 Å s-1 after 20 min at 6x10-6 mbar.

Layer stack details are given in Table 6.

## Technical Effect

Table 1: Calculated HOMO, LUMO and dipole moment of compounds of formula (Ia) and (Ib)

The HOMO and LUMO are calculated with the program package TURBOMOLE V6.5 (TURBOMOLE GmbH, Litzenhardtstrasse 19, 76135 Karlsruhe, Germany). The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected.

Table 1

| Calculated HOMO, LUMO and dipole moment of compounds of formula (Ia) and (Ib) | | | | | |
|---|---|---|---|---|---|
| Compound | Structure | HOMO [eV] | LUMO [eV] | Dipole moment [debye] | Molecular weight [g/mol] |
| Comp. | 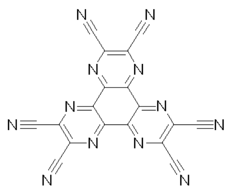 | 8.83 | 4.61 | 0.00 | 384.27 |
| Comparative example 1 | 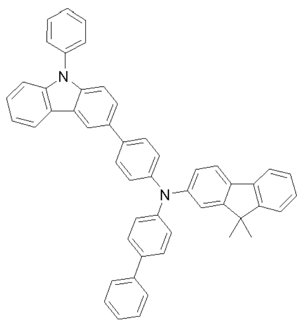 | -4.68 | -0.90 eV | 2.13 | 678.86 |
| A1 | 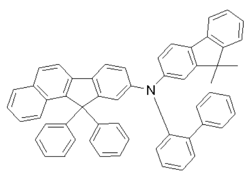 | 4.6825 | 1.1523 | 0.5353 | 727.93 |

(continued)

| Calculated HOMO, LUMO and dipole moment of compounds of formula (Ia) and (Ib) | | | | | |
|---|---|---|---|---|---|
| Compound | Structure | HOMO [eV] | LUMO [eV] | Dipole moment [debye] | Molecular weight [g/mol] |
| A2 | 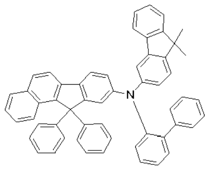 | 4.7362 | 1.1115 | 0.7560 | 727.93 |
| A3 | 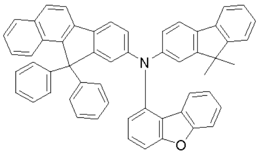 | 4.7964 | 1.2478 | 0.8304 | 741.92 |
| A4 | 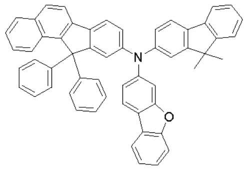 | 4.7141 | 1.2336 | 0.6440 | 741.92 |
| A5 | 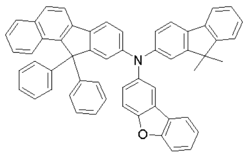 | 4.6905 | 1.2053 | 0.7655 | 741.92 |
| A6 | 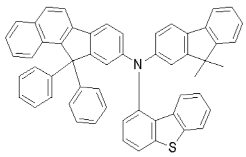 | 4.8062 | 1.2071 | 0.9481 | 741.92 |
| A7 | 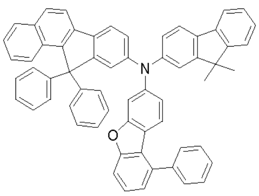 | 4.6975 | 1.2484 | 1.1138 | |
| A8 | 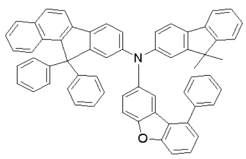 | 4.6506 | 1.1977 | 0.8072 | |
| A9 | 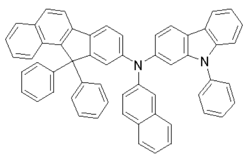 | 4.6686 | 1.1928 | 1.5584 | |

(continued)

| Calculated HOMO, LUMO and dipole moment of compounds of formula (Ia) and (Ib) | | | | | |
|---|---|---|---|---|---|
| Compound | Structure | HOMO [eV] | LUMO [eV] | Dipole moment [debye] | Molecular weight [g/mol] |
| A10 | 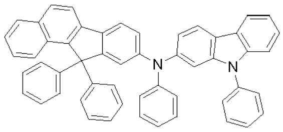 | 4.6736 | 1.1645 | 1.8471 | 700.87 |
| A11 | 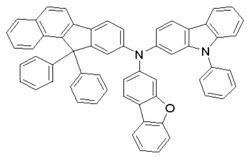 | 4.7022 | 1.2100 | 2.0510 | |
| A12 | 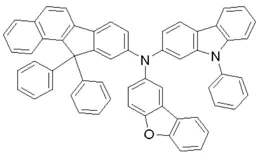 | 4.6764 | 1.1783 | 1.5764 | |
| A13 | 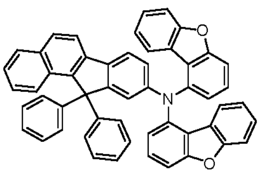 | 4.9660 | 1.2572 | 0.9906 | 715.85 |
| A14 | 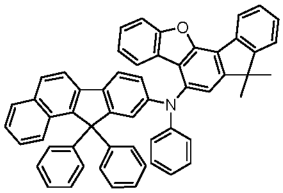 | 4.806 | 1.2325 | 0.8894 | 741.92 |
| A34 | 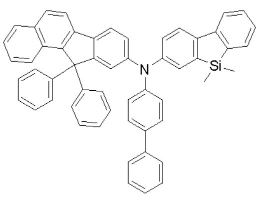 | 4.7067 | 1.2406 | 0.6969 | 744.03 |
| A35 | 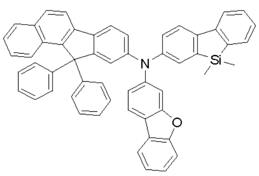 | 4.7234 | 1.2442 | 0.9493 | 758.01 |
| A36 | 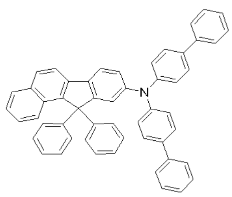 | 4.7679 | 1.2430 | 0.8740 | 687.87 |

Table 2

| Glass transition temperature of the inventive compounds and comparative compounds | | |
|---|---|---|
| Compound | Structure | Tg Glass Transition Onset (from DSC 10K7min) Novamat |
| Comparative example 1 | 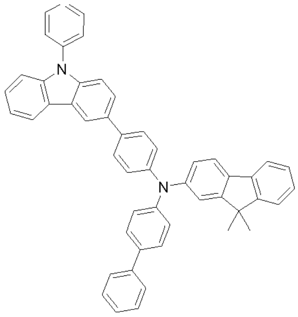 | 126 |
| A1 | 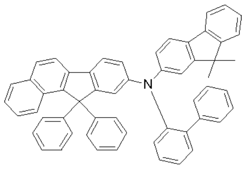 | 141 |
| A2 | 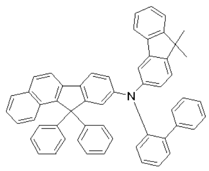 | 141 |
| A3 | 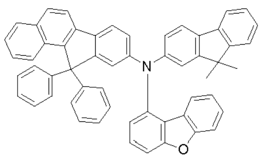 | 156 |
| A4 | 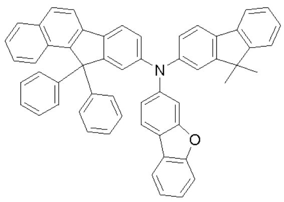 | 155 |
| A10 | 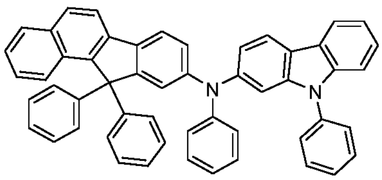 | 142 |
| A14 | 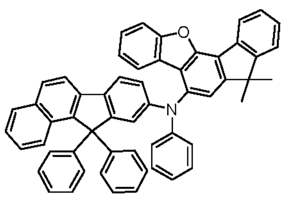 | 160 |

(continued)

| Glass transition temperature of the inventive compounds and comparative compounds | | |
|---|---|---|
| Compound | Structure | Tg Glass Transition Onset (from DSC 10K7min) Novamat |
| A36 | 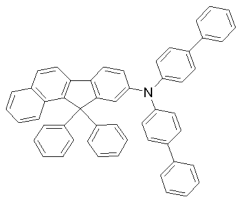 | 137 |
| d.n.=data needed in particular if compound is available yet | | |

It is apparent from Table 2 that the glass transition temperature are much higher for the inventive compounds in comparison to the comparative compound.

A high glass transition temperature may be beneficial for the stability of an organic electronic device, and for the manufacturing process of an organic electronic device.

Table 3

| Performance data of OLED containing the inventive compounds or comparative compounds | | | | | | | | |
|---|---|---|---|---|---|---|---|---|
| No | | Structure (p-HIL) | Conc. [Vol%] | HTL | V at 10mA/cm$^2$ [V] | EQE at 10 mA/cm$^2$ [%] | LT at 30mA/cm$^2$ | Voltage rise at 30mA/cm2 (1-100h) [V] |
| Ex.1 | A1 | 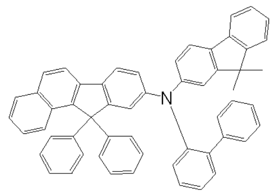 | 2 | F3 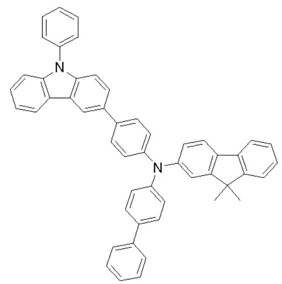 | 3.82 | 9.81 | 94.5 | 0.036 |
| Ex.2 | A1 | 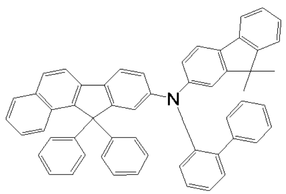 | 2 | A1 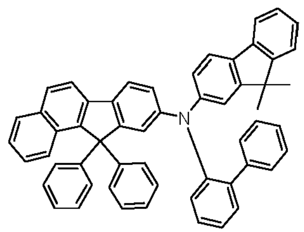 | 3.66 | 9.32 | 111.3 | 0.055 |
| Ex 3 | A36 | 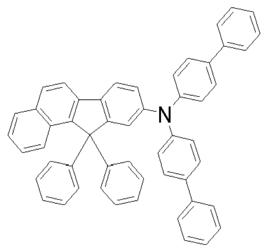 | 3 | F3 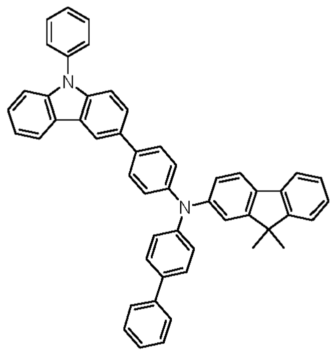 | 3.76 | 9.61 | 107.89 | 0.016 |

(continued)

| Performance data of OLED containing the inventive compounds or comparative compounds | | | | | | | | |
|---|---|---|---|---|---|---|---|---|
| No | | Structure (p-HIL) | Conc. [Vol%] | HTL | V at 10mA/cm$^2$ [V] | EQE at 10 mA/cm$^2$ [%] | LT at 30mA/cm$^2$ | Voltage rise at 30mA/cm2 (1-100h) [V] |
| Ex. 4 | A36 | 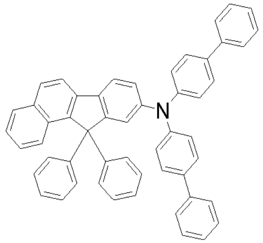 | 3 | A36 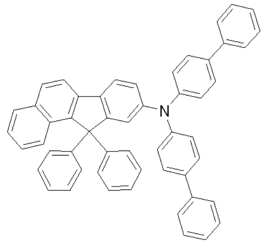 | 3.68 | 8.22 | 181.26 | 0.040 |
| Ex 5 | A3 | 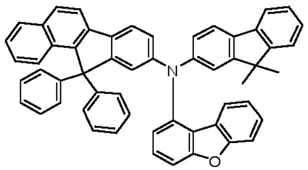 | 3 | F3 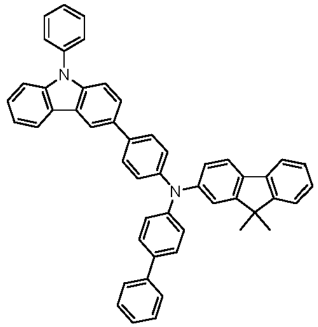 | 3.83 | 9.79 | 78.22 | 0.025 |
| Ex 6 | A3 | 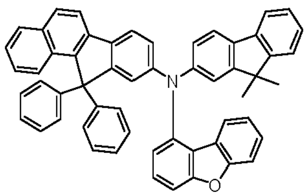 | 3 | A3 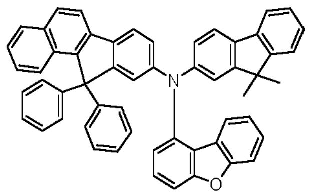 | 3.69 | 9.04 | 87.91 | 0.060 |

(continued)

| Performance data of OLED containing the inventive compounds or comparative compounds | | | | | | | | |
|---|---|---|---|---|---|---|---|---|
| No | | Structure (p-HIL) | Conc. [Vol%] | HTL | V at 10mA/cm$^2$ [V] | EQE at 10 mA/cm$^2$ [%] | LT at 30mA/cm$^2$ | Voltage rise at 30mA/cm2 (1-100h) [V] |
| Ex 7 | A2 | 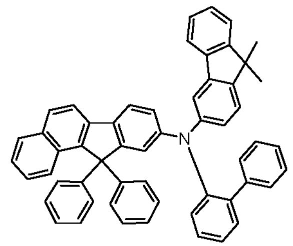 | 2 | F3 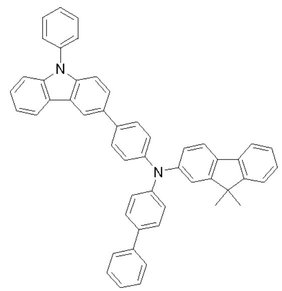 | 3.86 | 9.70 | 105.82 | 0.068 |
| Ex 8 | A2 | 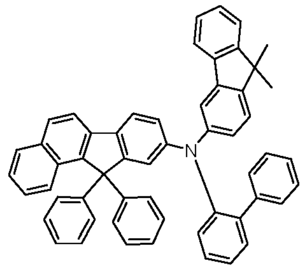 | 2 | A2 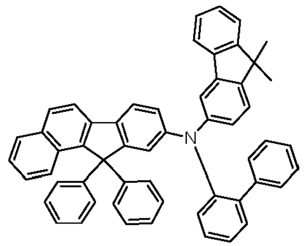 | 3.82 | 10.02 | 106.95 | 0.111 |
| Ex. 9 | A4 | 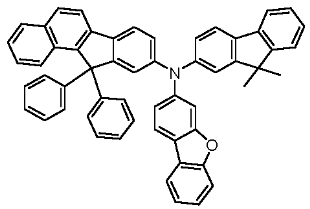 | 2 | F3 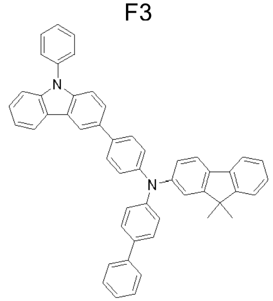 | 3.86 | 9.90 | 118.58 | 0.045 |
| Ex. 10 | A4 | | 2 | A4 | 3.72 | 9.53 | 138.85 | 0.028 |

(continued)

| Performance data of OLED containing the inventive compounds or comparative compounds | | | | | | | | |
|---|---|---|---|---|---|---|---|---|
| No | | Structure (p-HIL) | Conc. [Vol%] | HTL | V at 10mA/cm² [V] | EQE at 10 mA/cm² [%] | LT at 30mA/cm² | Voltage rise at 30mA/cm2 (1-100h) [V] |
| | | 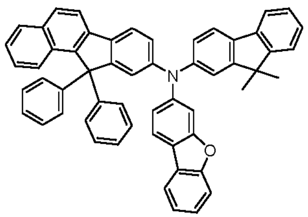 | | 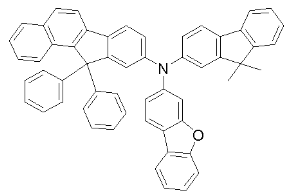 | | | | |
| Ex 11 | A10 | 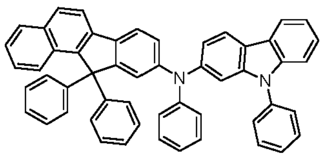 | 4 | F3 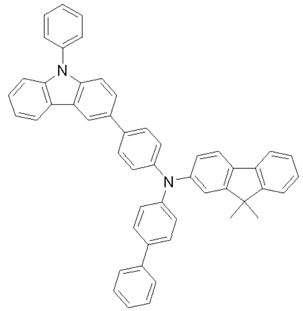 | 3.88 | 10.32 | 123.69 | 0.052 |
| Ex 12 | A10 | 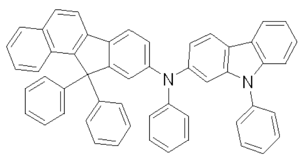 | 4 | A10 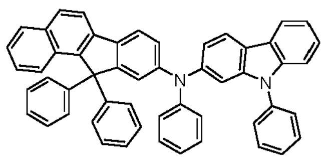 | 3.74 | 10.88 | 95.64 | 0.046 |
| Ex 13 | A14 | | 6 | F3 | 3.84 | 10.39 | 103,38 | 0.039 |

(continued)

| Performance data of OLED containing the inventive compounds or comparative compounds | | | | | | | | |
|---|---|---|---|---|---|---|---|---|
| No | | Structure (p-HIL) | Conc. [Vol%] | HTL | V at 10mA/cm$^2$ [V] | EQE at 10 mA/cm$^2$ [%] | LT at 30mA/cm$^2$ | Voltage rise at 30mA/cm2 (1-100h) [V] |
| | | 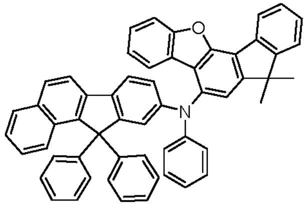 | | 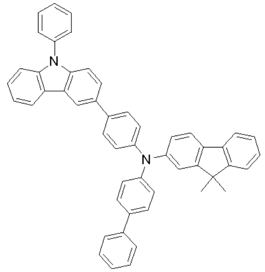 | | | | |
| Ex 14 | A14 | 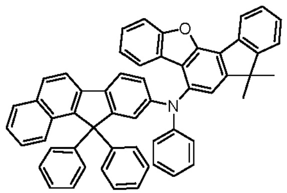 | 6 | A14 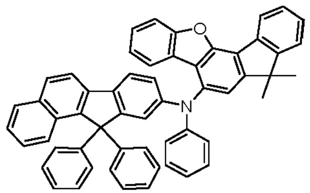 | 3.82 | 10.50 | 117,63 | 0.103 |

As can be seen in Table 3, the devices containing a compound according to formula (Ia) as a HTL exhibits all a lower operating voltage, a higher life time, a higher external quantum efficiency and/or a reduced increase of the operating voltage over time.

The compounds according to the present invention exhibits a lower dipole moment, see Table 4.

Table 4

| Structure | HOMO-1 | HOMO | LUMO | LUMO+1 | E_gap | Dipolemoment | S1 | T1 |
|---|---|---|---|---|---|---|---|---|
| 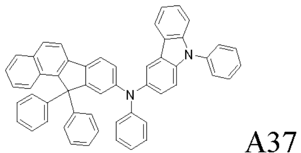 A37 | 5.4353 | 4.5999 | 1.1158 | 0.8245 | 3,4841 | 2.5131 | 3.0786 | 2.3300 |
| 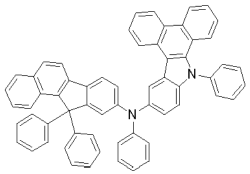 P1-prior art | 5.1963 | 4,6113 | 1,1253 | 1,0599 | 3,4860 | 3,1808 | 3,0515 | 2,3306 |
| 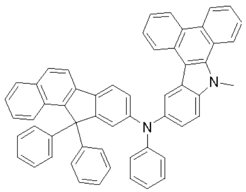 P2-prior art | 5,1939 | 4,6247 | 1,1490 | 1,0813 | 3,4757 | 2,8086 | 3,0319 | 2,3329 |

The compounds of the present invention also exhibits a lower HOMO-1 level than the prior art compounds, see Table 5.

Table 5

| Compound | Structure | HOMO-1 [eV] | HOMO [eV] | LUMO [eV] | LUMO+1 [e | S1 | T1 | Dipole moment [debye] | Molecular weight [g/mol] |
|---|---|---|---|---|---|---|---|---|---|
| A37 | 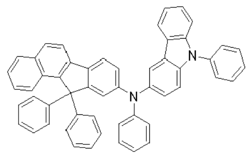 | 5.4353 | 4.5999 | 1.1158 | 0.8245 | 3.0786 | 2.3300 | 2.5131 | 700.89 |
| P1 - Prior art | 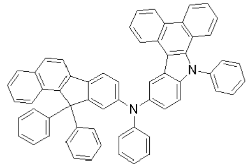 | 5.1963 | 4,6113 | 1,1253 | 1,0599 | 3,0515 | 2,3306 | 3,1808 | 801.01 |
| P2 - Prior art | 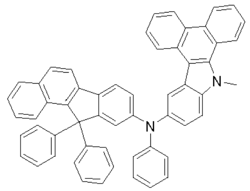 | 5,1939 | 4,6247 | 1,1490 | 1,0813 | 3,0319 | 2,3329 | 2,8086 | 738.93 |
| Comp. | 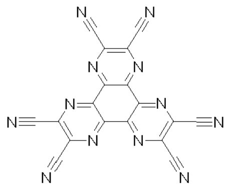 | --- | -8.83 | -4.61 | --- | 3.48 | 2.52 | 0.00 | 384.27 |

(continued)

| Compound | Structure | HOMO-1 [eV] | HOMO [eV] | LUMO [eV] | LUMO+1 [e | S1 | T1 | Dipole moment [debye] | Molecular weight [g/mol] |
|---|---|---|---|---|---|---|---|---|---|
| Comparative example 1 | 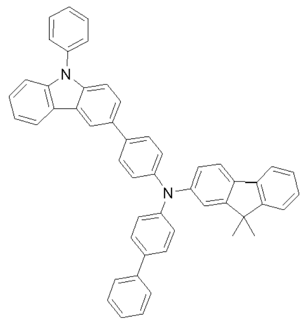 | -5.36 | -4.68 | -0.90 | 0.79 | 3.33 | 2.64 | 2.13 | 678.86 |
| A1 | 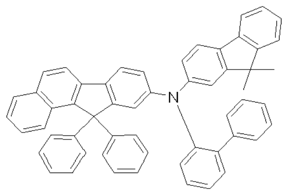 | 5.5136 | 4.6825 | 1.1523 | 0.8701 | 3.1120 | 2.3395 | 0.5353 | 727.93 |
| A2 | 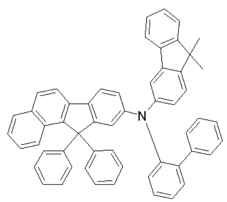 | 5.5453 | 4.7362 | 1.1115 | 0.8214 | 3.1896 | 2.3535 | 0.7560 | 727.93 |
| A3 | 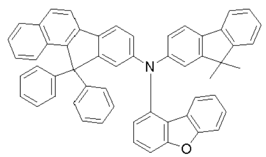 | 5.581 | 4.7964 | 1.2478 | 1.0082 | 3.1163 | 2.3494 | 0.8304 | 741.92 |

(continued)

| Compound | Structure | HOMO-1 [eV] | HOMO [eV] | LUMO [eV] | LUMO+1 [e | S1 | T1 | Dipole moment [debye] | Molecular weight [g/mol] |
|---|---|---|---|---|---|---|---|---|---|
| A4 | 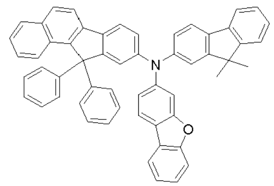 | 5.5836 | 4.7141 | 1.2336 | 1.0052 | 3.0505 | 2.3322 | 0.6440 | 741.92 |
| A5 | 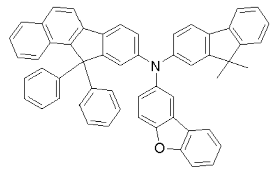 | 5.5632 | 4.6905 | 1.2053 | 1.0288 | 3.0631 | 2.3299 | 0.7655 | 741.92 |
| A6 | 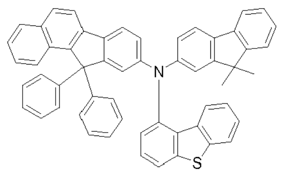 | 5.5619 | 4.8062 | 1.2071 | 1.0462 | 3.1429 | 2.3586 | 0.9481 | 741.92 |
| A7 | 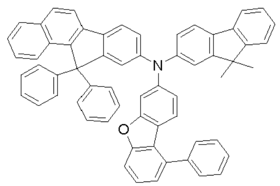 | 5.5730 | 4.6975 | 1.2484 | 1.0946 | 3.0263 | 2.3304 | 1.1138 | 818.03 |
| A8 | 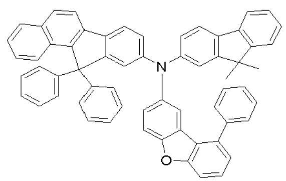 | 5.5361 | 4.6506 | 1.1977 | 1.1536 | 2.9819 | 2.3234 | 0.8072 | 818.03 |

(continued)

| Compound | Structure | HOMO-1 [eV] | HOMO [eV] | LUMO [eV] | LUMO+1 [e | S1 | T1 | Dipole moment [debye] | Molecular weight [g/mol] |
|---|---|---|---|---|---|---|---|---|---|
| A9 | 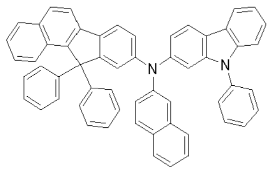 | 5.3856 | 4.6686 | 1.1928 | 0.9502 | 3.0629 | 2.3336 | 1.5584 | 750.95 |
| A10 | 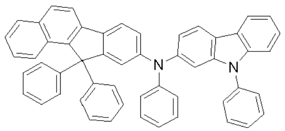 | 5.3700 | 4.6736 | 1.1645 | 0.7933 | 3.0950 | 2.3448 | 1.8471 | 700.87 |
| A11 | 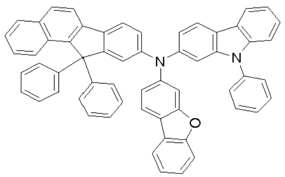 | 5.3794 | 4.7022 | 1.2100 | 0.9348 | 3.0653 | 2.3404 | 2.0510 | 790.97 |
| A12 | 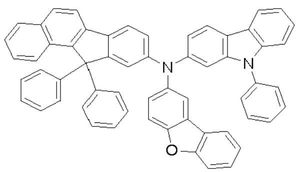 | 5.3741 | 4.6764 | 1.1783 | 0.9711 | 3.0707 | 2.3389 | 1.5764 | 790.97 |
| A13 | 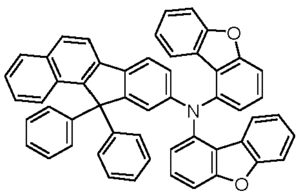 | 5.6885 | 4.9660 | 1.2572 | 1.0157 | 3.2818 | 2.3895 | 0.9906 | 715.85 |

(continued)

| Compound | Structure | HOMO-1 [eV] | HOMO [eV] | LUMO [eV] | LUMO+1 [e | S1 | T1 | Dipole moment [debye] | Molecular weight [g/mol] |
|---|---|---|---|---|---|---|---|---|---|
| A14 | 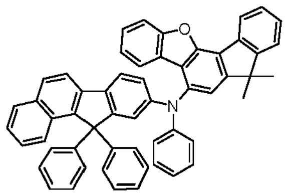 | 5.5792 | 4.806 | 1.2325 | 1.0515 | 3.0712 | 2.3576 | 0.8894 | 741.92 |
| A34 | 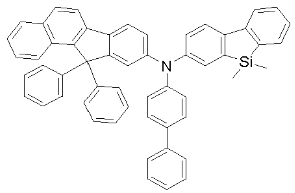 | 5.59 | 4.7067 | 1.2406 | 1.01 | 3.04 | 2.33 | 0.6969 | 744.03 |
| A35 | 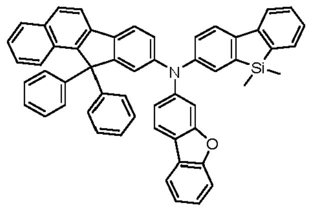 | 5.58 | 4.7234 | 1.2442 | 1.06 | 3.05 | 2.34 | 0.9493 | 758.01 |
| A36 | 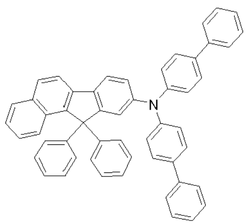 | 5.6208 | 4.7679 | 1.2430 | 0.8771 | 3.1012 | 2.3468 | 0.8740 | 687.87 |

A low operating voltage may be beneficial for reduced power consumption and improved battery life, in particular in mobile devices.

An improved external quantum efficiency may be beneficial for reduced power consumption and improved battery life, in particular in mobile devices.

An improved lifetime is beneficial for improved long-term stability of organic electronic devices.

A reduced increase in operating voltage over time is an indication for improved stability of the electronic device. An increase in lifetime is important for improved stability of the electronic device.

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.

Table 6: List of compounds used for solar cells

Table 6

| Compound Name | IUPAC name | Reference |
|---|---|---|
| Comparative compound (CC) | N-([1,1'-biphenyl]-4-yl)-9,9-dimethyl-N-(4-(9-phenyl-9H-carbazol-3-yl)phenyl)-9H-fluoren-2-amine | |
| A3 | N-(9,9-dimethyl-9H-fluoren-2-yl)-N-(11,11-diphenyl-11H-benzo[a]fluoren-9-yl)dibenzo[b,d]furan-1-amine | US2005121667A1<br>US2005139810A1 |
| p-dopant | 4,4',4"-((1E,1'E,1"E)-cyclopropane-1,2,3-triylidenetris(cyanomethanylylidene))tris(2,3,5,6-tetrafluorobenzonitrile) | |
| MAPI | MAPbI3 prepared in-situ from precursor materials $PbI_2$ and $CH_3NH_3I$ (MAI) | |
| PhIm | N1,N4-bis(tri-p-tolylphosphoranylidene)benzene-1,4-diamine (PhIm)<br>CAS 51870-56-5 | WO2012175219A1 |
| C60 | Fullerene-C60<br>CAS 99685-96-8 | Reed, Bolskar, Chem. Rev. 100, 1075 (2000) |

Table 7: Over of layer stack of inventive solar cell and comparative example

Table 7

| Cell name | Layer Stack |
|---|---|
| Inventive pervoskite solar cell | ITO/p-dopant(10wt%):A3[40nm]/A3[10nm]/MAPI[500nm]/C60[10 nm]/C60(30wt%:Phim[40nm]/Ag |
| Comparative pervoskite solar Cell | ITO/p-dopant(10wt%):CC[40nm]/CC[10nm]/MAPI[500nm]/C60[10nm]/ C60:Phim(30 wt%)[40nm]/Ag |

Table 8: Performance of inventive solar cell - Aged at 85°C

Table 8

| | Hole transport material of doped hole transport layer | Hole transport material of the undoped hole transport layer | $J_{SC}$ (mA $cm^{-2}$) | $V_{OC}$ (mV) | FF (%) | PCE (%) | PCE (%) after 450h at 85°C/PCE(%) after oh at 23 ±2°C |
|---|---|---|---|---|---|---|---|
| Comparative example at 23±2°C After 0h | N-([1,1'-biphenyl]-4-yl)-9,9-dimethyl-N-(4-(9-phenyl-9H-carbazol-3-yl)phenyl)-9H-fluoren-2-amine | N-([1,1'-biphenyl]-4-yl)-9,9-dimethyl-N-(4-(9-phenyl-9H-carbazol-3-yl)phenyl)-9H-fluoren-2-amine | 19.7 | 1.03 | 64% | 13.0% | --- |
| Comparative example aged at 85°C after 450h | N-([1,1'-biphenyl]-4-yl)-9,9-dimethyl-N-(4-(9-phenyl-9H-carbazol-3-yl)phenyl)-9H-fluoren-2-amine | N-([1,1'-biphenyl]-4-yl)-9,9-dimethyl-N-(4-(9-phenyl-9H-carbazol-3-yl)phenyl)-9H-fluoren-2-amine | *19.5* | *0.99* | 44% | *8.5%* | 0.65 |
| Inventive example at 23 ±2°C After 0h | N-(9,9-dimethyl-9H-fluoren-2-yl)-N-(11,11-diphenyl-11H-benzo[a]fluoren-9-yl)dibenzo[b,d]furan-1-amine (A3) | N-(9,9-dimethyl-9H-fluoren-2-yl)-N-(11,11-diphenyl-11H-benzo[a]fluoren-9-yl)dibenzo[b,d]furan-1-amine (A3) | 19.8 | 1.01 | 66% | 13.2% | --- |
| Inventive example at aged at 85°C after 450h | N-(9,9-dimethyl-9H-fluoren-2-yl)-N-(11,11-diphenyl-11H-benzo[a]fluoren-9-yl)dibenzo[b,d]furan-1-amine (A3) | N-(9,9-dimethyl-9H-fluoren-2-yl)-N-(11,11-diphenyl-11H-benzo[a]fluoren-9-yl)dibenzo[b,d]furan-1-amine (A3) | 19.6 | 1.08 | 67% | 14.2% | 1.08 |

After the inventive perovskite solar cell containing compound A3 is aged at 85°C for 450h, the power conversion efficiency is constant or even improved as can be seen from Table 8 and Fig. 5. In contrast, the comparative perovskite solar cell exhibits a strong reduction of the power conversion efficiency when it is aged at 85°C for 450h as can be seen from Table 8 and Fig. 5.

Thus, the inventive perovskite solar cell containing an inventive compound may exhibit an unexpected high power conversion efficiency even after ageing at 85°C for 450h.

The fill factor FF of the inventive perovskite solar cell is constant even after ageing at 85°C for 450h. In contrast, the fill factor FF of the comparative perovskite solar cell decrease remarkably after ageing at 85°C for 450h as can be seen from Table 8.

Thus, the inventive solar cell may be very robust or stable at very harsh conditions.

## Claims

**1.** A benzo diphenyl fluorene compound, represented by formula (I):

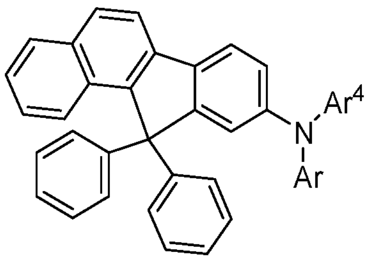

(I),

wherein $Ar^4$ is represented by formula (Ia) or (Ib),

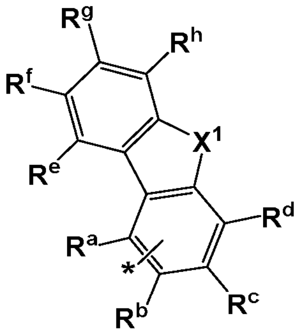

(Ia),

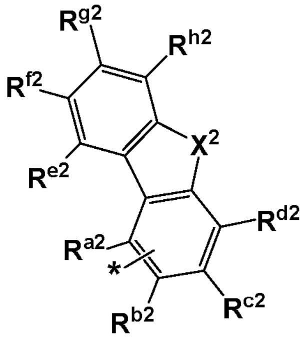

(Ib),

wherein the asterix "*" denotes the binding position of (Ia) and (Ib), and wherein

Ar =$Ar^1$ and $Ar^1$ is selected from substituted or unsubstituted $C_6$ to $C_{24}$ aryl, or substituted or unsubstituted $C_3$ to $C_{25}$ heteroaryl,
at least one substituent on $Ar^1$ is selected from $C_1$ to $C_6$ alkyl, $C_6$ to $C_{12}$ aryl, $C_3$ to $C_{12}$ heteroaryl;
$R^a$, $R^b$, $R^c$ and $R^d$ are independently selected from H, $C_1$ to $C_6$ alkyl, $C_6$ to $C_{18}$ aryl, and $C_2$ to $C_{18}$ heteroaryl, and one of the $R^a$, $R^b$, $R^c$ or $R^d$ represents a single bond that bonds to N of formula (I), optional two of adjacent substituents selected from the group of $R^a$, $R^b$, $R^c$ and $R^d$ form a substituted or unsubstituted condensed ring system, wherein
at least one substituent on the condensed ring system is independently selected from $C_1$ to $C_6$ alkyl, $C_6$ to $C_{12}$ aryl, and $C_2$ to $C_{12}$ heteroaryl;
$R^e$, $R^f$, $R^g$, and $R^h$ are independently selected from H, $C_1$ to $C_6$ alkyl, $C_6$ to $C_{18}$ aryl, and $C_2$ to $C_{18}$ heteroaryl;
$X^1$ is selected from O, S, $NAr^{1a}$;
$Ar^{1a}$ is selected from $C_6$ to $C_{12}$ aryl;
or
Ar =$Ar^2$ and $Ar^2$ is selected from $C_6$ to $C_{12}$ aryl or $C_2$ to $C_{25}$ heteroaryl;
$R^{a2}$, $R^{b2}$, $R^{c2}$ and $R^{d2}$ are independently selected from H, $C_1$ to $C_6$ alkyl, $C_6$ to $C_{18}$ aryl, and $C_2$ to $C_{18}$ heteroaryl, and one of the $R^{a2}$, $R^{b2}$, $R^{c2}$ or $R^{d2}$ represents a single bond that bonds to N of formula (I), optional two of adjacent substituents selected from the group of $R^{a2}$, $R^{b2}$, $R^{c2}$ and $R^{d2}$ form a substituted or unsubstituted condensed ring system, wherein
at least one substituent on the condensed ring system is independently selected from $C_1$ to $C_6$ alkyl, $C_6$ to $C_{12}$ aryl or $C_2$ to $C_{12}$ heteroaryl;
$R^{e2}$, $R^{f2}$, $R^{g2}$, and $R^{h2}$ are independently selected from H, $C_1$ to $C_6$ alkyl, $C_6$ to $C_{18}$ aryl, and $C_2$ to $C_{18}$ heteroaryl;
$X^2$ is selected from O, S, $NAr^{2b}$, $CR^{1b}R^{2b}$, $SiR^{1b}R^{2b}$;
$Ar^{2b}$ is selected from $C_6$ to $C_{12}$ aryl; and
$R^{1b}$ and $R^{2b}$ are independently selected from $C_1$ to $C_6$ alkyl, $C_6$ to $C_{12}$ aryl or $C_2$ to $C_{12}$ heteroaryl;

or represented by compound A36:

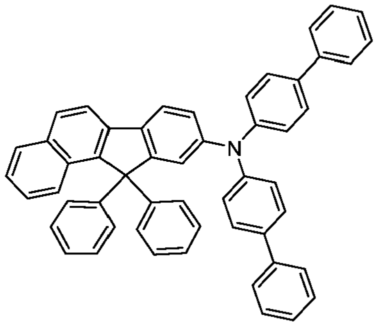

(A36).

2. The benzo diphenyl fluorene compound according to claim 1, wherein the compound of formula (I) is represented by a compound of formula (Ic), (Id) or (Ie), wherein

formula (Ic) is:

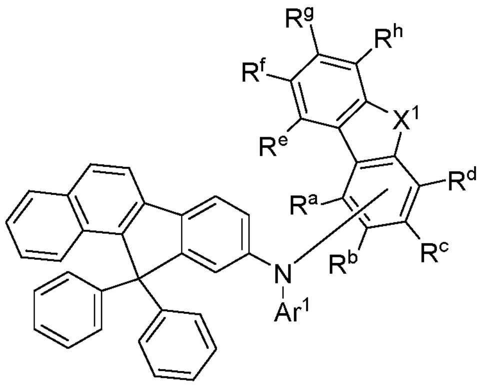

(Ic),

wherein

Ar[1] is selected from substituted or unsubstituted $C_6$ to $C_{24}$ aryl, or substituted or unsubstituted $C_5$ to $C_{25}$ heteroaryl,
at least one substituent on Ar[1] is selected from $C_1$ to $C_6$ alkyl, $C_6$ to $C_{12}$ aryl, $C_5$ to $C_{12}$ heteroaryl;
$R^a$, $R^b$, $R^c$ and $R^d$ are independently selected from H, $C_1$ to $C_6$ alkyl, $C_6$ to $C_{18}$ aryl, and $C_2$ to $C_{18}$ heteroaryl, and one of the $R^a$, $R^b$, $R^c$ or $R^d$ represents a single bond that bonds to N of formula (I), optional two of adjacent substituents selected from the group of $R^a$, $R^b$, $R^c$ and $R^d$ form a substituted or unsubstituted condensed ring system, wherein
at least one substituent on the condensed ring system is independently selected from $C_1$ to $C_6$ alkyl, $C_6$ to $C_{12}$ aryl or $C_2$ to $C_{12}$ heteroaryl;
$R^e$, $R^f$, $R^g$, and $R^h$ are independently selected from H, $C_1$ to $C_6$ alkyl, $C_6$ to $C_{18}$ aryl, and $C_2$ to $C_{18}$ heteroaryl;
$X^1$ is selected from O, S, $NAr^{1a}$;
$Ar^{1a}$ is selected from $C_6$ to $C_{12}$ aryl;
or

formula (Id) is:

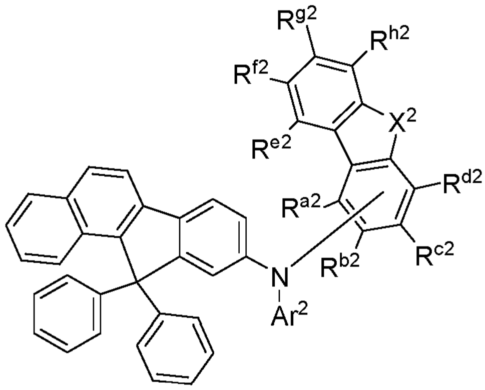

(Id),

wherein

$Ar^2$ is selected from $C_6$ to $C_{12}$ aryl or $C_5$ to $C_{25}$ heteroaryl;
$R^{a2}$, $R^{b2}$, $R^{c2}$ and $R^{d2}$ are independently selected from H, $C_1$ to $C_6$ alkyl, $C_6$ to $C_{18}$ aryl, and $C_2$ to $C_{18}$ heteroaryl, and one of the $R^{a2}$, $R^{b2}$, $R^{c2}$ or $R^{d2}$ represents a single bond that bonds to N of formula (Ib), optional two of adjacent substituents selected from the group of $R^{a2}$, $R^{b2}$, $R^{c2}$ and $R^{d2}$ form a substituted or unsubstituted condensed ring system, wherein
at least one substituent on the condensed ring system is independently selected from $C_1$ to $C_6$ alkyl, $C_6$ to $C_{12}$ aryl or $C_2$ to $C_{12}$ heteroaryl;
$R^{e2}$, $R^{f2}$, $R^{g2}$, and $R^{h2}$ are independently selected from H, $C_1$ to $C_6$ alkyl, $C_6$ to $C_{18}$ aryl, and $C_2$ to $C_{18}$ heteroaryl;
$X^2$ is selected from O, S, $NAr^{2b}$, $CR^{1b}R^{2b}$, $SiR^{1b}R^{2b}$;
$Ar^{2b}$ is selected from $C_6$ to $C_{12}$ aryl; and
$R^{1b}$ and $R^{2b}$ are independently selected from $C_1$ to $C_6$ alkyl, $C_6$ to $C_{12}$ aryl or $C_2$ to $C_{12}$ heteroaryl;
or

formula (Ie) is:

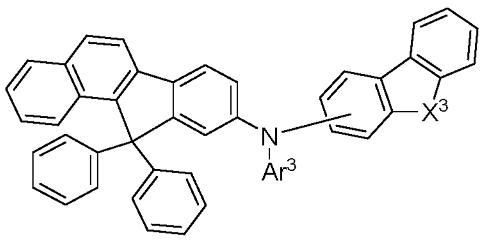

(Ie),

wherein

$Ar^3$ is selected from biphenyl,
$X^3$ is selected from O, S, $NAr^{2b}$, $CR^{1b}R^{2b}$;
$Ar^{2b}$ is selected from $C_6$ to $C_{12}$ aryl; and
$R^{1b}$ and $R^{2b}$ are independently selected from $C_1$ to $C_6$ alkyl, $C_6$ to $C_{12}$ aryl or $C_2$ to $C_{12}$ heteroaryl.

**3.** The benzo diphenyl fluorene compound according to claim 1 or 2, wherein for formula (Ic) $X^1$ is selected from O or $NAR^2$, preferably O; and for formula (Id) $X^2$ is selected from O, $NAR^{2b}$, $CR^{1b}R^{2b}$ and $SiR^{1b}R^{2b}$, preferably selected from O, and $CR^{1b}R^{2b}$, and in addition preferred selected from $CR^{1b}R^{2b}$; and for formula (Ie) $X^3$ is selected from O, S and $SiR^{1b}R^{2b}$, preferably selected from O and S, and in addition preferred selected from O.

**4.** The benzo diphenyl fluorene compound according to any of the preceding claims 1 to 3, wherein for formula (Ic) $Ar^1$ is selected from substituted or unsubstituted $C_6$ to $C_{13}$ aryl, and substituted or unsubstituted $C_{12}$ heteroaryl, preferably $Ar^1$ is selected from substituted $C_6$ to $C_{13}$ aryl; and for formula (Id) $Ar^2$ is selected from $C_6$ to $C_{10}$ aryl, and preferably $Ar^2$ is selected from $C_6$ aryl.

**5.** The benzo diphenyl fluorene compound according to any of the preceding claims 1 to 4, wherein Ar, $Ar^1$ and/or $Ar^2$ are selected from the group of B1 to B13:

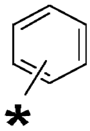 (B1), 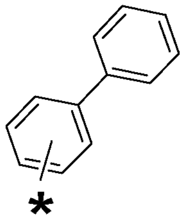 (B2), 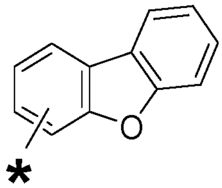 (B3), 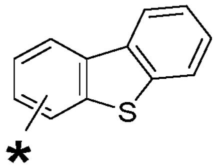 (B4),

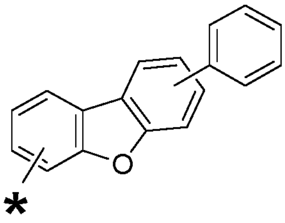 (B5), 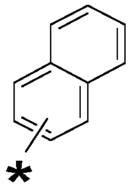 (B6), 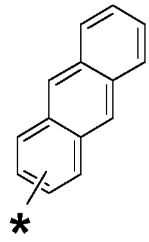 (B7),

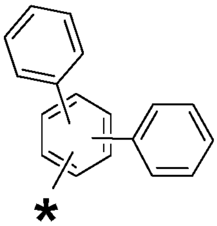 (B8), 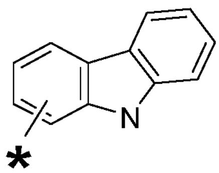 (B9), 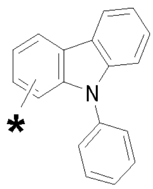 (B10),

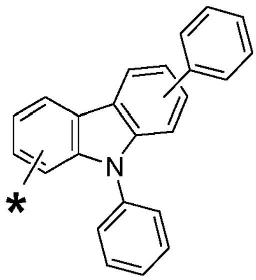 (B11), 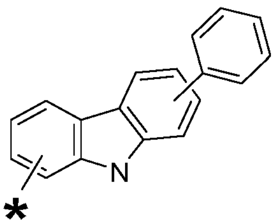 (B12);

wherein the asterix "*" denotes the binding position of Ar, $Ar^1$ and/or $Ar^2$.

**6.** The benzo diphenyl fluorene compound according to claim 5, wherein

- $Ar^1$ is selected from the group of B1 to B12, preferably selected from the group of B1, B2, B3, B4, B5, B6, and in addition preferred selected from the group of B1, B2, B3;
- $Ar^2$ is selected from the group of B1 to B12, preferably selected from the group of B1, B2, B3, B4, B5, B6, and in addition preferred selected from the group of B1, B2, B3.

**7.** The benzo diphenyl fluorene compound to any of the preceding claims 1 to 5, wherein $Ar^4$ is selected from the group of D1 to D7:

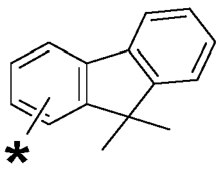 (D1), 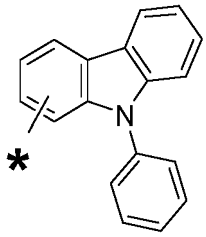 (D2), 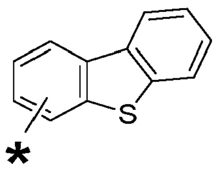 (D3),

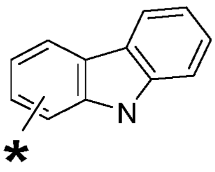 (D4), 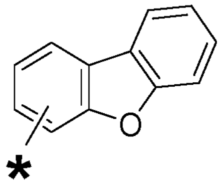 (D5),

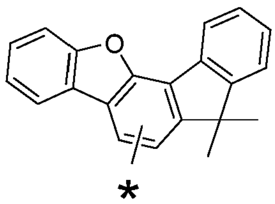 (D6), 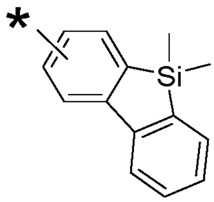 (D7);

wherein the asterix "*" denotes the binding position of $Ar^4$.

**8.** The benzo diphenyl fluorene compound according to any of the preceding claims 1 to 7, wherein the compound of formula I is selected from A1 to A35, 37:

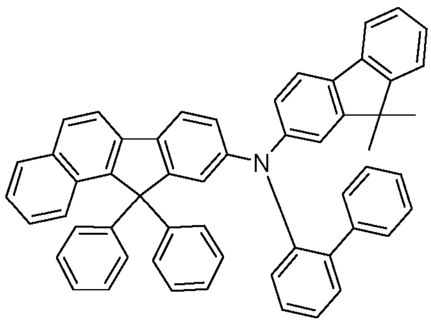 (A1), 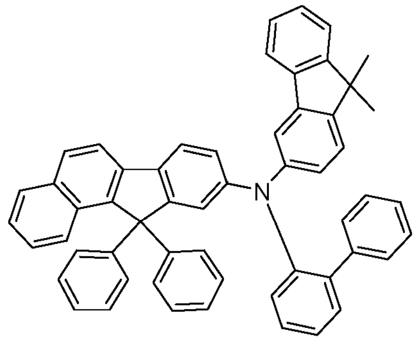 (A2),

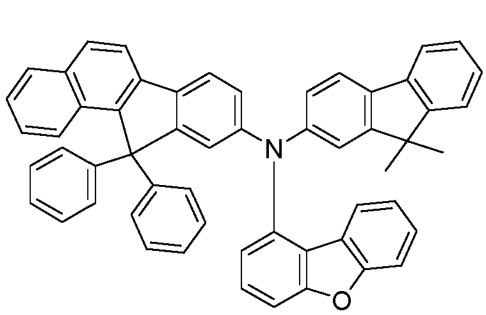 (A3), 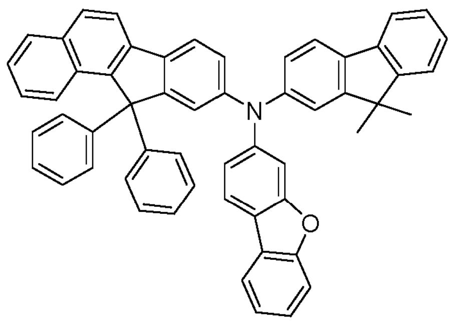 (A4),

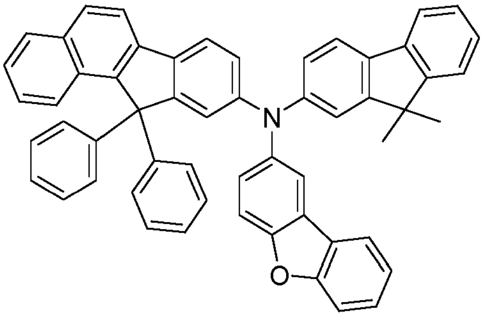
(A5),

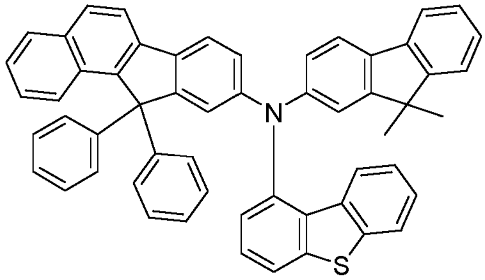
(A6),

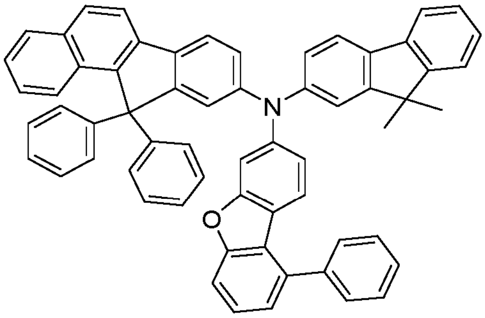
(A7),

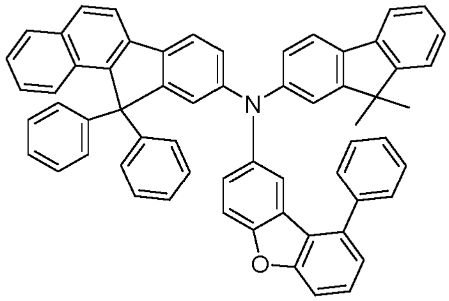
(A8),

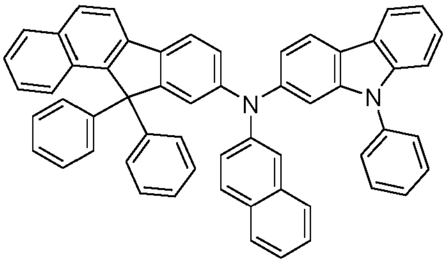
(A9),

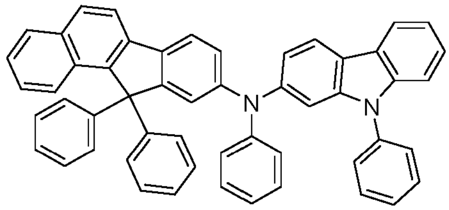
(A10),

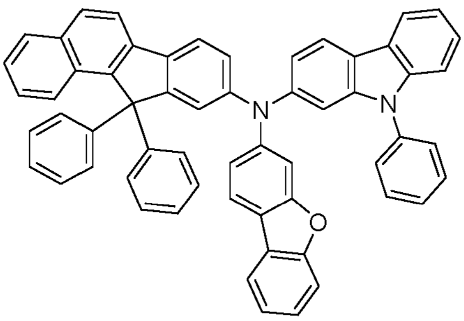
(A11),

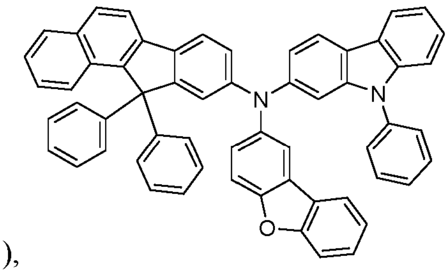
(A12),

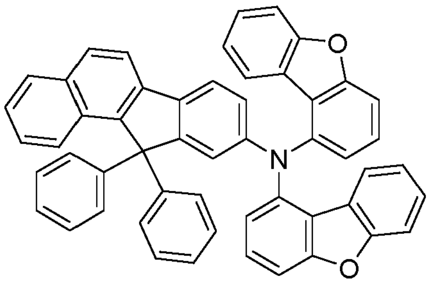
(A13),

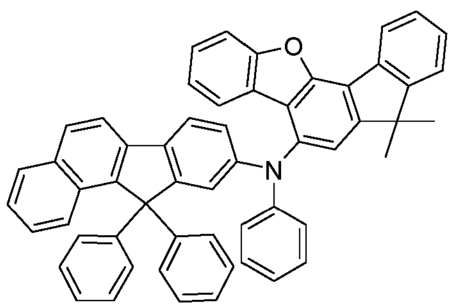
(A14),

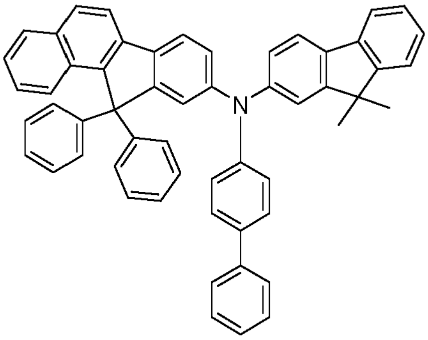
(A15),

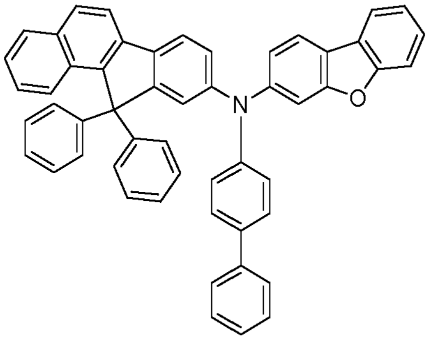
(A16),

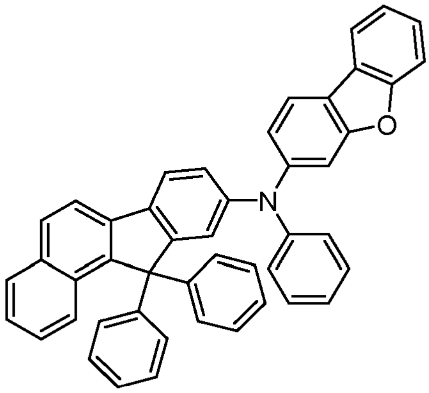
(A17),

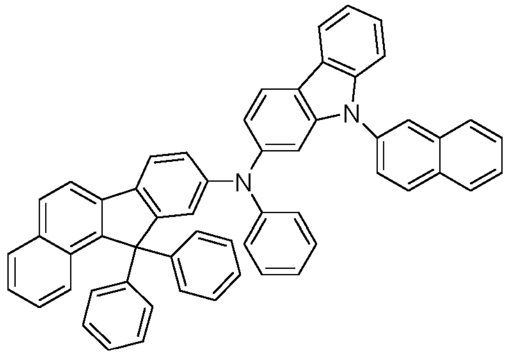
(A18),

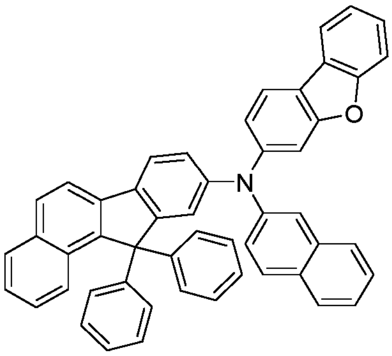
(A19),

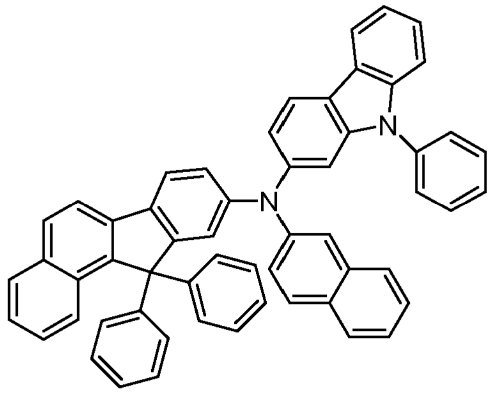
(A20),

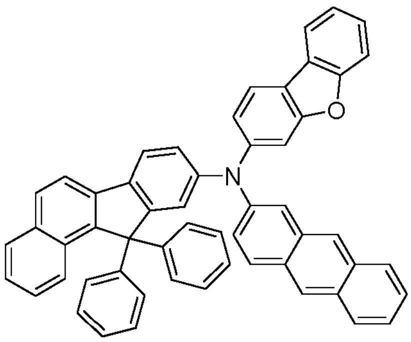
(A21),

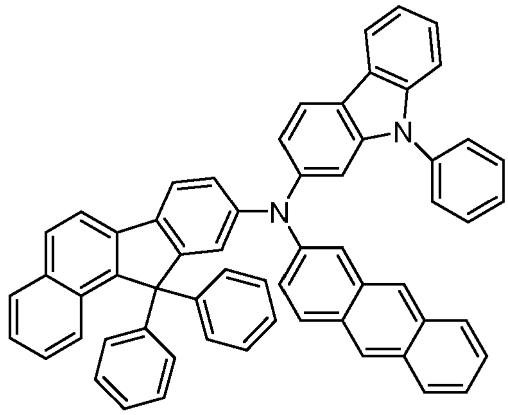
(A22),

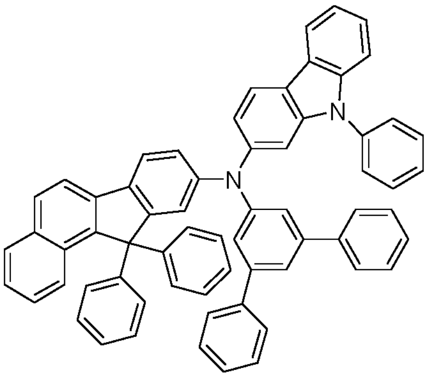
(A23),

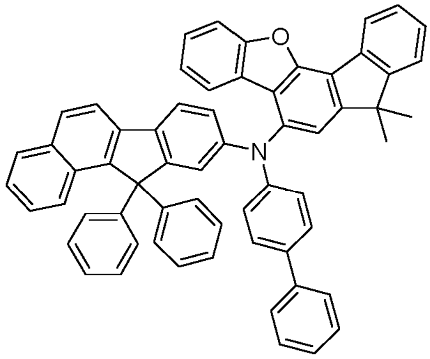
(A24),

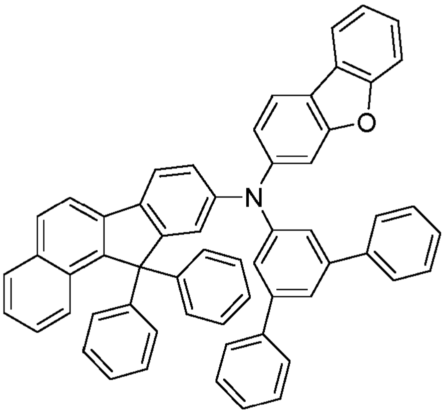
(A25),

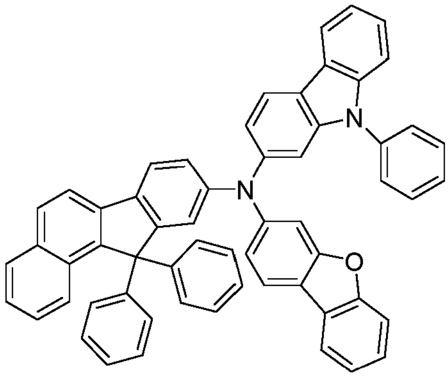
(A26),

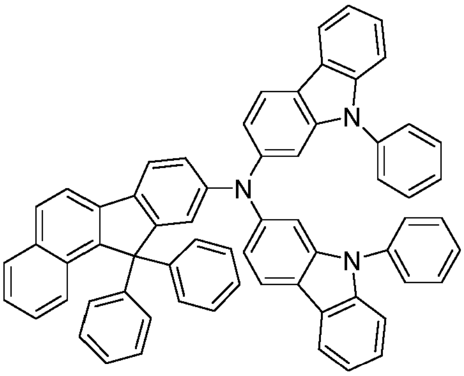
(A27),

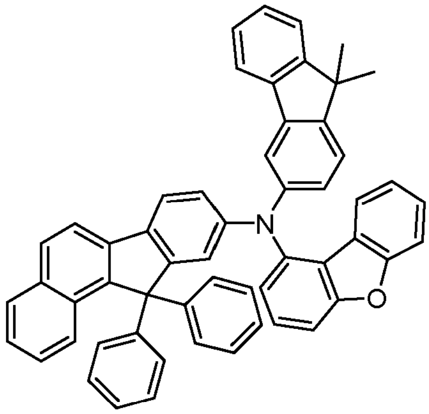
(A28),

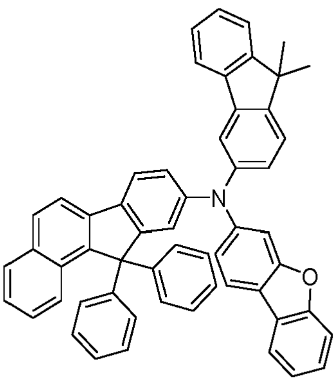
(A29),

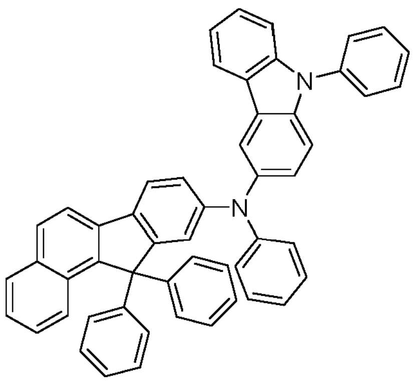
(A30),

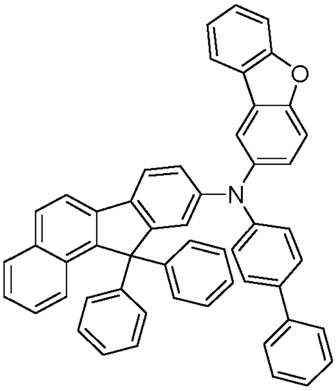

(A31),

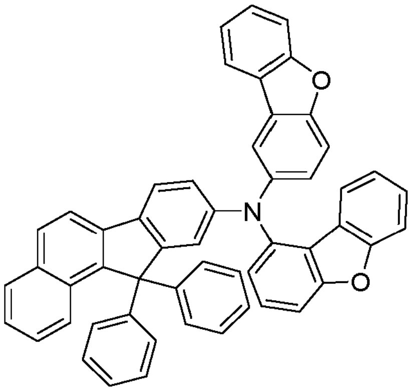

(A32),

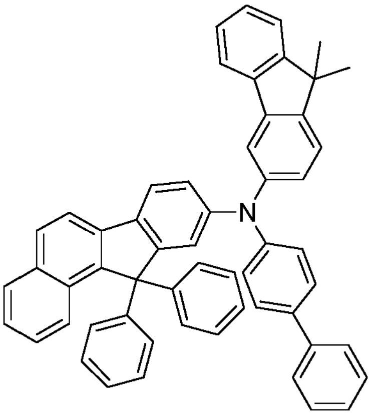

(A33),

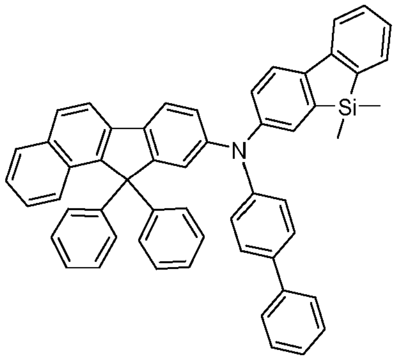

(A34),

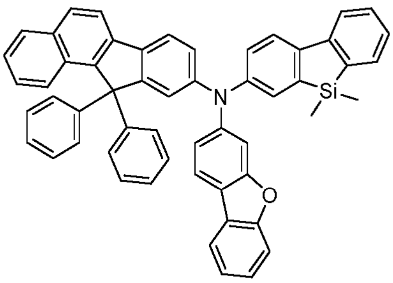

(A35),

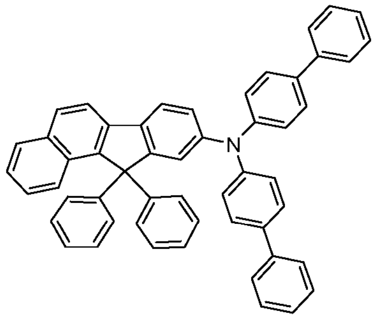

(A36),

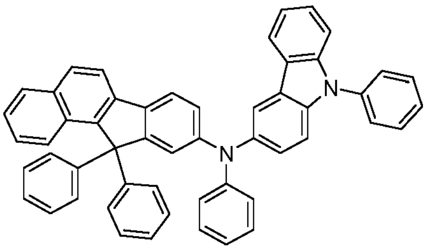

(A37).

**9.** An organic semiconductor layer comprising a benzo diphenyl fluorene compound of formula I according to any of the preceding claims 1 to 8.

**10.** The organic semiconductor layer according to claim 9, wherein the semiconductor layer is a hole injection layer and/or hole transport layer.

**11.** The organic semiconductor layer according to claim 9 or 10, wherein the organic semiconductor layer comprises in

addition an organic p-dopant.

**12.** The organic semiconductor layer according to claim 11, wherein the organic p-dopant is a radialene compound.

**13.** An organic electronic device comprising at least one semiconductor layer according to claims 9 to 12.

**14.** The organic electronic device according to claim 13, comprising an anode layer, a cathode layer and at least one organic semiconductor layer, wherein at least one organic semiconductor layer is arranged between the anode layer and the cathode layer.

**15.** The organic electronic device according to claim 13 or 14, wherein the organic electronic device is an organic light emitting diode (OLED), a light emitting device, thin film transistor, a battery, a display device, an organic photovoltaic cell (OPV), a solar cell, a perovskite solar cell, a photoconductor, photodiode or a photodetector.

## Patentansprüche

**1.** Benzodiphenylfluorenverbindung, wiedergegeben durch Formel (I):

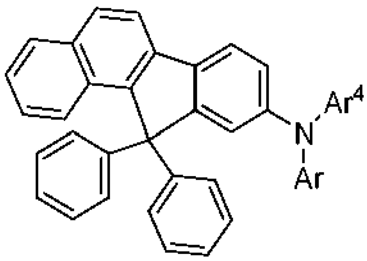

(I),

wobei $Ar^4$ durch Formel (Ia) oder (Ib) wiedergegeben wird,

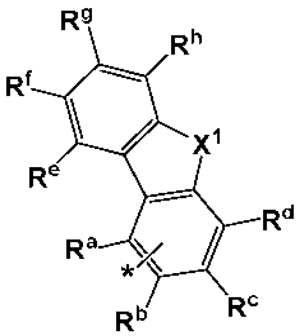

(Ia),

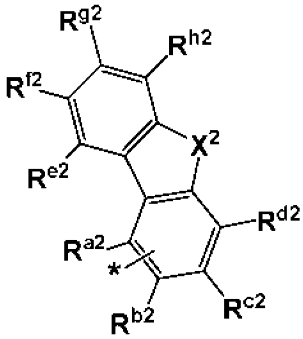

(Ib),

wobei das Sternchen "*" die Anbindungsposition von (Ia) und (Ib) bezeichnet und wobei

Ar = $Ar^1$ und $Ar^1$ aus substituiertem oder unsubstituiertem $C_6$- bis $C_{24}$-Aryl oder substituiertem oder unsubstituiertem $C_3$- bis $C_{25}$-Heteroaryl ausgewählt ist,

mindestens ein Substituent an $Ar^1$ aus $C_1$- bis $C_6$-Alkyl, $C_6$- bis $C_{12}$-Aryl und $C_3$- bis $C_{12}$-Heteroaryl ausgewählt ist; $R^a$, $R^b$, $R^c$ und $R^d$ unabhängig aus H, $C_1$- bis $C_6$-Alkyl, $C_6$-bis $C_{18}$-Aryl und $C_2$- bis $C_{18}$-Heteroaryl ausgewählt sind und eines von $R^a$, $R^b$, $R^c$ oder $R^d$ für eine Einfachbindung steht, die an N der Formel (I) bindet, und gegebenenfalls zwei benachbarte Substituenten aus der Gruppe von $R^a$, $R^b$, $R^c$ und $R^d$ ein substituiertes oder unsubstituiertes kondensiertes Ringsystem bilden, wobei

mindestens ein Substituent an dem kondensierten Ringsystem unabhängig aus $C_1$- bis $C_6$-Alkyl, $C_6$- bis $C_{12}$-Aryl und $C_2$- bis $C_{12}$-Heteroaryl ausgewählt ist;

$R^e$, $R^f$, $R^g$ und $R^h$ unabhängig aus H, $C_1$- bis $C_6$-Alkyl, $C_6$-bis $C_{18}$-Aryl und $C_2$- bis $C_{18}$-Heteroaryl ausgewählt sind;

$X^1$ aus O, S, $NAr^{1a}$ ausgewählt ist;

$Ar^{1a}$ aus $C_6$- bis $C_{12}$-Aryl ausgewählt ist;

oder

Ar = $Ar^2$ und $Ar^2$ aus $C_6$- bis $C_{12}$-Aryl oder $C_2$- bis $C_{25}$-Heteroaryl ausgewählt ist;

$R^{a2}$, $R^{b2}$ $R^{c2}$ und $R^{d2}$ unabhängig aus H, $C_1$- bis $C_6$-Alkyl, $C_6$-bis $C_{18}$-Aryl und $C_2$- bis $C_{18}$-Heteroaryl ausgewählt sind und eines von $R^{a2}$, $R^{b2}$, $R^{c2}$ oder $R^{d2}$ für eine Einfachbindung steht, die an N der Formel (I) bindet, und gegebenenfalls zwei benachbarte Substituenten aus der Gruppe von $R^{a2}$, $R^{b2}$, $R^{c2}$ und $R^{d2}$ ein substituiertes oder unsubstituiertes kondensiertes Ringsystem bilden, wobei

mindestens ein Substituent an dem kondensierten Ringsystem unabhängig aus $C_1$- bis $C_6$-Alkyl, $C_6$- bis $C_{12}$-Aryl oder $C_2$- bis $C_{12}$-Heteroaryl ausgewählt ist;

$R^{e2}$, $R^{f2}$, $R^{g2}$ und $R^{h2}$ unabhängig aus H, $C_1$- bis $C_6$-Alkyl, $C_6$- bis $C_{18}$-Aryl und $C_2$- bis $C_{18}$-Heteroaryl ausgewählt sind;

$X^2$ aus O, S, $NAr^{2b}$, $CR^{1b}R^{2b}$ und $SiR^{1b}R^{2b}$ ausgewählt ist; $Ar^{2b}$ aus $C_6$- bis $C_{12}$-Aryl ausgewählt ist; und $R^{1b}$ und $R^{2b}$ unabhängig aus $C_1$- bis $C_6$-Alkyl, $C_6$- bis $C_{12}$-Aryl oder $C_2$- bis $C_{12}$-Heteroaryl ausgewählt sind;

oder wiedergegeben durch Verbindung A36:

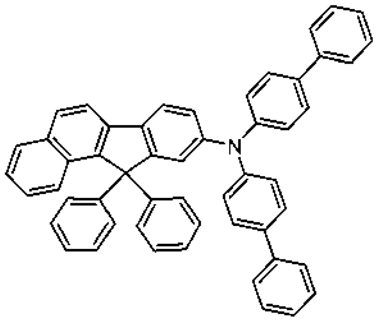

(A36).

**2.** Benzodiphenylfluorenverbindung nach Anspruch 1, wobei die Verbindung der Formel (I) durch eine Verbindung der Formel (Ic), (Id) oder (Ie) wiedergegeben wird, wobei die Formel (Ic) Folgendes ist:

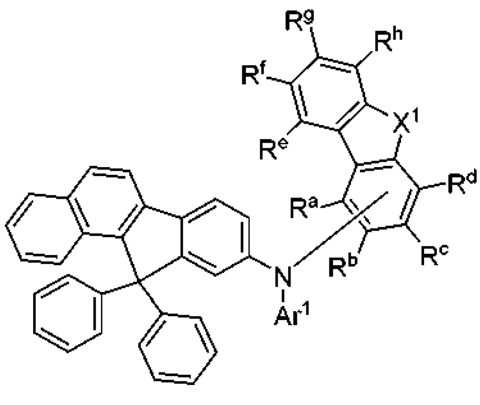

(Ic),

wobei

$Ar^1$ aus substituiertem oder unsubstituiertem $C_6$- bis $C_{24}$-Aryl oder substituiertem oder unsubstituiertem $C_5$- bis $C_{25}$-Heteroaryl ausgewählt ist,

mindestens ein Substituent an $Ar^1$ aus $C_1$- bis $C_6$-Alkyl, $C_6$- bis $C_{12}$-Aryl und $C_5$- bis $C_{12}$-Heteroaryl ausgewählt ist; $R^a$, $R^b$, $R^c$ und $R^d$ unabhängig aus H, $C_1$- bis $C_6$-Alkyl, $C_6$-bis $C_{18}$-Aryl und $C_2$- bis $C_{18}$-Heteroaryl ausgewählt sind und eines von $R^a$, $R^b$, $R^c$ oder $R^d$ für eine Einfachbindung steht, die an N der Formel (I) bindet, und gegebenenfalls zwei benachbarte Substituenten aus der Gruppe von $R^a$, $R^b$, $R^c$ und $R^d$ ein substituiertes oder unsubstituiertes kondensiertes Ringsystem bilden, wobei

mindestens ein Substituent an dem kondensierten Ringsystem unabhängig aus $C_1$- bis $C_6$-Alkyl, $C_6$- bis $C_{12}$-Aryl oder $C_2$- bis $C_{12}$-Heteroaryl ausgewählt ist;

$R^e$, $R^f$, $R^g$ und $R^h$ unabhängig aus H, $C_1$- bis $C_6$-Alkyl, $C_6$-bis $C_{18}$-Aryl und $C_2$- bis $C_{18}$-Heteroaryl ausgewählt sind;

$X^1$ aus O, S, $NAr^{1a}$ ausgewählt ist;

$Ar^{1a}$ aus $C_6$- bis $C_{12}$-Aryl ausgewählt ist;

oder

Formel (Id) Folgendes ist:

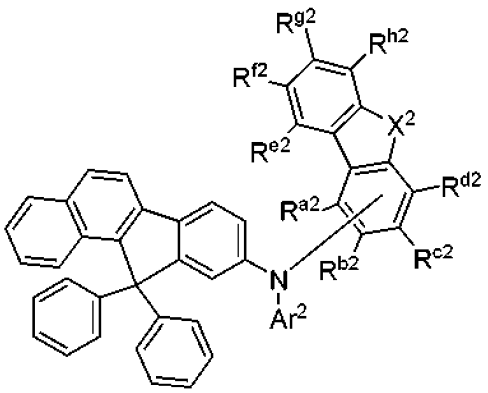

(Id),

wobei

$Ar^2$ aus $C_6$- bis $C_{12}$-Aryl oder $C_5$- bis $C_{25}$-Heteroaryl ausgewählt ist;
$R^{a2}$, $R^{b2}$ $R^{c2}$ und $R^{d2}$ unabhängig aus H, $C_1$- bis $C_6$-Alkyl, $C_6$-bis $C_{18}$-Aryl und $C_2$- bis $C_{18}$-Heteroaryl ausgewählt sind und eines von $R^{a2}$, $R^{b2}$, $R^{c2}$ oder $R^{d2}$ für eine Einfachbindung steht, die an N der Formel (Ib) bindet, und gegebenenfalls zwei benachbarte Substituenten aus der Gruppe von $R^{a2}$, $R^{b2}$, $R^{c2}$ und $R^{d2}$ ein substituiertes oder unsubstituiertes kondensiertes Ringsystem bilden, wobei mindestens ein Substituent an dem kondensierten Ringsystem unabhängig aus $C_1$- bis $C_6$-Alkyl, $C_6$- bis $C_{12}$-Aryl oder $C_2$- bis $C_{12}$-Heteroaryl ausgewählt ist;
$R^{e2}$, $R^{f2}$, $R^{g2}$ und $R^{h2}$ unabhängig aus H, $C_1$- bis $C_6$-Alkyl, $C_6$- bis $C_{18}$-Aryl und $C_2$- bis $C_{18}$-Heteroaryl ausgewählt sind;
$X^2$ aus O, S, $NAr^{2b}$, $CR^{1b}R^{2b}$ und $SiR^{1b}R^{2b}$ ausgewählt ist; $Ar^{2b}$ aus $C_6$- bis $C_{12}$-Aryl ausgewählt ist; und $R^{1b}$ und $R^{2b}$ unabhängig aus $C_1$- bis $C_6$-Alkyl, $C_6$- bis $C_{12}$-Aryl oder $C_2$- bis $C_{12}$-Heteroaryl ausgewählt sind; oder

Formel (Ie) Folgendes ist:

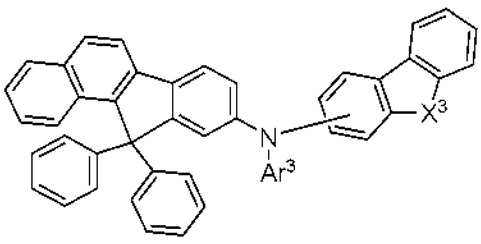

(Ie),

wobei

$Ar^3$ aus Biphenyl ausgewählt ist,
$X^3$ aus O, S, $NAr^{2b}$, $CR^{1b}R^{2b}$ ausgewählt ist;
$Ar^{2b}$ aus $C_6$- bis $C_{12}$-Aryl ausgewählt ist; und
$R^{1b}$ und $R^{2b}$ unabhängig aus $C_1$- bis $C_6$-Alkyl, $C_6$- bis $C_{12}$-Aryl oder $C_2$- bis $C_{12}$-Heteroaryl ausgewählt sind.

**3.** Benzodiphenylfluorenverbindung nach Anspruch 1 oder 2, wobei für Formel (Ic) $X^1$ aus O oder $NAR^2$, vorzugsweise O, ausgewählt ist und für Formel (Id) $X^2$ aus O, $NAR^{2b}$, $CR^{1b}R^{2b}$ und $SiR^{1b}R^{2b}$ ausgewählt ist, vorzugsweise aus O und $CR^{1b}R^{2b}$ ausgewählt ist und zusätzlich bevorzugt aus $CR^{1b}R^{2b}$ ausgewählt ist und für Formel (Ie) $X^3$ aus O, S und $SiR^{1b}R^{2b}$ ausgewählt ist, vorzugsweise aus O und S ausgewählt ist und zusätzlich bevorzugt aus O ausgewählt ist.

**4.** Benzodiphenylfluorenverbindung nach einem der vorhergehenden Ansprüche 1 bis 3, wobei für Formel (Ic) $Ar^1$ aus substituiertem oder unsubstituiertem $C_6$- bis $C_{13}$-Aryl und substituiertem oder unsubstituiertem $C_{12}$-Heteroaryl ausgewählt ist, vorzugsweise $Ar^1$ aus substituiertem $C_6$- bis $C_{13}$-Aryl ausgewählt ist; und für Formel (Id) $Ar^2$ aus

$C_6$- bis $C_{10}$-Aryl ausgewählt ist und vorzugsweise $Ar^2$ aus $C_6$-Aryl ausgewählt ist.

**5.** Benzodiphenylfluorenverbindung nach einem der vorhergehenden Ansprüche 1 bis 4, wobei Ar, $Ar^1$ und/oder $Ar^2$ aus der Gruppe von B1 bis B13 ausgewählt sind:

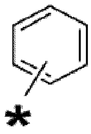 (B1), 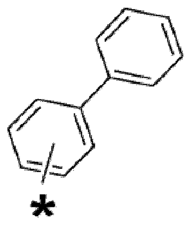 (B2), 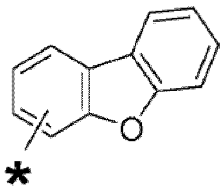 (B3), 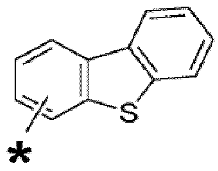 (B4),

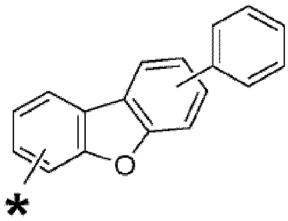 (B5), 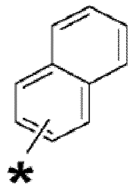 (B6), 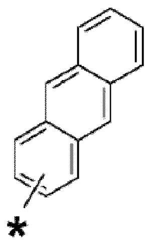 (B7),

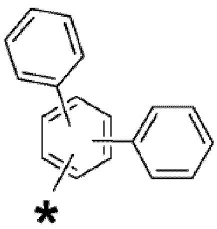 (B8), 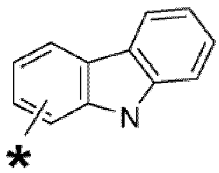 (B9), 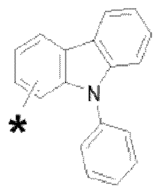 (B10),

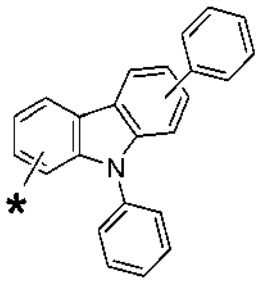 (B11), 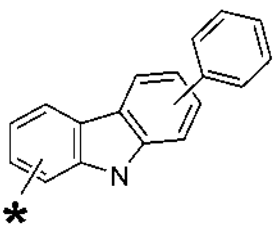 (B12);

wobei das Sternchen "*" die Anbindungsposition von Ar, $Ar^1$ und/oder $Ar^2$ bezeichnet.

**6.** Benzodiphenylfluorenverbindung nach Anspruch 5, wobei

- $Ar^1$ aus der Gruppe von B1 bis B12 ausgewählt ist, vorzugsweise aus der Gruppe von B1, B2, B3, B4, B5, B6 ausgewählt ist und zusätzlich bevorzugt aus der Gruppe von B1, B2, B3 ausgewählt ist;
- $Ar^2$ aus der Gruppe von B1 bis B12 ausgewählt ist, vorzugsweise aus der Gruppe von B1, B2, B3, B4, B5, B6 ausgewählt ist und zusätzlich bevorzugt aus der Gruppe von B1, B2, B3 ausgewählt ist.

**7.** Benzodiphenylfluorenverbindung nach einem der vorhergehenden Ansprüche 1 bis 5, wobei $Ar^4$ aus der Gruppe von D1 bis D7 ausgewählt ist:

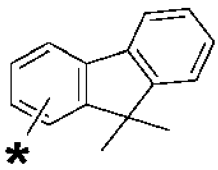 (D1), 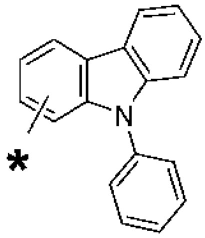 (D2), 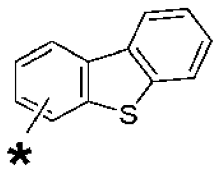 (D3),

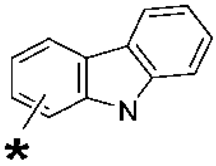 (D4), 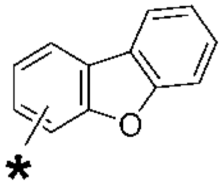 (D5),

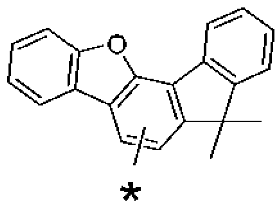 (D6), 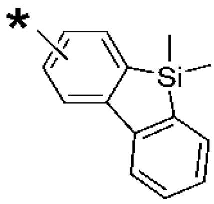 (D7);

wobei das Sternchen "*" die Anbindungsposition von $Ar^4$ bezeichnet.

**8.** Benzodiphenylfluorenverbindung nach einem der vorhergehenden Ansprüche 1 bis 7, wobei die Verbindung der Formel I aus A1 bis A35, 37 ausgewählt ist:

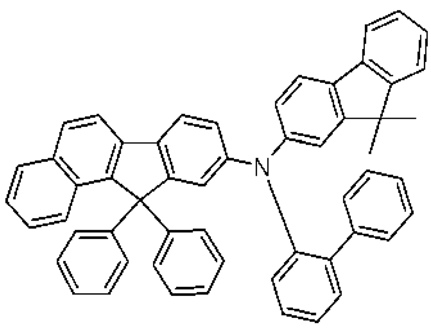 (A1), 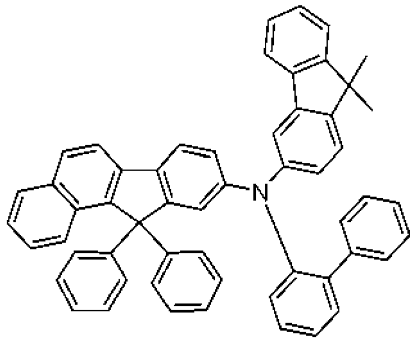 (A2),

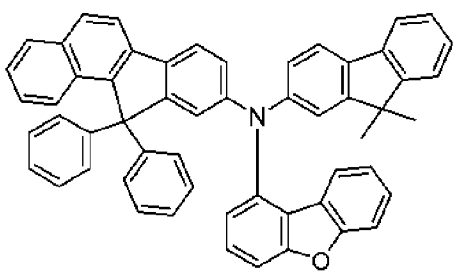 (A3), 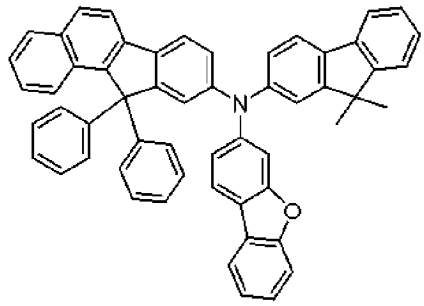 (A4),

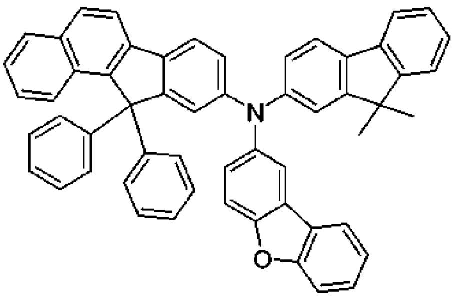
(A5),

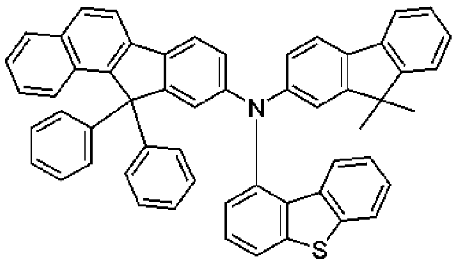
(A6),

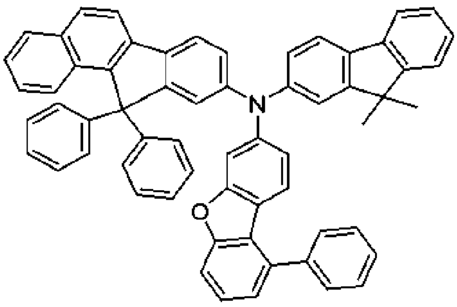
(A7),

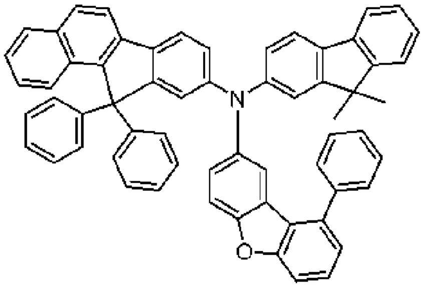
(A8),

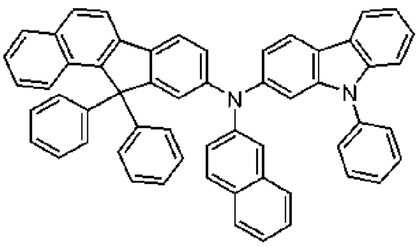
(A9),

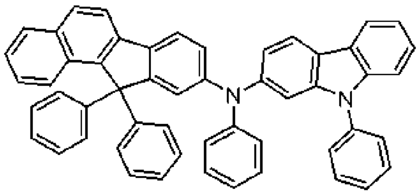
(A10),

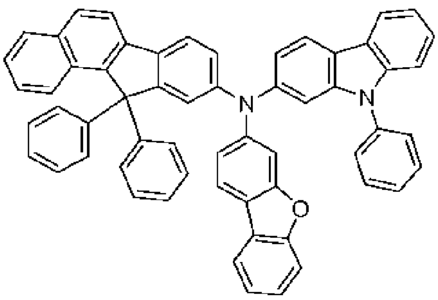
(A11),

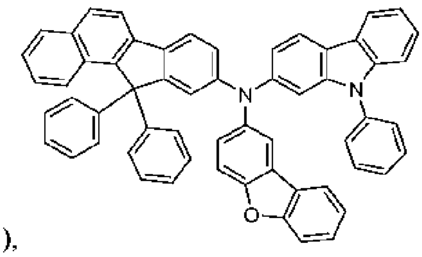
(A12),

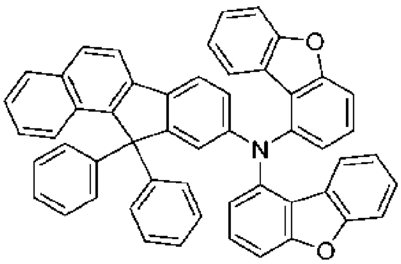
(A13),

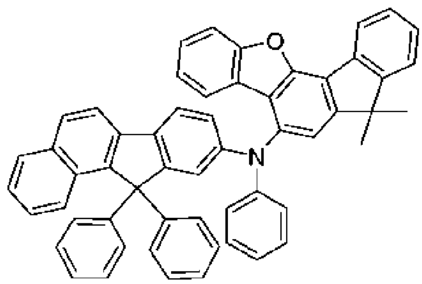
(A14),

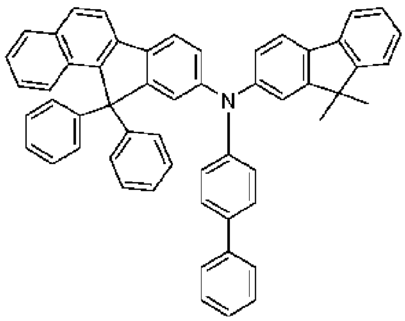
(A15),

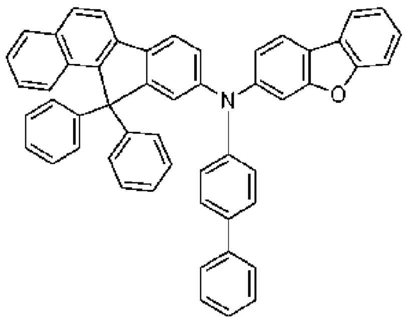
(A16),

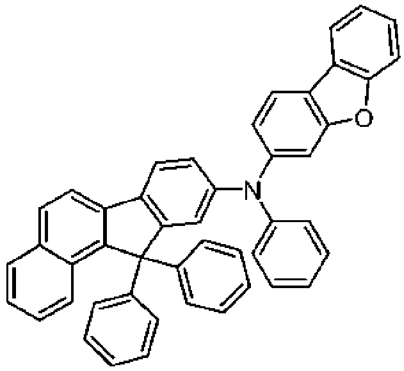
(A17),

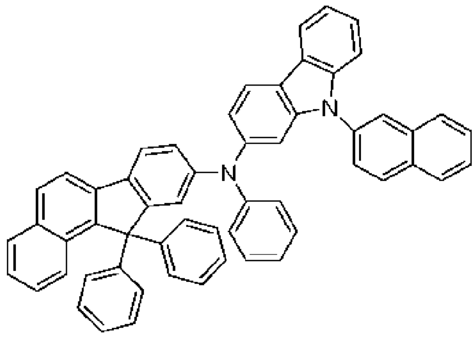
(A18),

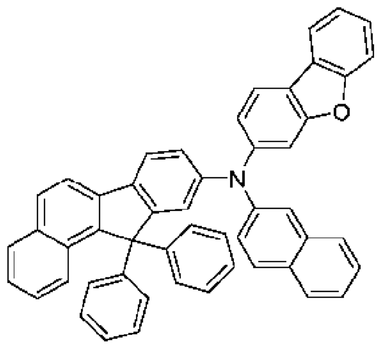
(A19),

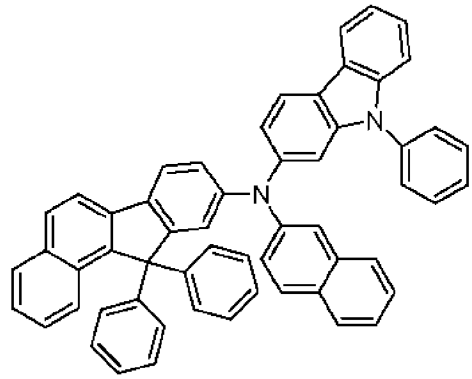
(A20),

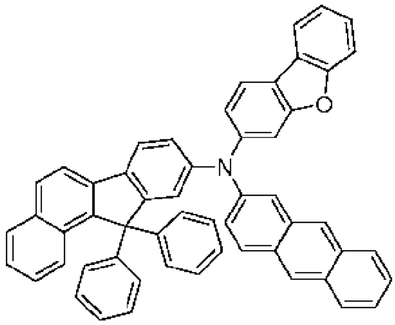
(A21),

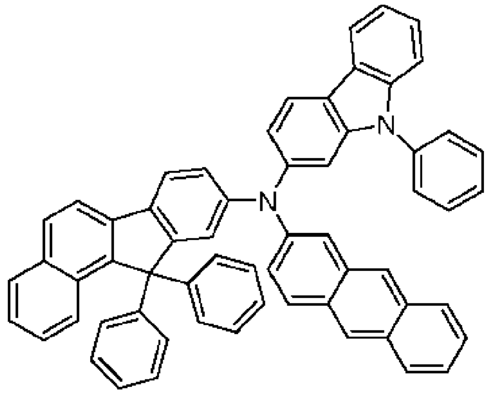
(A22),

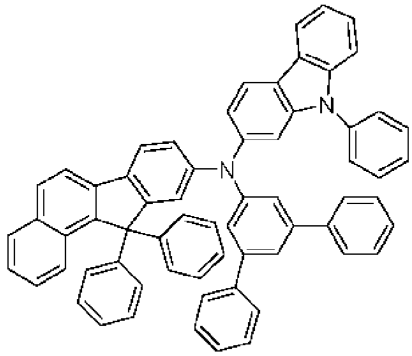
(A23),

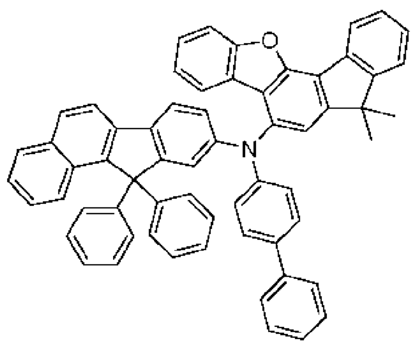
(A24),

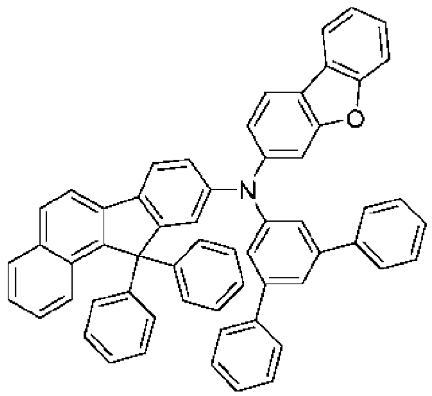
(A25),

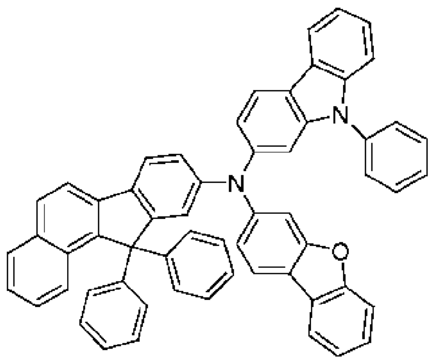
(A26),

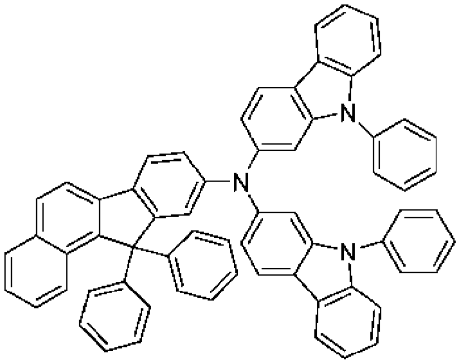
(A27),

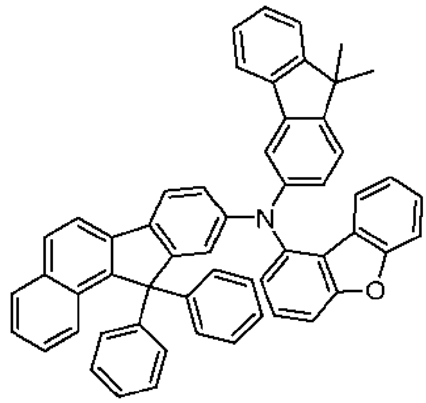
(A28),

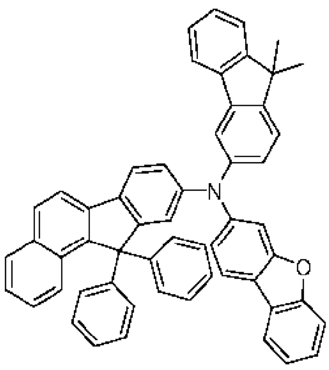
(A29),

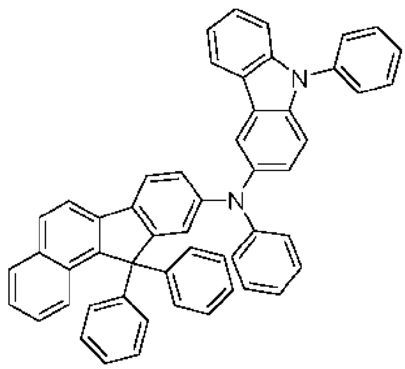
(A30),

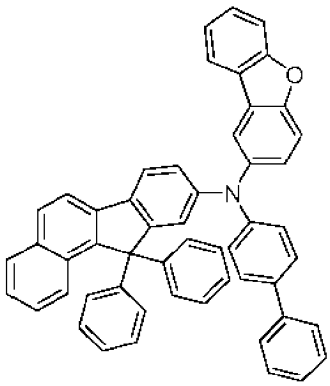 (A31),
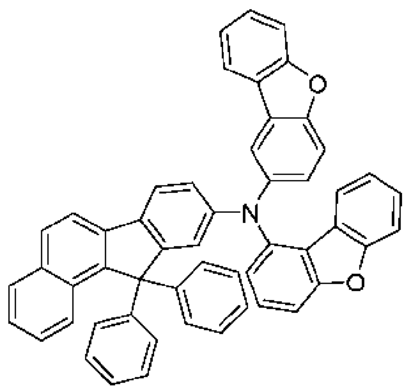 (A32),

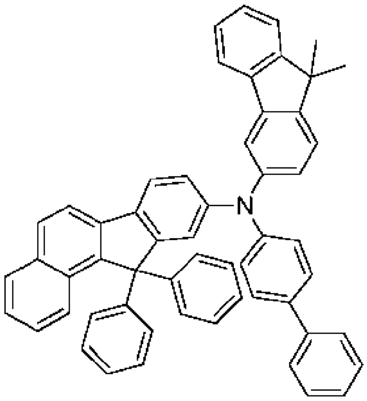 (A33),
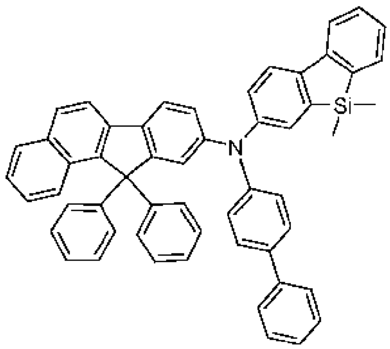 (A34),

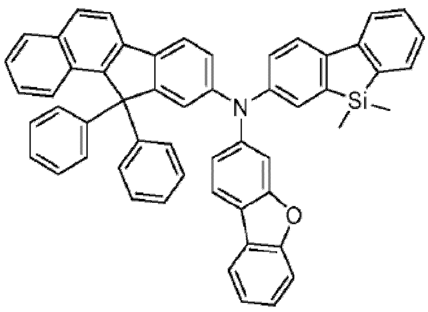 (A35),
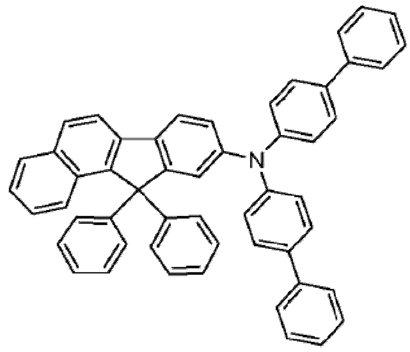 (A36),

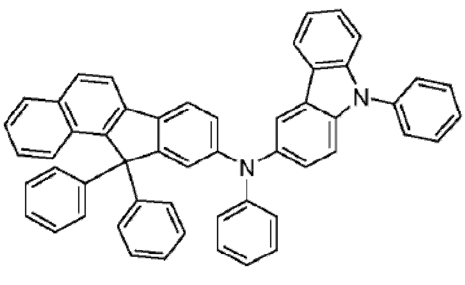 (A37).

9. Organische Halbleiterschicht, umfassend eine Benzodiphenylfluorenverbindung der Formel I nach einem der vorhergehenden Ansprüche 1 bis 8.

10. Organische Halbleiterschicht nach Anspruch 9, wobei es sich bei der Halbleiterschicht um eine Lochinjektionsschicht und/oder Lochtransportschicht handelt.

11. Organische Halbleiterschicht nach Anspruch 9 oder 10, wobei die organische Halbleiterschicht zusätzlich einen

organischen p-Dotierstoff umfasst.

**12.** Organische Halbleiterschicht nach Anspruch 11, wobei es sich bei dem organischen p-Dotierstoff um eine Radialenverbindung handelt.

**13.** Organische elektronische Vorrichtung, umfassend mindestens eine Halbleiterschicht nach den Ansprüchen 9 bis 12.

**14.** Organische elektronische Vorrichtung nach Anspruch 13, umfassend eine Anodenschicht, eine Kathodenschicht und mindestens eine organische Halbleiterschicht, wobei mindestens eine organische Halbleiterschicht zwischen der Anodenschicht und der Kathodenschicht angeordnet ist.

**15.** Organische elektronische Vorrichtung nach Anspruch 13 oder 14, wobei es sich bei der organischen elektronischen Vorrichtung um eine organische Leuchtdiode (OLED), eine lichtemittierende Vorrichtung, ein Dünnschichttransistor, eine Batterie, eine Anzeigevorrichtung, eine organische Photovoltaikzelle (OPV), eine Solarzelle, eine Perowskit-Solarzelle, ein Photoleiter, eine Photodiode oder einen Photodetektor handelt.

## Revendications

**1.** Composé benzodiphénylfluorène, représenté par la formule (I) :

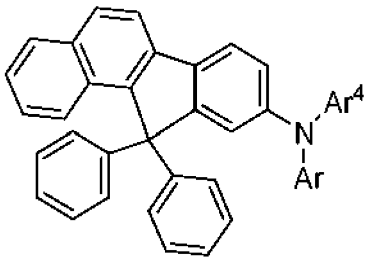

(I),

$Ar^4$ étant représenté par la formule (Ia) ou (Ib),

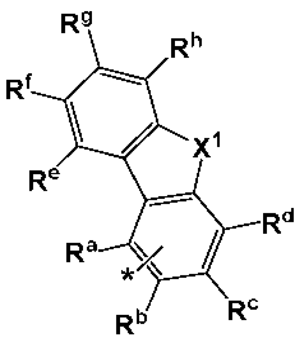

(Ia),

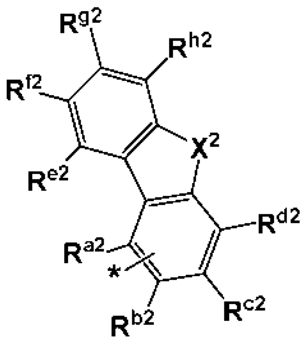

(Ib),

l'astérisque « * » désignant la position de liaison de (Ia) et (Ib), et
Ar = $Ar^1$ et $Ar^1$ étant choisi parmi $C_6$ à $C_{24}$ aryle substitué ou non substitué, ou $C_3$ à $C_{25}$ hétéroaryle substitué ou non substitué,
au moins un substituant sur $Ar^1$ étant choisi parmi $C_1$ à $C_6$ alkyle, $C_6$ à $C_{12}$ aryle, $C_3$ à $C_{12}$ hétéroaryle ;
$R^a$, $R^b$, $R^c$ et $R^d$ étant indépendamment choisis parmi H, $C_1$ à $C_6$ alkyle, $C_6$ à $C_{18}$ aryle, et $C_2$ à $C_{18}$ hétéroaryle, et l'un parmi le $R^a$, $R^b$, $R^c$ ou $R^d$ représente une simple liaison qui se lie à N de la formule (I), éventuellement deux des substituants adjacents choisis dans le groupe de $R^a$, $R^b$, $R^c$ et $R^d$ formant un système cyclique condensé substitué ou non substitué,
au moins un substituant sur le système cyclique condensé étant indépendamment choisi parmi $C_1$ à $C_6$ alkyle, $C_6$ à $C_{12}$ aryle, et $C_2$ à $C_{12}$ hétéroaryle ;
$R^e$, $R^f$, $R^g$, et $R^h$ étant indépendamment choisis parmi H, $C_1$ à $C_6$ alkyle, $C_6$ à $C_{18}$ aryle, et $C_2$ à $C_{18}$ hétéroaryle ;
$X^1$ étant choisi parmi O, S, $NAr^{1a}$ ;

$Ar^{1a}$ étant choisi parmi $C_6$ à $C_{12}$ aryle ;
ou
Ar = $Ar^2$ et $Ar^2$ étant choisi parmi $C_6$ à $C_{12}$ aryle ou $C_2$ à $C_{25}$ hétéroaryle ;
$R^{a2}$, $R^{b2}$ $R^{c2}$ et $R^{d2}$ étant indépendamment choisis parmi H, $C_1$ à $C_6$ alkyle, $C_6$ à $C_{18}$ aryle, et $C_2$ à $C_{18}$ hétéroaryle, et l'un parmi le $R^{a2}$, $R^{b2}$, $R^{c2}$ ou $R^{d2}$ représente une simple liaison qui se lie à N de la formule (I), éventuellement deux des substituants adjacents choisis dans le groupe de $R^{a2}$, $R^{b2}$, $R^{c2}$ et $R^{d2}$ formant un système cyclique condensé substitué ou non substitué,
au moins un substituant sur le système cyclique condensé étant indépendamment choisi parmi $C_1$ à $C_6$ alkyle, $C_6$ à $C_{12}$ aryle ou $C_2$ à $C_{12}$ hétéroaryle ;
$R^{e2}$, $R^{f2}$, $R^{g2}$, et $R^{h2}$ étant indépendamment choisis parmi H, $C_1$ à $C_6$ alkyle, $C_6$ à $C_{18}$ aryle, et $C_2$ à $C_{18}$ hétéroaryle ; $X^2$ étant choisi parmi O, S, $NAr^{2b}$, $CR^{1b}R^{2b}$, $SiR^{1b}R^{2b}$ ; $Ar^{2b}$ étant choisi parmi $C_6$ à $C_{12}$ aryle ; et $R^{1b}$ et $R^{2b}$ étant indépendamment choisis parmi $C_1$ à $C_6$ alkyle, $C_6$ à $C_{12}$ aryle ou $C_2$ à $C_{12}$ hétéroaryle ;
ou représenté par le composé A36 :

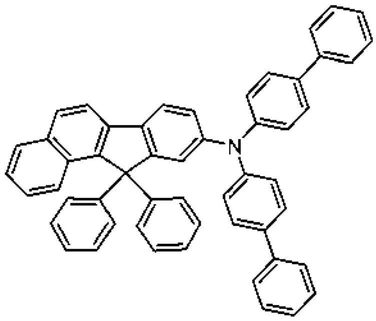

(A36).

2. Composé benzodiphénylfluorène selon la revendication 1, le composé de formule (I) étant représenté par un composé de formule (Ic), (Id) ou (Ie), la formule (Ic) étant :

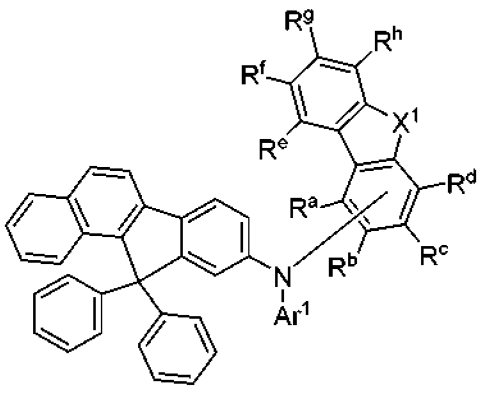

(Ic),

$Ar^1$ étant choisi parmi $C_6$ à $C_{24}$ aryle substitué ou non substitué, ou $C_5$ à $C_{25}$ hétéroaryle substitué ou non substitué,
au moins un substituant sur $Ar^1$ étant choisi parmi $C_1$ à $C_6$ alkyle, $C_6$ à $C_{12}$ aryle, $C_5$ à $C_{12}$ hétéroaryle ;
$R^a$, $R^b$, $R^c$ et $R^d$ étant indépendamment choisis parmi H, $C_1$ à $C_6$ alkyle, $C_6$ à $C_{18}$ aryle, et $C_2$ à $C_{18}$ hétéroaryle, et l'un parmi le $R^a$, $R^b$, $R^c$ ou $R^d$ représente une simple liaison qui se lie à N de la formule (I), éventuellement deux des substituants adjacents choisis dans le groupe de $R^a$, $R^b$, $R^c$ et $R^d$ formant un système cyclique condensé substitué ou non substitué,
au moins un substituant sur le système cyclique condensé étant indépendamment choisi parmi $C_1$ à $C_6$ alkyle, $C_6$ à $C_{12}$ aryle ou $C_2$ à $C_{12}$ hétéroaryle ;
$R^e$, $R^f$, $R^g$, et $R^h$ étant indépendamment choisis parmi H, $C_1$ à $C_6$ alkyle, $C_6$ à $C_{18}$ aryle, et $C_2$ à $C_{18}$ hétéroaryle ;
$X^1$ étant choisi parmi O, S, $Nar^{1a}$ ;
$Ar^{1a}$ étant choisi parmi $C_6$ à $C_{12}$ aryle ;
ou
la formule (Id) étant :

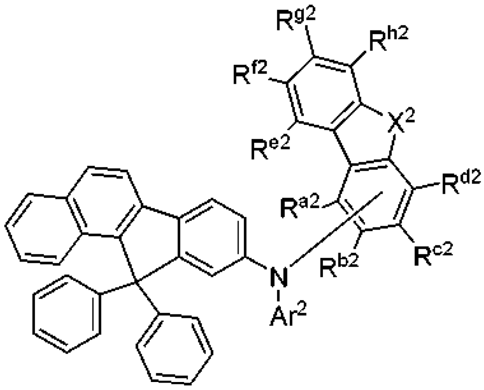

(Id),

$Ar^2$ étant choisi parmi $C_6$ à $C_{12}$ aryle ou $C_5$ à $C_{25}$ hétéroaryle ;
$R^{a2}$, $R^{b2}$ $R^{c2}$ et $R^{d2}$ étant indépendamment choisis parmi H, $C_1$ à $C_6$ alkyle, $C_6$ à $C_{18}$ aryle, et $C_2$ à $C_{18}$ hétéroaryle, et l'un parmi le $R^{a2}$, $R^{b2}$, $R^{c2}$ ou $R^{d2}$ représente une simple liaison qui se lie à N de la formule (Ib), éventuellement deux des substituants adjacents choisis dans le groupe de $R^{a2}$, $R^{b2}$, $R^{c2}$ et $R^{d2}$ formant un système cyclique condensé substitué ou non substitué,
au moins un substituant sur le système cyclique condensé étant indépendamment choisi parmi $C_1$ à $C_6$ alkyle, $C_6$ à $C_{12}$ aryle ou $C_2$ à $C_{12}$ hétéroaryle ;
$R^{e2}$, $R^{f2}$, $R^{g2}$, et $R^{h2}$ étant indépendamment choisis parmi H, $C_1$ à $C_6$ alkyle, $C_6$ à $C_{18}$ aryle, et $C_2$ à $C_{18}$ hétéroaryle ; $X^2$ étant choisi parmi O, S, $NAr^{2b}$, $CR^{1b}R^{2b}$, $SiR^{1b}R^{2b}$ ; $Ar^{2b}$ étant choisi parmi $C_6$ à $C_{12}$ aryle ; et $R^{1b}$ et $R^{2b}$ étant indépendamment choisis parmi $C_1$ à $C_6$ alkyle, $C_6$ à $C_{12}$ aryle ou $C_2$ à $C_{12}$ hétéroaryle ;
ou
la formule (Ie) étant :

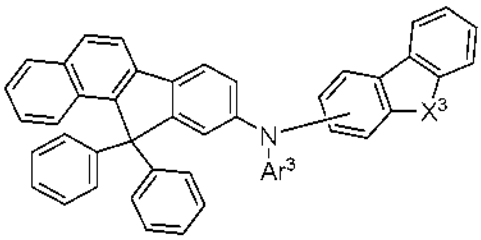

(Ie),

$Ar^3$ étant choisi parmi biphényle,
$X^3$ étant choisi parmi O, S, $NAr^{2b}$, $CR^{1b}R^{2b}$ ;
$Ar^{2b}$ étant choisi parmi $C_6$ à $C_{12}$ aryle ; et
$R^{1b}$ et $R^{2b}$ étant indépendamment choisis parmi $C_1$ à $C_6$ alkyle, $C_6$ à $C_{12}$ aryle ou $C_2$ à $C_{12}$ hétéroaryle.

**3.** Composé benzodiphénylfluorène selon la revendication 1 ou 2, pour la formule (Ic) $X^1$ étant choisi parmi O ou $NAR^2$, préférablement O ; et pour la formule (Id) $X^2$ étant choisi parmi O, $NAR^{2b}$, $CR^{1b}R^{2b}$ et $SiR^{1b}R^{2b}$, préférablement choisi parmi O, et $CR^{1b}R^{2b}$, et de plus préférablement choisi parmi $CR^{1b}R^{2b}$ ; et pour la formule (Ie) $X^3$ étant choisi parmi O, S et $SiR^{1b}R^{2b}$, préférablement choisi parmi O et S, et de plus préférablement choisi parmi O.

**4.** Composé benzodiphénylfluorène selon l'une quelconque des revendications précédentes 1 à 3, pour la formule (Ic) $Ar^1$ étant choisi parmi $C_6$ à $C_{13}$ aryle substitué ou non substitué, et $C_{12}$ hétéroaryle substitué ou non substitué, préférablement $Ar^1$ étant choisi parmi $C_6$ à $C_{13}$ aryle substitué ; et pour la formule (Id) $Ar^2$ étant choisi parmi $C_6$ à $C_{10}$ aryle, et préférablement $Ar^2$ étant choisi parmi $C_6$ aryle.

**5.** Composé benzodiphénylfluorène selon l'une quelconque des revendications 1 à 4,
Ar, $Ar^1$ et/ou $Ar^2$ étant choisis dans le groupe de B1 à B13 :

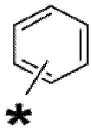 (B1), 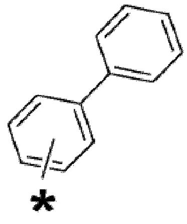 (B2), 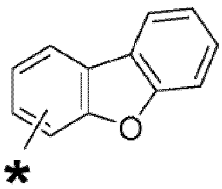 (B3), 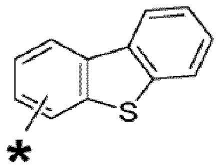 (B4),

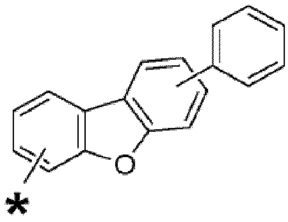 (B5), 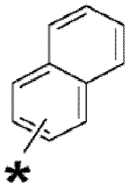 (B6), 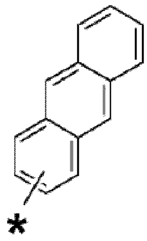 (B7),

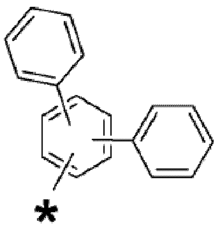 (B8), 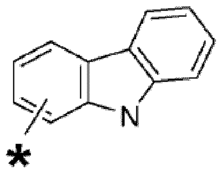 (B9), 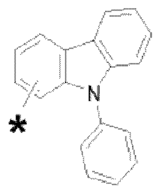 (B10),

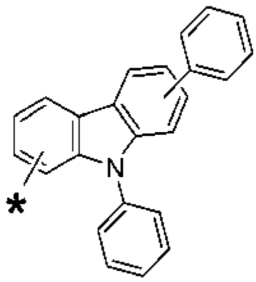 (B11), 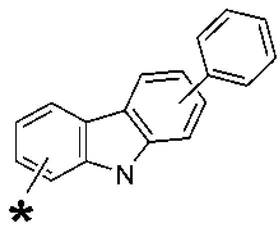 (B12);

l'astérisque « * » désignant la position de liaison de Ar, $Ar^1$ et/ou $Ar^2$.

6. Composé benzodiphénylfluorène selon la revendication 5,

- $Ar^1$ étant choisi dans le groupe de B1 à B12, préférablement choisi dans le groupe de B1, B2, B3, B4, B5, B6, et de plus préférablement choisi dans le groupe de B1, B2, B3 ;
- $Ar^2$ étant choisi dans le groupe de B1 à B12, préférablement choisi dans le groupe de B1, B2, B3, B4, B5, B6, et de plus préférablement choisi dans le groupe de B1, B2, B3.

7. Composé benzodiphénylfluorène selon l'une quelconque des revendications précédentes 1 à 5, $Ar^4$ étant choisi dans le groupe de D1 à D7 :

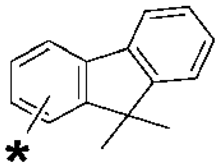 (D1), 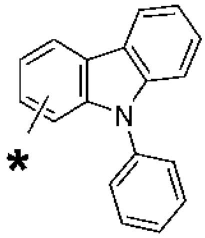 (D2), 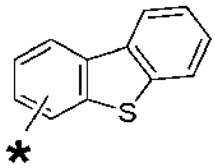 (D3),

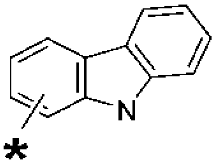 (D4), 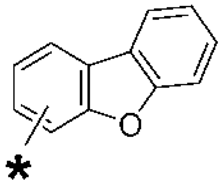 (D5),

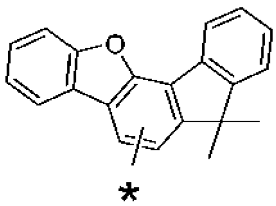 (D6), 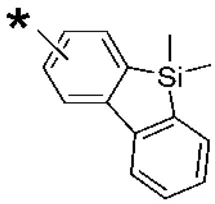 (D7);

l’astérisque « * » désignant la position de liaison de Ar4.

**8.** Composé benzodiphénylfluorène selon l’une quelconque des revendications 1 à 7, le composé de formule I étant choisi parmi A1 à A35, 37 :

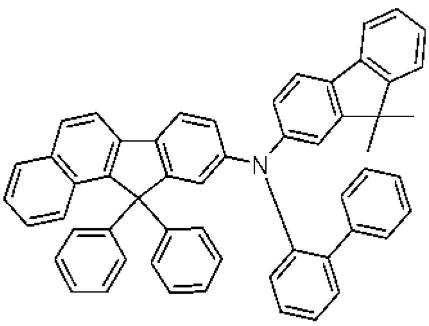 (A1), 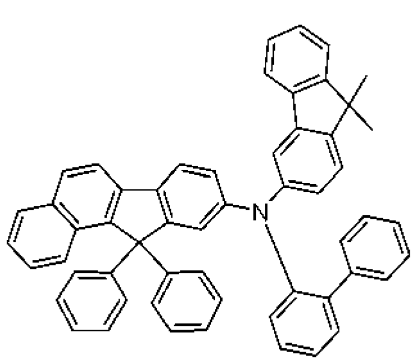 (A2),

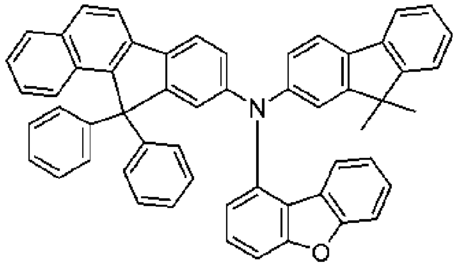 (A3), 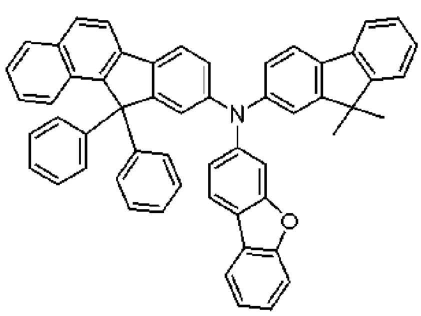 (A4),

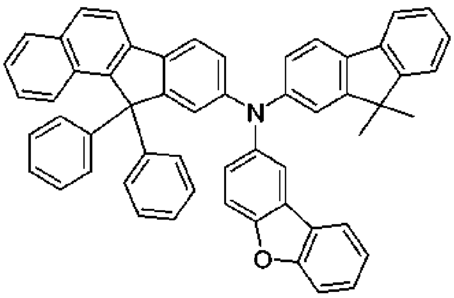 (A5), 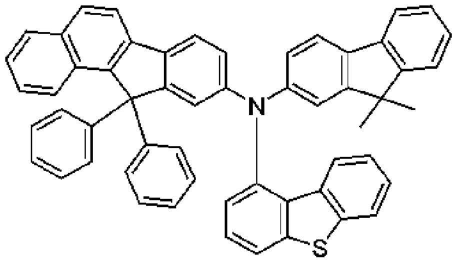 (A6),

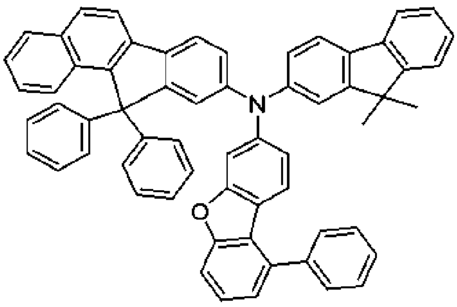 (A7), 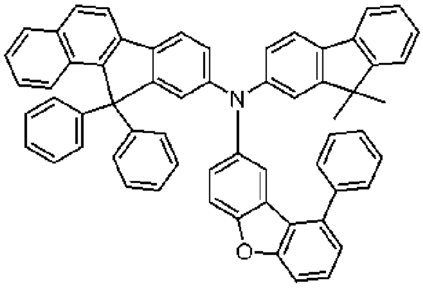 (A8),

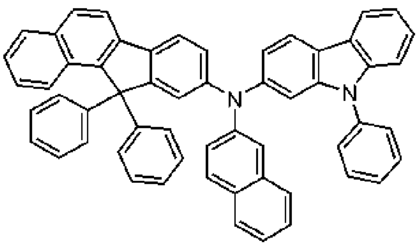 (A9), 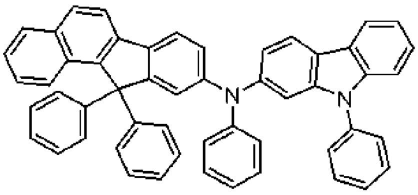 (A10),

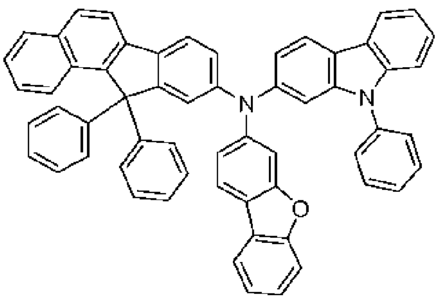 (A11), 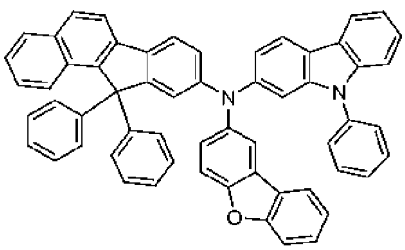 (A12),

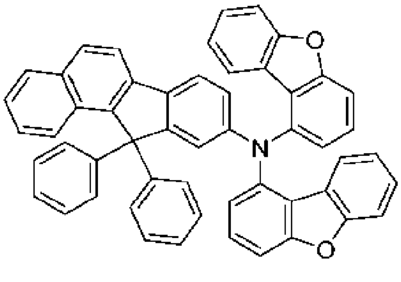 (A13), 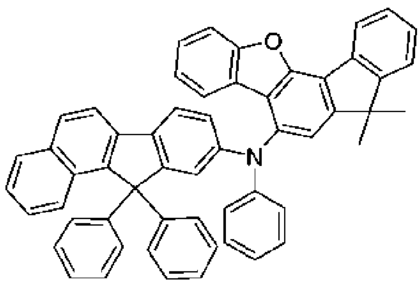 (A14),

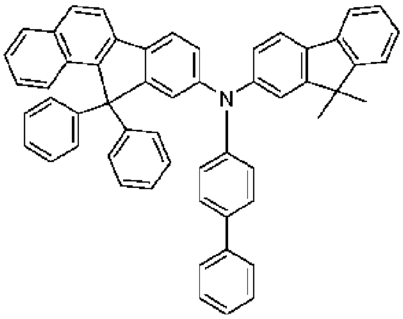 (A15), 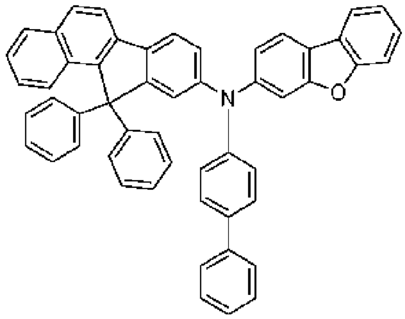 (A16),

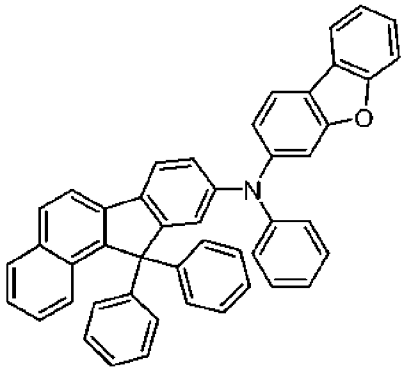 (A17), 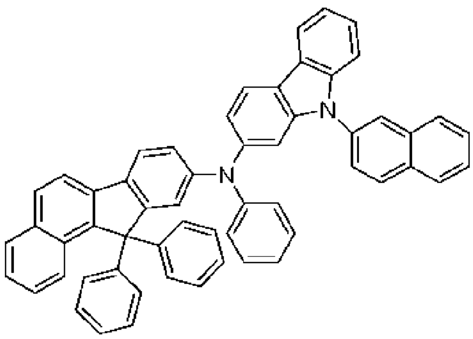 (A18),

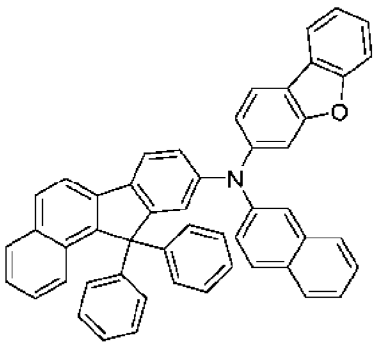 (A19), 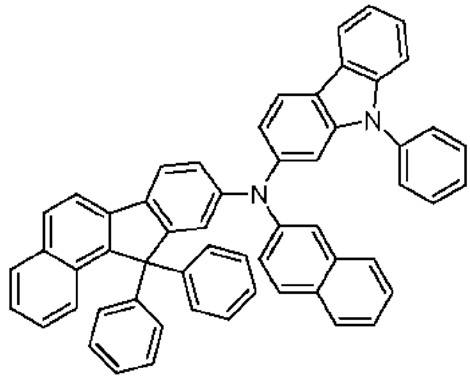 (A20),

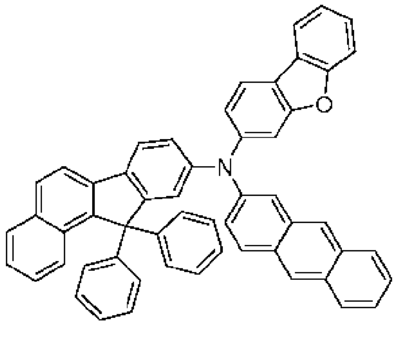 (A21), 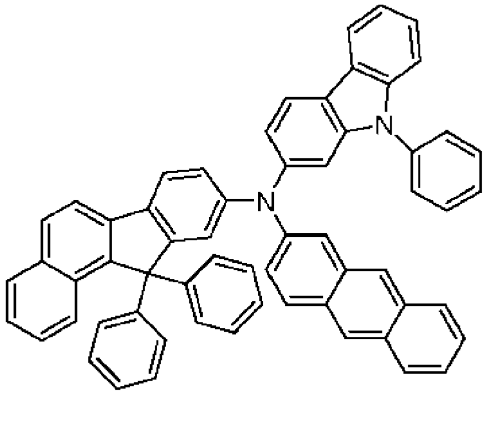 (A22),

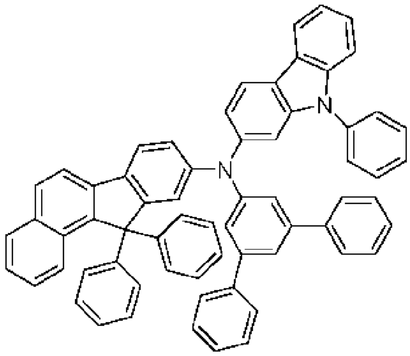 (A23),

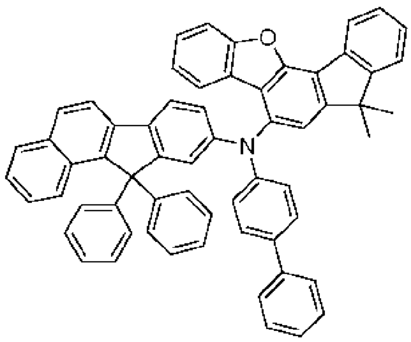 (A24),

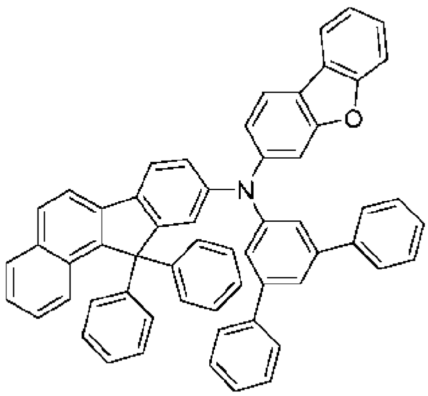 (A25),

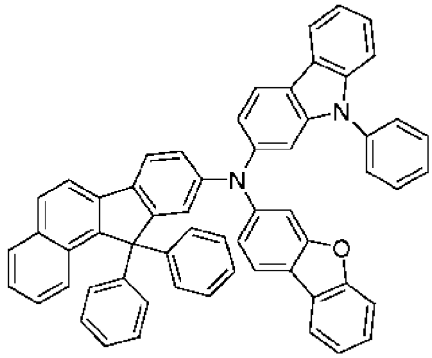 (A26),

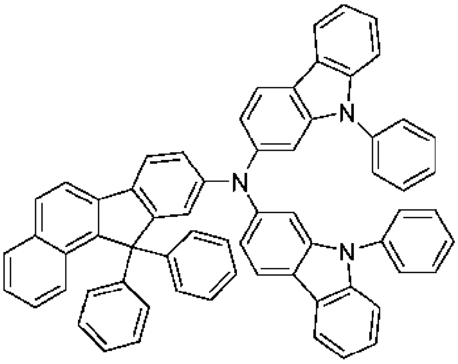 (A27),

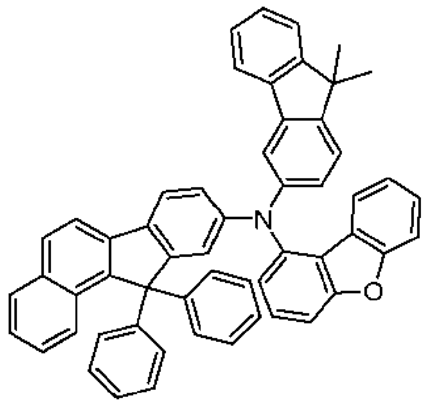 (A28),

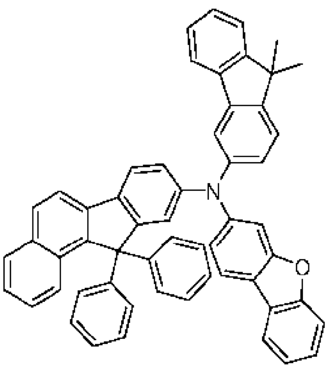 (A29),

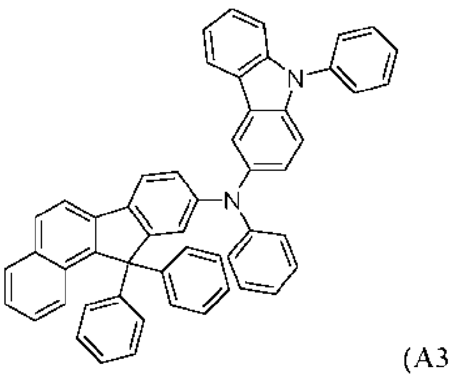 (A30),

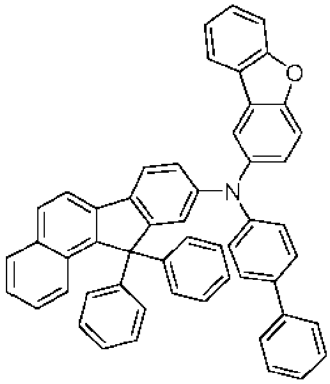
(A31),

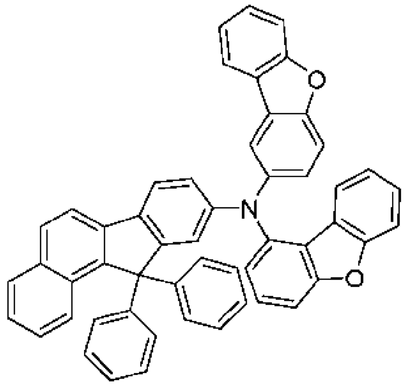
(A32),

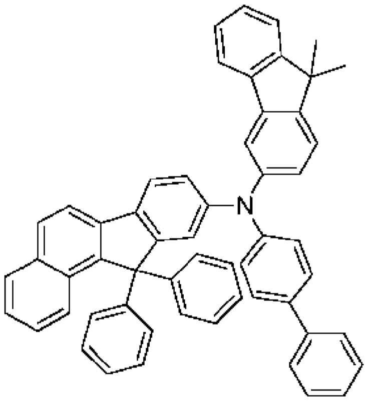
(A33),

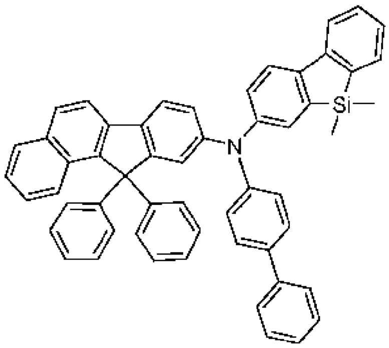
(A34),

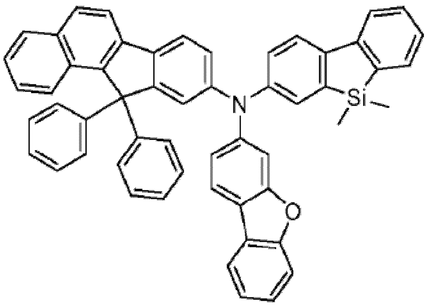
(A35),

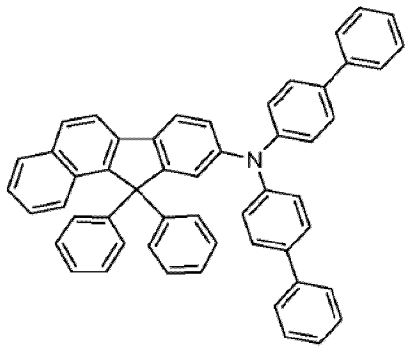
(A36),

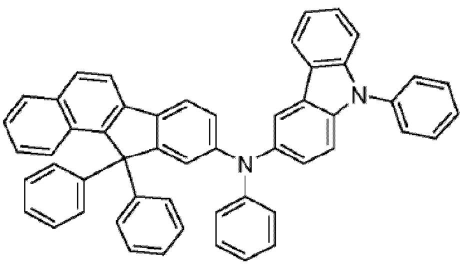
(A37).

**9.** Couche de semi-conducteur organique comprenant un composé benzodiphénylfluorène de formule I selon l'une quelconque des revendications précédentes 1 à 8.

**10.** Couche de semi-conducteur organique selon la revendication 9, dans laquelle la couche de semi-conducteur est une couche d'injection de trous et/ou une couche de transport de trous.

**11.** Couche de semi-conducteur organique selon la revendication 9 ou 10, dans laquelle la couche de semi-conducteur organique comprend en outre un dopant p organique.

**12.** Couche de semi-conducteur organique selon la revendication 11, dans laquelle le dopant p organique est un composé radialène.

**13.** Dispositif électronique organique comprenant au moins une couche de semi-conducteur selon les revendications 9 à 12.

**14.** Dispositif électronique organique selon la revendication 13, comprenant une couche d'anode, une couche de cathode et au moins une couche de semi-conducteur organique, dans lequel au moins une couche de semi-conducteur organique est agencée entre la couche d'anode et la couche de cathode.

**15.** Dispositif électronique organique selon la revendication 13 ou 14, dans lequel le dispositif électronique organique est une diode émettrice de lumière organique (OLED), un dispositif émetteur de lumière, un transistor en film mince, une batterie, un dispositif d'affichage, une cellule photovoltaïque organique (OPV), une cellule solaire, une cellule solaire de pérovskite, un photoconducteur, une photodiode ou un photodétecteur.

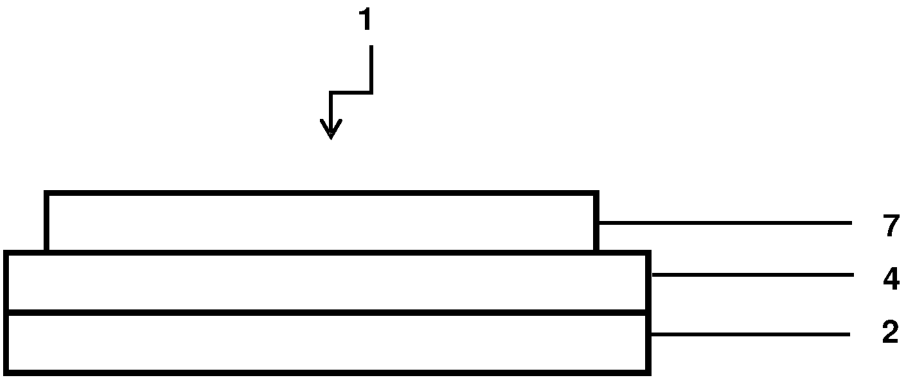


Fig. 1

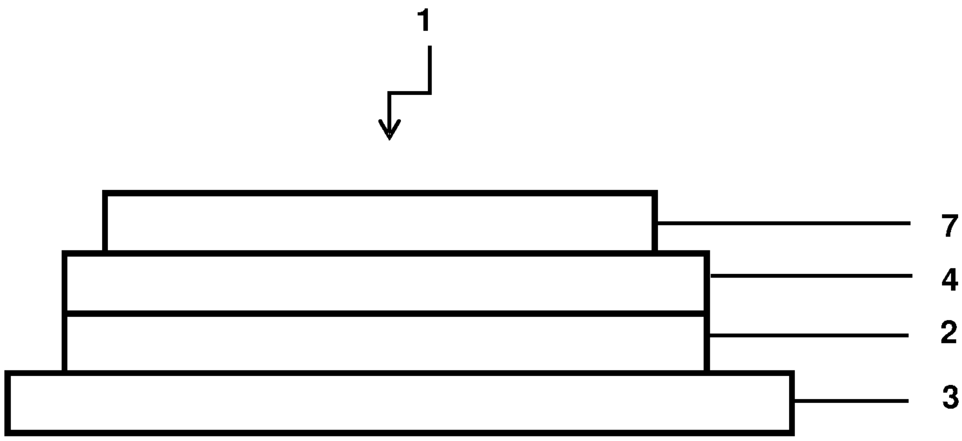


Fig. 2

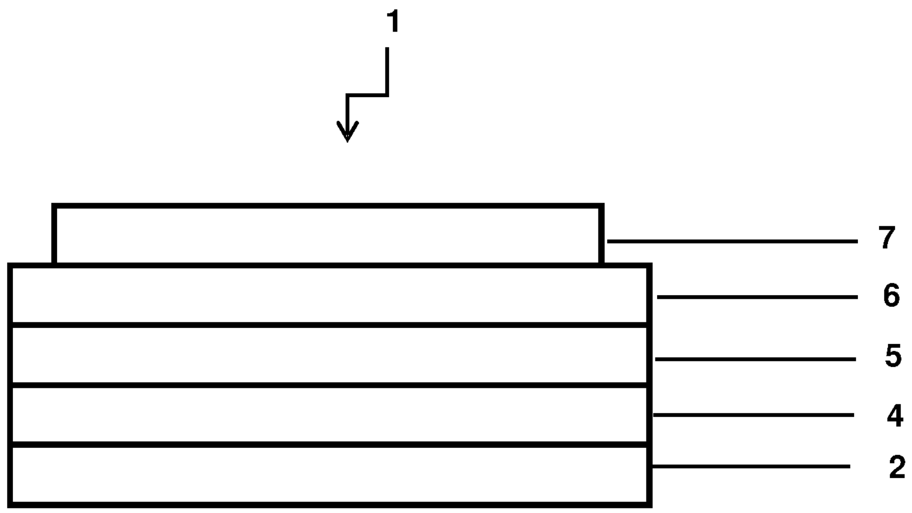


Fig. 3

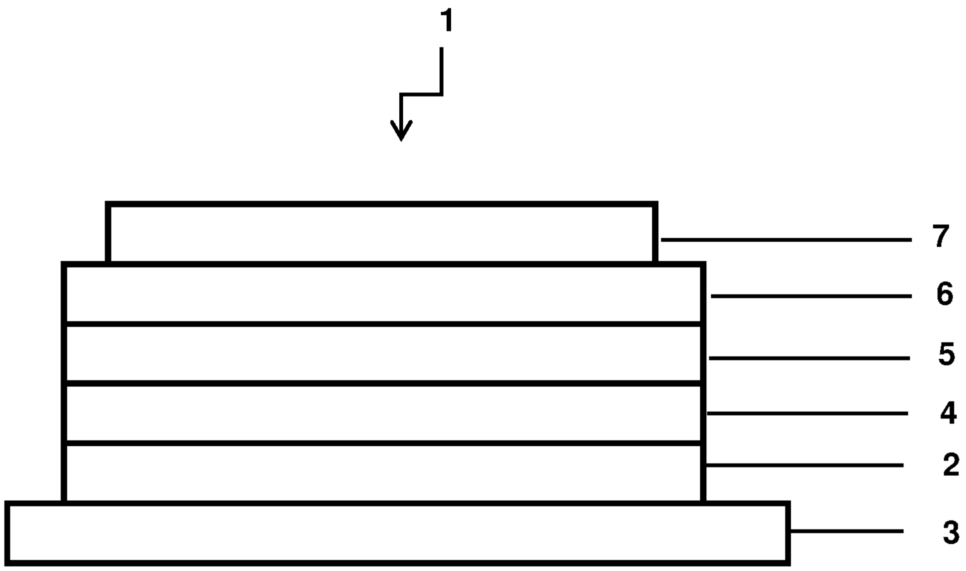


Fig. 4

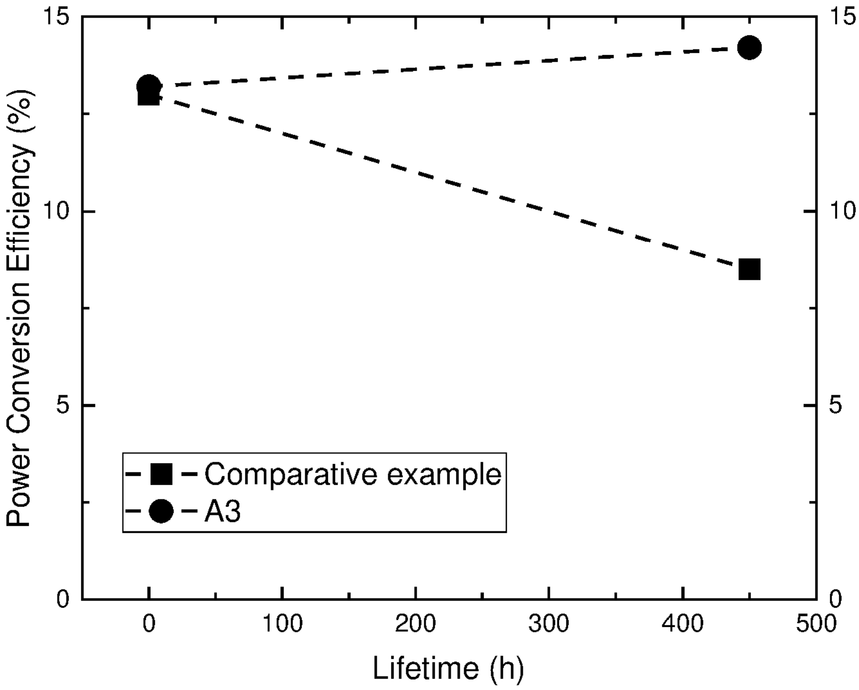
Power Conversion Efficiency (%)
Lifetime (h)
Comparative example
A3
0
5
10
15
0
100
200
300
400
500

Fig. 5

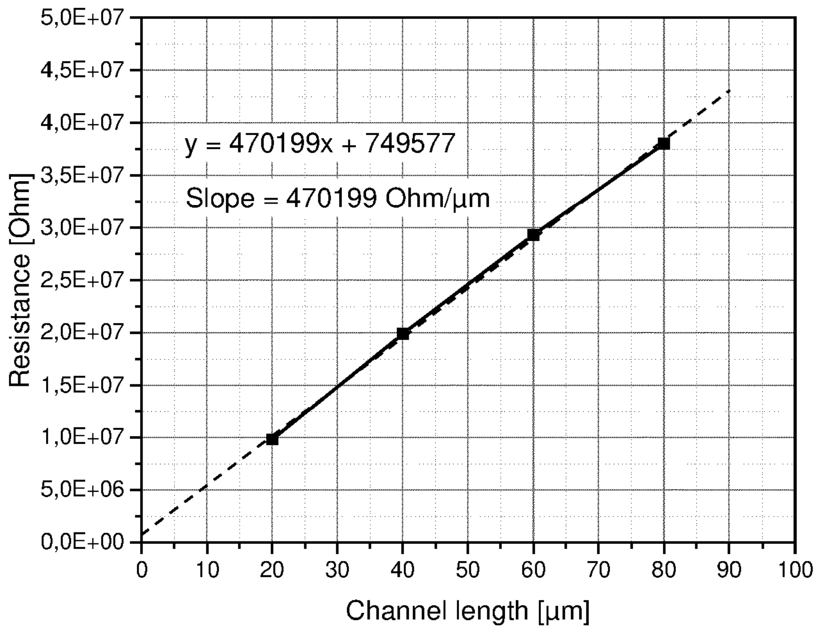
y = 470199x + 749577
Slope = 470199 Ohm/µm
Resistance [Ohm]
Channel length [µm]
5,0E+07
4,5E+07
4,0E+07
3,5E+07
3,0E+07
2,5E+07
2,0E+07
1,5E+07
1,0E+07
5,0E+06
0,0E+00
0
10
20
30
40
50
60
70
80
90
100

Fig. 7

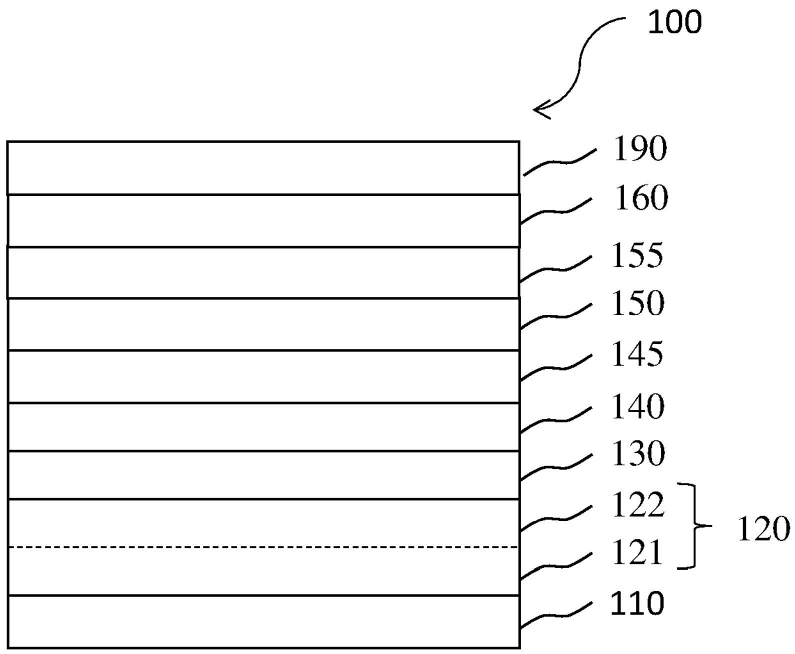
100
190
160
155
150
145
140
130
122
121
120
110

Fig. 8

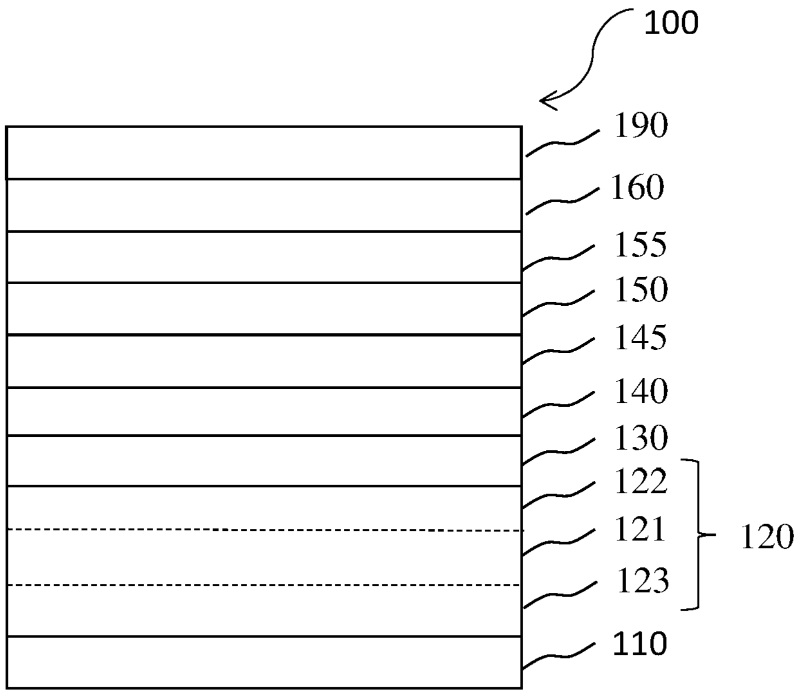


Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 107602441 A
- US 2020365813 A1
- EP 2722908 A1
- US 2005121667 A1
- US 2005139810 A1
- WO 2012175219 A1


### Non-patent literature cited in the description


- CRC Handbook of Chemistry and Physics. 2008, 12-114
- **YASUHIKO SHIROTA** ; **HIROSHI KAGEYAMA**. *Chem. Rev.*, 2007, vol. 107, 953-1010
- *CHEMICAL ABSTRACTS*, 1198395-24-2
- *CHEMICAL ABSTRACTS*, 1421789-39-0
- *CHEMICAL ABSTRACTS*, 2225845-23-6
- *CHEMICAL ABSTRACTS*, 1427556-50-0
- *CHEMICAL ABSTRACTS*, 1427316-55-9
- *CHEMICAL ABSTRACTS*, 1824678-59-2
- *CHEMICAL ABSTRACTS*, 140CT
- *CHEMICAL ABSTRACTS*, 51870-56-5
- **REED, BOLSKAR**. *Chem. Rev.*, 2000, vol. 100, 1075
- *CHEMICAL ABSTRACTS*, 99685-96-8